(19) Europäisches Patentamt
European Patent Office
Office européen des brevets

(11) EP 0 719 763 A1

# (12) EUROPÄISCHE PATENTANMELDUNG

(43) Veröffentlichungstag:
**03.07.1996 Patentblatt 1996/27**

(21) Anmeldenummer: **95118606.3**

(22) Anmeldetag: **27.11.1995**

(51) Int. Cl.[6]: **C07D 215/14**, A61K 31/47, C07D 405/12, C07D 403/12, C07D 401/12, C07D 417/12, C07D 409/12

(84) Benannte Vertragsstaaten:
**CH DE FR GB IT LI**

(30) Priorität: **09.12.1994 DE 4443892**

(71) Anmelder: **BAYER AG**
**51368 Leverkusen (DE)**

(72) Erfinder:
- **Müller, Ulrich, Dr.**
  **D-42111 Wuppertal (DE)**
- **Connell, Richard, Dr.**
  **Trumbull, CT 06611 (US)**
- **Goldmann, Siegfried, Dr.**
  **D-42327 Wuppertal (DE)**
- **Mohrs, Klaus-Helmut, Dr.**
  **D-42113 Wuppertal (DE)**
- **Angerbauer, Rolf, Dr.**
  **D-42113 Wuppertal (DE)**
- **Müller-Gliemann, Matthias, Dr.**
  **D-42697 Solingen (DE)**
- **Niewöhner, Ulrich, Dr.**
  **D-42929 Wermelskirchen (DE)**
- **Grützmann, Rudi, Dr.**
  **D-42657 Solingen (DE)**
- **Beuck, Martin, Dr.**
  **Milford, CT 06460 (US)**
- **Wohlfeil, Stefan, Dr.**
  **D-40724 Hilden (DE)**
- **Bischoff, Stefan, Dr.**
  **D-42113 Wuppertal (DE)**
- **Denzer, Dirk, Dr.**
  **D-42115 Wuppertal (DE)**

## (54) 4-(Chinolin-2-yl-methoxy)-phenyl-essigsäurederivate mit antiatherosklerotischer Wirkung

(57) 4-(Chinolin-2-yl-methoxy)-phenyl-essigsäurederivate werden hergestellt indem man Chinolin-methoxy-phenyl-essigsäuren, gegebenenfalls unter Aktivierung der Carbonsäurefunktion mit den entsprechenden Glycinolderivaten umsetzt. Die neuen Verbindungen sind geeignet als Wirkstoffe in Arzneimitteln insbesondere in antiatherosklerotischen Arzneimitteln.

EP 0 719 763 A1

Printed by Rank Xerox (UK) Business Services
2.12.4/3.4

## Beschreibung

Die vorliegende Erfindung betrifft 4-(Chinolin-2-yl-methoxy)-phenyl-essigsäurederivate, Verfahren zu ihrer Herstellung und ihre Verwendung als Arzneimittel, insbesondere als antiatherosklerotische Arzneimittel.

Es ist bekannt, daß erhöhte Blutspiegel von Triglyceriden (Hypertriglyceridämie) und Cholesterin (Hypercholesterinämie) mit der Genese von atherosklerotischen Gefäßwand Veränderungen und koronaren Herzkrankheiten assoziiert sind.

Ein deutlich erhöhtes Risiko für die Entwicklung koronarer Herzerkrankungen liegt darüber hinaus vor, wenn diese beiden Risikofaktoren kombiniert auftreten, was wiederum mit einer Überproduktion an Apolipoprotein B-100 einhergeht. Es ist daher nach wie vor ein starkes Bedürfnis, wirksame Arzneimittel zur Bekämpfung der Atherosklerose sowie koronarer Herzkrankheiten zur Verfügung zu stellen.

Aus der Publikation EP-344 519 sind die Verbindungen 2(R*)- und 2(S*)-2-[4-(Chinolin-2-yl-methoxy)phenyl]-2-cyclopentylessigsäure(S)-phenylglycinolamid bekannt.

Die vorliegende Erfindung betrifft 4-(Chinolin-2-yl-methoxy)-phenyl-essigsäureamide der allgemeinen Formel (I),

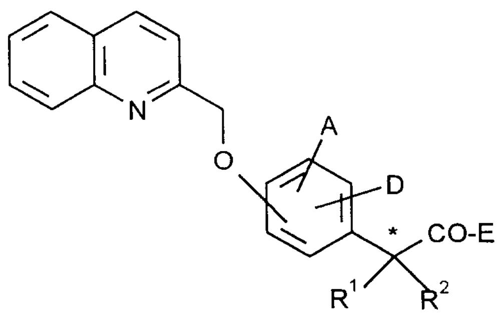

(I)

in welcher

A und D gleich oder verschieden sind und für Wasserstoff, Cycloalkyl mit 3 bis 8 Kohlenstoffatomen, Azido, Hydroxy, Halogen, geradkettiges oder verzweigtes Alkyl, Alkoxy oder Alkenyl mit jeweils bis zu 6 Kohlenstoffatomen, einen 5- bis 6-gliedrigen ungesättigten oder gesättigten Heterocyclus mit bis zu 3 Heteroatomen aus der Reihe S, N und/oder O stehen,

$R^1$ für Wasserstoff, Hydroxy oder für geradkettiges oder verzweigtes Alkyl mit bis zu 4 Kohlenstoffatomen steht,

$R^2$ für Wasserstoff, Hydroxy, Halogen, geradkettiges oder verzweigtes Alkenyl oder Alkoxy mit jeweils bis zu 8 Kohlenstoffatomen oder Cycloalkyl mit 3 bis 14 Kohlenstoffatomen steht, oder
für geradkettiges oder verzweigtes Alkyl mit bis zu 8 Kohlenstoffatomen steht, das gegebenenfalls durch Cycloalkyl mit 3 bis 14 Kohlenstoffatomen, Phenyl oder Tetrahydropyranyl substituiert ist, welche ihrerseits durch Halogen substituiert sein können, oder
für den Indanylrest steht,

oder

$R^1$ und $R^2$ gemeinsam mit dem Kohlenstoffatom einen gesättigten carbocyclischen Ring mit 5 bis 7 Kohlenstoffatomen bilden,

oder

$R^1$ und $R^2$ gemeinsam für einen Doppelbindungs-Rest der Formel

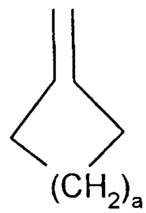

stehen,
worin

a eine Zahl 2, 3, 4, 5 oder 6 bedeutet,

E für einen Rest der Formel

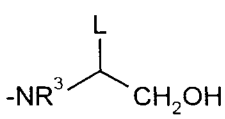 , 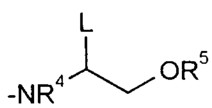 , 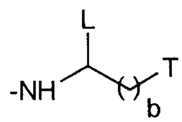 ,

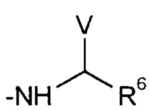 , 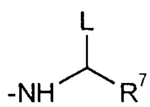 , 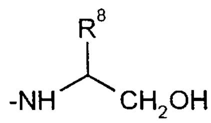 ,

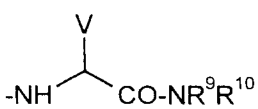 ,

$-NR^{4'}-W-OH$,

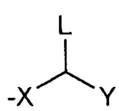 ,

$-NHR^{11}$, oder

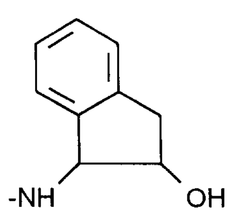

steht,

in welchen

$R^3$ Phenyl, Methyl oder eine typische Aminoschutzgruppe bedeutet,

$R^4$ und $R^{4'}$ gleich oder verschieden sind und die oben angegebene Bedeutung von $R^3$ haben oder Wasserstoff bedeuten,

$R^5$ geradkettiges oder verzweigtes Acyl oder Alkyl mit jeweils bis zu 4 Kohlenstoffatomen bedeutet,

L Phenyl, Benzyl oder Naphtyl bedeutet, das gegebenenfalls bis zu 2-fach gleich oder verschieden durch Halogen, Hydroxy, Pyrrolidinyl, Morpholino, Amino, Trifluormethyl, Trifluormethoxy, Cycloalkyl mit 3 bis 6 Kohlenstoffatomen, geradkettiges oder verzweigtes Alkyl oder Alkoxy mit jeweils bis zu 6 Kohlenstoffatomen substituiert ist, die ihrerseits durch Hydroxy substituiert sein können, oder gegebenenfalls durch Phenyl oder Phenoxy substituiert sind, die ihrerseits bis zu 2-fach gleich oder verschieden durch Halogen oder durch geradkettiges oder verzweigtes Alkyl mit bis zu 4 Kohlenstoffatomen substituiert sein können, und/oder gegebenenfalls durch eine Gruppe der Formel $-(O)_c-SO_2R^{12}$ substituiert sind,
worin

c eine Zahl 0 oder 1 bedeutet und

$R^{12}$ geradkettiges oder verzweigtes Alkyl mit bis zu 4 Kohlenstoffatomen oder Phenyl bedeutet,

b eine Zahl 0, 1 oder 2 bedeutet,

T einen 5- bis 7-gliedrigen, gegebenenfalls benzokondensierten, gesättigten, partiell ungesättigten oder ungesättigten Heterocyclus mit bis zu 3 Heteroatomen aus der Reihe S, N und/oder O bedeutet, wobei beide Cyclen gegebenenfalls bis zu 3-fach gleich oder verschieden durch Halogen, Hydroxy, Morpholino, Amino, Cycloalkyl mit 3 bis 6 Kohlenstoffatomen, geradkettiges oder verzweigtes Alkyl oder Alkoxy mit jeweils bis zu 6 Kohlenstoffatomen oder Phenyl substituiert sind,

V die oben angegebene Bedeutung von L oder T hat, oder
einen Rest der Formel

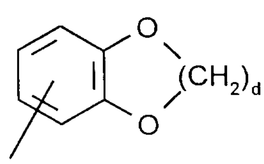

bedeutet,
worin

d eine Zahl 1, 2 oder 3 bedeutet,

$R^6$ einen Rest der Formel $-(CH_2)_e-R^{13}$ bedeutet,
worin

e eine Zahl 0 oder 1 bedeutet,

$R^{13}$ Hydroxy, Carboxy oder geradkettiges oder verzweigtes Alkoxycarbonyl mit bis zu 4 Kohlenstoffatomen bedeutet,

$R^7$ Wasserstoff, Cyano, Trifluormethyl, geradkettiges oder verzweigtes Alkenyl oder Alkyl mit bis 7 Kohlenstoffatomen bedeutet, das gegebenenfalls bis zu 2-fach gleich oder verschieden durch geradkettiges oder verzweigtes Alkoxy mit bis zu 4 Kohlenstoffatomen substituiert ist, oder Alkoxy mit bis zu 6 Kohlenstoffatomen bedeutet, oder

eine Gruppe der Formel $-CO-NH-(CH_2)_f-NR^{14}R^{15}$ bedeutet,
worin

f — eine Zahl 1, 2 oder 3 bedeutet,

$R^{14}$ und $R^{15}$ — gleich oder verschieden sind und Wasserstoff, Phenyl oder geradkettiges oder verzweigtes Alkyl mit bis zu 4 Kohlenstoffatomen bedeuten,

$R^8$ — Wasserstoff, Carboxy, geradkettiges oder verzweigtes Alkoxycarbonyl mit bis zu 4 Kohlenstoffatomen oder geradkettiges oder verzweigtes Alkyl mit bis zu 5 Kohlenstoffatomen bedeutet, das gegebenenfalls durch Hydroxy, Carboxy oder durch geradkettiges oder verzweigtes Alkoxycarbonyl mit jeweils bis zu 4 Kohlenstoffatomen oder Phenyl substituiert ist,

$R^9$ und $R^{10}$ — gleich oder verschieden sind und Wasserstoff, Cycloalkyl mit 3 bis 6 Kohlenstoffatomen oder Phenyl bedeuten, oder geradkettiges oder verzweigtes Alkyl mit bis zu 6 Kohlenstoffatomen bedeuten, das gegebenenfalls durch Phenyl, Cycloalkyl mit 3 bis 6 Kohlenstoffatomen, Hydroxy, Carboxy, durch geradkettiges oder verzweigtes Alkoxy oder Alkoxycarbonyl mit jeweils bis zu 4 Kohlenstoffatomen oder durch eine Gruppe der Formel $-NR^{16}R^{17}$ substituiert ist,
worin

$R^{16}$ und $R^{17}$ — gleich oder verschieden sind und Wasserstoff, Phenyl oder geradkettiges oder verzweigtes Alkyl mit bis zu 4 Kohlenstoffatomen bedeuten,

oder

$R^9$ und $R^{10}$ — gemeinsam mit dem Stickstoffatom einen Heterocyclus der Formel

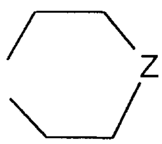

bilden,
worin

Z — ein Sauerstoffatom oder die Gruppe $-NR^{18}$ oder -CH bedeutet,
worin

$R^{18}$ — Wasserstoff, Acetyl, eine typische Aminoschutzgruppe oder einen Rest der Formel $-SO_2R^{19}$ bedeutet,
worin

$R^{19}$ — geradkettiges oder verzweigtes Alkyl mit bis zu 4 Kohlenstoffatomen, Benzyl oder Phenyl bedeutet, das gegebenenfalls durch Phenyl oder Tolyl substiuiert ist,

W — geradkettiges oder verzweigtes Alkyl von 2 bis 7 Kohlenstoffatomen bedeutet, das 1- bis 3-fach gleich oder verschieden durch Hydroxy, Pyridyl, Norbornyl oder Phenyl substituiert ist, das seinerseits durch Hydroxy oder Benzyloxy substituiert sein kann,
oder durch eine Gruppe der Formel $-OR^{20}$ oder $-NR^{21}R^{22}$ substituiert ist,
worin

$R^{20}$ — geradkettiges oder verzweigtes Alkyl ist bis zu 6 Kohlenstoffatomen bedeutet, und

$R^{21}$ und $R^{22}$ gleich oder verschieden sind und die oben angegebene Bedeutung von $R^{16}$ und $R^{17}$ haben

X ein Sauerstoff- oder Schwefelatom bedeutet,

Y Formyl oder die Gruppe $-CHR^{23}R^{24}$ bedeutet,
worin

$R^{23}$ Wasserstoff bedeutet und

$R^{24}$ Hydroxy oder geradkettiges oder verzweigtes Alkoxy oder Acyl mit jeweils bis zu 4 Kohlenstoffatomen bedeutet,
oder

$R^{23}$ und $R^{24}$ gleich oder verschieden sind und geradkettiges oder verzweigtes Alkoxy mit bis zu 4 Kohlenstoffatomen bedeuten

$R^{11}$ einen Rest der Formel

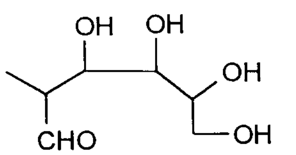
,
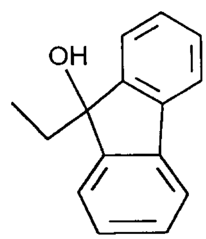
,
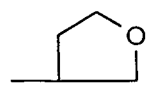

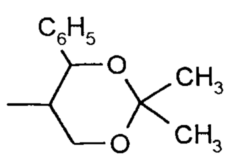
,
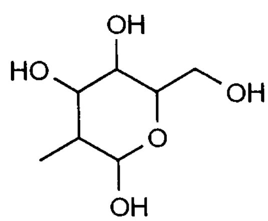

oder
Phenyl bedeutet, das gegebenenfalls bis zu 2-fach gleich oder verschieden durch Halogen, Carboxy oder geradkettiges oder verzweigtes Alkoxycarbonyl mit bis zu 5 Kohlenstoffatomen substituiert ist,
oder
geradkettiges oder verzweigtes Alkyl mit bis zu 6 Kohlenstoffatomen bedeutet, das gegebenenfalls bis zu 2-fach gleich oder verschieden durch Hydroxy, Carboxy, Halogen oder durch geradkettiges oder verzweigtes Alkoxycarbonyl mit bis zu 5 Kohlenstoffatomen substituiert ist,

und deren Salze,
wobei
2-[4-(Chinolin-2-yl-methoxy)phenyl]-2-cyclopentyl-essigsäuremethyloxycarbonylmethylamid,
2-[4-(Chinolin-2-yl-methoxy)phenyl]-2-cyclopentyl-essigsäurecarboxymethylamid,
N-Methyl-2-[3-isobutyl-4-(chinolin-2-yl-methoxy)phenyl]2-cycloheptylacetamid,
N-Methyl-2-[4-(chinolin-2-yl-methoxy)phenyl]-2-cycloheptyl-essigsäureamid,
N-Ethyl-2-[4-(chinolin-2-yl-methoxy)phenyl]-2-cyclopentyl-essigsäureamid und
N-Ethyl-2-[4-(chinolin-2-yl-methoxy)phenyl]-2-cyclohexyl-essigsäureamid
ausgenommen sind.

Die erfindungsgemäßen substituierten 4-(Chinolin-2-yl-methoxy-phenyl)-essigsäurederivate können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe oder einen Tetrazolylrest besitzen, sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Heterocyclus steht im allgemeinen für einen 5- bis 6-gliedrigen, gesättigten, partiell ungesättigten oder ungesättigten Ring, der als Heteroatome bis zu 3 Sauerstoff-, Schwefel- und/oder Stickstoffatome enthalten kann. Bevorzugt sind 5- und 6-gliedrige Ringe mit einem Sauerstoff-, Schwefel und/oder bis zu 2 Stickstoffatomen. Bevorzugt werden genannt: Thienyl, Furyl, Pyrrolyl, Pyrazolyl, Pyridyl, Pyrimidyl, Pyrazinyl, Pyridazinyl, Thiazolyl, Oxazolyl, Imidazolyl, Pyrrolidinyl, Piperidinyl, Piperazinyl.

Ein 5- bis 6-gliedriger, gesättigter Heterocyclus, der außerdem als Heteroatome bis zu 3 Sauerstoff-, Schwefel- und/oder Stickstoffatome enthalten kann, steht im allgemeinen für Piperidyl, Morpholinyl, Piperazinyl oder Pyrrolidyl. Bevorzugt ist Morpholinyl.

Carbocyclus steht im allgemeinen für einen 3- bis 7-gliedrigen, bevorzugt 5- bis 7-gliedrigen gesättigten Kohlenwasserstoffring. Bevorzugt werden genannt Cyclopentyl, Cyclohexyl oder Cycloheptyl.

Hydroxyschutzgruppe im Rahmen der oben angegebenen Definition steht im allgemeinen für eine Schutzgruppe aus der Reihe: tert.-Butoxydiphenylsilyl, Trimethylsilyl, Triethylsilyl, Triisopropylsilyl, tert.-Butyl-dimethylsilyl, tert.-Butyldiphenylsilyl, Triphenylsilyl, Trimethylsilylethoxycarbonyl, Benzyl, Benzyloxycarbonyl, 2-Nitrobenzyl, 4-Nitrobenzyl, 2-Nitrobenzyloxycarbonyl, 4-Nitrobenzyloxycarbonyl, tert.-Butyloxycarbonyl, Allyloxycarbonyl, 4-Methoxybenzyl, 4-Methoxybenzyloxycarbonyl, Formyl, Acetyl, Trichloracetyl, 2,2,2-Trichlorethoxycarbonyl, 2,4-Dimethoxybenzyl, 2,4-Dimethoxybenzyloxycarbonyl, Methylthiomethyl, Methoxyethoxymethyl, [2-(Trimethylsilyl)ethoxy]methyl, 2-(Methylthiomethoxy)ethoxycarbonyl, Benzoyl, 4-Methylbenzoyl, 4-Nitrobenzoyl, 4-Fluorbenzoyl, 4-Chlorbenzoyl oder 4-Methoxybenzoyl. Bevorzugt sind Acetyl, Benzoyl, Benzyl oder Methylbenzyl.

Aminoschutzgruppe im Rahmen der Erfindung sind die üblichen in der Peptid-Chemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert.-Butoxycarbonyl, Allyloxycarbonyl, Vinyloxycarbonyl, 2-Nitrobenzyloxycarbonyl, 3,4,5-Trimethoxybenzyloxycarbonyl, Cyclohexoxycarbonyl, 1,1-Dimethylethoxycarbonyl, Adamantylcarbonyl, Phthaloyl, 2,2,2-Trichlorethoxycarbonyl, 2,2,2-Trichlor-tert.-butoxycarbonyl, Menthyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl oder 4-Nitrophenyl.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Diese Mischungen der Enantiomeren und Diastereomeren lassen sich in bekannter Weise in die stereoisomer einheitlichen Bestandteile trennen.

Beispielsweise können Diastereomere durch folgende Formeln dargestellt werden:

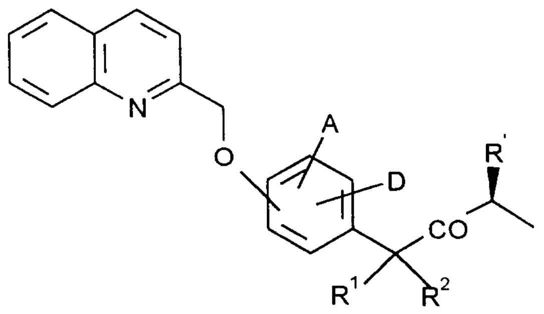

(Ia)

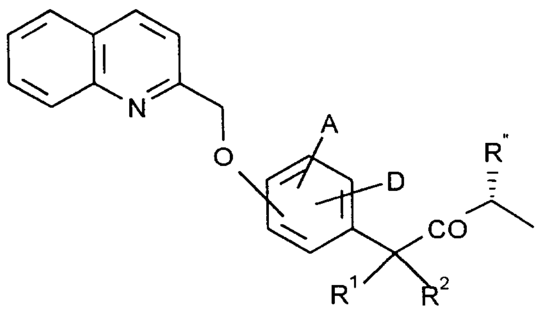

(Ib)

Bevorzugt sind Verbindungen der allgemeinen Formel (I), in welcher

A und D gleich oder verschieden sind und für Wasserstoff, Cyclopropyl, Cyclopentyl, Cyclohexyl, Azido, Hydroxy, Fluor, Chlor, Brom, geradkettiges oder verzweigtes Alkyl, Alkoxy oder Alkenyl mit jeweils bis zu 5 Kohlenstoffatomen, Pyrryl oder Imidazolyl stehen,

$R^1$ für Wasserstoff, Hydroxy oder für geradkettiges oder verzweigtes Alkyl mit bis zu 3 Kohlenstoffatomen steht,

$R^2$ für Wasserstoff, Hydroxy, Fluor, Chlor, Brom, geradkettiges oder verzweigtes Alkenyl oder Alkoxy mit jeweils bis zu 7 Kohlenstoffatomen, Cyclopentyl, Cyclohexyl, Cycloheptyl, Cyclooctyl, Cyclononyl, Cyclodecyl, Cycloundecyl oder Cyclododecyl steht, oder
für geradkettiges oder verzweigtes Alkyl mit bis zu 7 Kohlenstoffatomen steht, das gegebenenfalls durch Cyclopentyl, Cyclohexyl, Cycloheptyl, Cyclooctyl, Cyclononyl, Cyclodecyl, Cycloundecyl oder Cyclododecyl, Phenyl oder Tetrahydropyranyl substituiert ist, welche ihrerseits durch Fluor, Chlor oder Brom substituiert sein können, oder
für den Indanylrest steht,

oder

$R^1$ und $R^2$ gemeinsam mit dem Kohlenstoffatom einen Cyclopentyl-, Cyclohexyl- oder Cycloheptylring bilden,

oder

$R^1$ und $R^2$ gemeinsam für einen Doppelbindungs-Rest der Formel

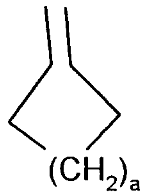

stehen,
worin

a eine Zahl 2, 3, 4 oder 5 bedeutet,

E für einen Rest der Formel

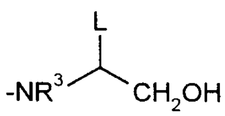
,
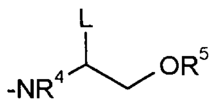
,
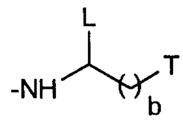
,

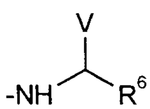
,
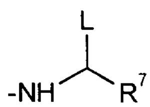
,
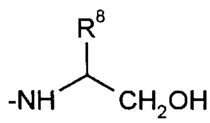
,

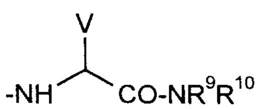
,

$-NR^{4'}-W-OH$,

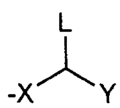
,

$-NH-R^{11}$ oder

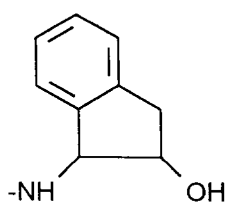

steht,

in welcher

| | |
|---|---|
| $R^3$ | Phenyl, Methyl, Acetyl oder tert. Butoxycarbonyl (Boc) bedeutet, |
| $R^4$ und $R^{4'}$ | gleich oder verschieden sind und die oben angegebene Bedeutung von $R^3$ haben oder Wasserstoff bedeuten, |
| $R^5$ | geradkettiges oder verzweigtes Acyl oder Alkyl mit jeweils bis zu 3 Kohlenstoffatomen bedeutet, |
| L | Phenyl, Benzyl oder Naphtyl bedeutet, die gegebenenfalls bis zu 2-fach gleich oder verschieden durch Fluor, Chlor, Brom, Iod, Hydroxy, Pyrrolidinyl, Morpholino, Amino, Trifluormethyl, Trifluormethoxy, Cyclopropyl, Cyclopentyl, Cyclohexyl, geradkettiges oder verzweigtes Alkyl oder Alkoxy mit jeweils bis zu 6 Kohlenstoffatomen substituiert sind, die ihrerseits durch Hydroxy substituiert sein können oder gegebenenfalls durch Phenyl oder Phenoxy substituiert sind, die ihrerseits bis zu 2-fach gleich oder verschieden durch Fluor, Chlor, Brom oder durch geradkettiges oder verzweigtes Alkyl mit bis zu 3 Kohlenstoffatomen substituiert sein können,<br>und/oder gegebenenfalls durch eine Gruppe der Formel $-(O)_cSO_2-R^{12}$ substituiert sind,<br>worin |
| c | eine Zahl 0 oder 1 bedeutet,<br>und |
| $R^{12}$ | geradkettiges oder verzweigtes Alkyl mit bis zu 4 Kohlenstoffatomen oder Phenyl bedeutet, |
| b | eine Zahl 0 oder 1 bedeutet, |

T einen heterocyclischen Rest der Formel

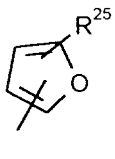 , 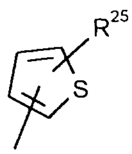 , 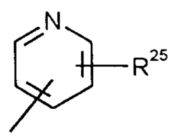 , 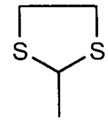 , 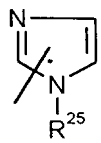 ,

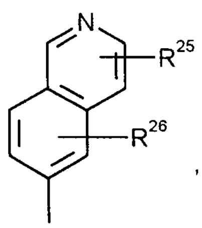 , 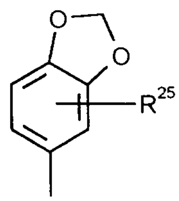 , 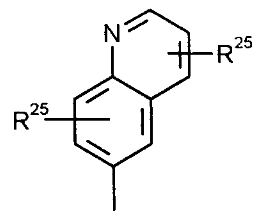 ,

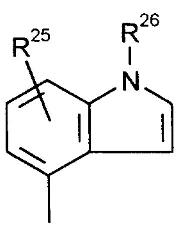 , 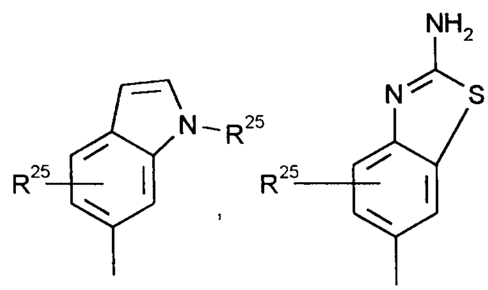 ,

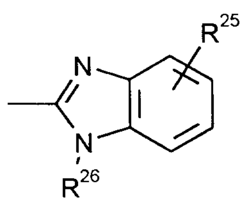 , 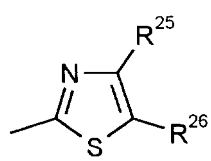 , 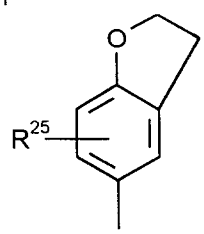 ,

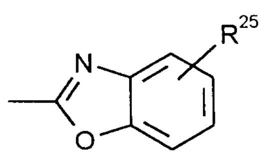

oder

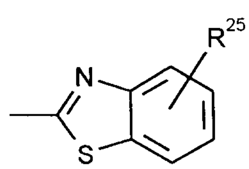

bedeutet

worin

$R^{25}$ und $R^{26}$ gleich oder verschieden sind und Wasserstoff, geradkettiges oder verzweigtes Alkyl mit bis zu 4 Kohlenstoffatomen oder Amino bedeuten,

V — die oben angegebene Bedeutung von L oder T hat oder einen Rest der Formel

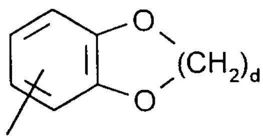

bedeutet, worin

d — eine Zahl 1 oder 2 bedeutet,

$R^6$ — einen Rest der Formel $-(CH_2)_d-R^{13}$ bedeutet, worin

e — eine Zahl 0 oder 1 bedeutet,

$R^{13}$ — Hydroxy, Carboxy oder geradkettiges oder verzweigtes Alkoxycarbonyl mit bis zu 3 Kohlenstoffatomen bedeutet,

$R^7$ — Wasserstoff, Cyano, Trifluormethyl, Vinyl, geradkettiges oder verzweigtes Alkyl mit bis zu 6 Kohlenstoffatomen bedeutet, das gegebenenfalls bis zu 2-fach gleich oder verschieden durch geradkettiges oder verzweigtes Alkoxy mit bis zu 3 Kohlenstoffatomen substituiert ist, oder Alkoxy mit bis zu 5 Kohlenstoffatomen bedeutet, oder eine Gruppe der Formel $-CO-NH-(CH_2)_f-NR^{14}R^{15}$ bedeutet, worin

f — eine Zahl 1, 2 oder 3 bedeutet,

$R^{14}$ und $R^{15}$ — gleich oder verschieden sind und Wasserstoff, Phenyl oder geradkettiges oder verzweigtes Alkyl mit bis zu 3 Kohlenstoffatomen bedeuten,

$R^8$ — Wasserstoff, Carboxy, geradkettiges oder verzweigtes Alkoxycarbonyl mit bis zu 3 Kohlenstoffatomen oder geradkettiges oder verzweigtes Alkyl mit bis zu 5 Kohlenstoffatomen bedeutet, das gegebenenfalls durch Hydroxy, Carboxy oder durch geradkettiges oder verzweigtes Alkoxycarbonyl mit jeweils bis zu 3 Kohlenstoffatomen oder Phenyl substituiert ist,

$R^9$ und $R^{10}$ — gleich oder verschieden sind und Wasserstoff, Cyclopropyl, Cyclopentyl, Cyclohexyl oder Phenyl bedeuten, oder geradkettiges oder verzweigtes Alkyl mit bis zu 5 Kohlenstoffatomen bedeuten, das gegebenenfalls durch Phenyl, Cyclopropyl, Cyclopentyl, Cyclohexyl, Hydroxy, Carboxy, durch geradkettiges oder verzweigtes Alkoxy oder Alkoxycarbonyl mit jeweils bis zu 3 Kohlenstoffatomen oder durch eine Gruppe der Formel $-NR^{16}R^{17}$ substituiert ist worin

$R^{16}$ und $R^{17}$ — gleich oder verschieden sind und Wasserstoff, Phenyl oder geradkettiges oder verzweigtes Alkyl mit bis zu 3 Kohlenstoffatomen bedeuten,

oder

$R^9$ und $R^{10}$ — gemeinsam mit dem Stickstoffatom einen Heterocyclus der Formel

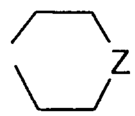

bilden,
worin

Z ein Sauerstoffatom oder die Gruppe $-NR^{18}$ oder -CH bedeutet,
worin

$R^{18}$ Wasserstoff, Acetyl, tert.-Butoxycarbonyl oder einen Rest der Formel $-SO_2R^{19}$ bedeutet,
worin

$R^{19}$ geradkettiges oder verzweigtes Alkyl mit bis zu 3 Kohlenstoffatomen, Benzyl oder Phenyl bedeutet, das gegebenenfalls durch Phenyl oder Tolyl substiuiert ist,

W geradkettiges oder verzweigtes Alkyl von 2 bis 7 Kohlenstoffatomen bedeutet, das 1- bis 3-fach gleich oder verschieden durch Hydroxy, Pyridyl, Norbornyl oder Phenyl substituiert ist, das seinerseits durch Hydroxy oder Benzyloxy substituiert sein kann, oder durch eine Gruppe der Formel $-OR^{20}$ oder $NR^{21}R^{22}$ substituiert ist,
worin

$R^{20}$ geradkettiges oder verzweigtes Alkyl mit bis zu 5 Kohlenstoffatomen bedeutet, und

$R^{21}$ und $R^{22}$ gleich oder verschieden sind und die oben angegebene Bedeutung von $R^{16}$ und $R^{17}$ haben,

X ein Sauerstoff- oder Schwefelatom bedeutet,

Y Formyl oder die Gruppe $-CHR^{23}R^{24}$ bedeutet,
worin

$R^{23}$ Wasserstoff bedeutet,

$R^{24}$ Hydroxy oder geradkettiges oder verzweigtes Alkoxy oder Acyl mit jeweils bis zu 3 Kohlenstoffatomen bedeutet,
oder

$R^{23}$ und $R^{24}$ gleich oder verschieden sind und geradkettiges oder verzweigtes Alkoxy mit bis zu 3 Kohlenstoffatomen bedeuten,

$R^{11}$ einen Rest der Formel

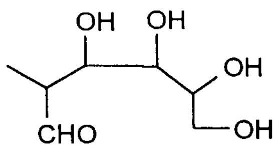 , 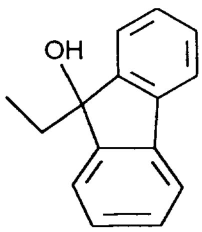 , 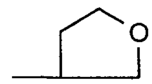

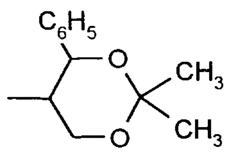 , 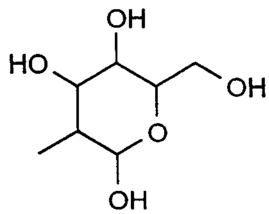

oder

Phenyl bedeutet, das gegebenenfalls bis zu 2-fach gleich oder verschieden durch Fluor, Chlor, Brom, Carboxy oder geradkettiges oder verzweigtes Alkoxycarbonyl mit bis zu 4 Kohlenstoffatomen substituiert ist,

oder

geradkettiges oder verzweigtes Alkyl mit bis zu 5 Kohlenstoffatomen bedeutet, das gegebenenfalls bis zu 2-fach gleich oder verschieden durch Hydroxy, Carboxy, Fluor, Chlor, Brom oder durch geradkettiges oder verzweigtes Alkoxycarbonyl mit bis zu 4 Kohlenstoffatomen substituiert ist,

und deren Salze,
wobei
2-[4-(Chinolin-2-yl-methoxy)phenyl]-2-cyclopentyl-essigsäuremethyloxycarbonylmethylamid,
2-[4-(Chinolin-2-yl-methoxy)phenyl]-2-cyclopenryl-essigsäurecarboxymethylamid,
N-Methyl-2-[3-isobutyl-4-(chinolin-2-yl-methoxy)phenyl]2-cycloheptylacetamid,
N-Methyl-2-[4-(chinolin-2-yl-methoxy)phenyl]-2-cycloheptyl-essigsäureamid,
N-Ethyl-2-[4-(chinolin-2-yl-methoxy)phenyl]-2-cyclopentyl-essigsäureamid und
N-Ethyl-2-[4-(chinolin-2-yl-methoxy)phenyl]-2-cyclohexyl-essigsäureamid ausgenommen sind.

Besonders bevorzugt sind Verbindungen der allgemeinen Formel (I), in welcher

A und D gleich oder verschieden sind und für Wasserstoff, Cyclopropyl, Cyclopentyl, Cyclohexyl, Azido, Hydroxy, Fluor, Chlor, Brom, geradkettiges oder verzweigtes Alkyl, Alkoxy oder Alkenyl mit jeweils bis zu 4 Kohlenstoffatomen, Imidazolyl oder Pyrryl stehen,

$R^1$ für Wasserstoff, Hydroxy oder geradkettiges oder verzweigtes Alkyl mit bis zu 3 Kohlenstoffatomen steht,

$R^2$ für Wasserstoff, Hydroxy, Fluor, Chlor, geradkettiges oder verzweigtes Alkenyl oder Alkoxy mit jeweils bis zu 5 Kohlenstoffatomen, Cyclopentyl, Cyclohexyl, Cycloheptyl, Cyclooctyl, Cyclononyl, Cyclodecyl oder Cycloundecyl steht, oder
für geradkettiges oder verzweigtes Alkyl mit bis zu 6 Kohlenstoffatomen steht, das gegebenenfalls durch Cyclopentyl, Cyclohexyl, Cycloheptyl, Cyclooctyl, Cyclononyl, Cycloundecyl, Phenyl oder Tetrahydropyranyl substituiert ist, welche ihrerseits durch Fluor, Chlor oder Brom substituiert sein können, oder
für den Indanylrest steht,

$R^1$ und $R^2$ gemeinsam mit dem Kohlenstoffatom einen Cyclopentyl-, Cyclohexyl- oder Cycloheptylring bilden,

E für einen Rest der Formel

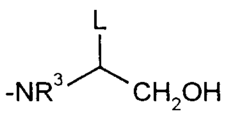 , 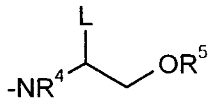 , 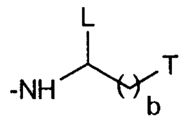 ,

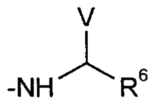 , 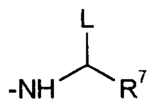 , 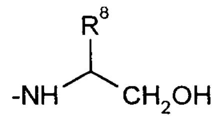 ,

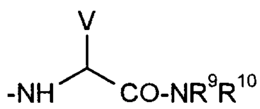 ,

$-NR^{4'}-W-OH$,

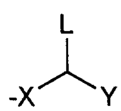 ,

$-NH-R^{11}$ oder

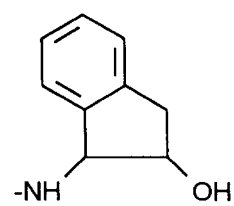

steht,

in welcher

$R^3$ Phenyl, Methyl, Acetyl oder tert. Butoxycarbonyl (Boc) bedeutet,

$R^4$ und $R^{4'}$ gleich oder verschieden sind und die oben angegebene Bedeutung von $R^3$ haben oder Wasserstoff bedeuten,

$R^5$ geradkettiges oder verzweigtes Acyl oder Alkyl mit jeweils bis zu 3 Kohlenstoffatomen bedeutet,

L Phenyl, Benzyl oder Naphthyl bedeutet, die gegebenenfalls bis zu 2-fach gleich oder verschieden durch Fluor, Chlor, Brom, Iod, Hydroxy, Pyrrolidinyl, Morpholino, Trifluormethoxy, Trifluormethyl, Amino, Cyclopropyl, Cyclopentyl, Cyclohexyl, geradkettiges oder verzweigtes Alkyl oder Alkoxy mit jeweils bis zu 6 Kohlenstoffatomen substituiert sind, die ihrerseits durch Hydroxy substituiert sein können, oder gegebenenfalls durch Phenyl oder Phenoxy substituiert sind, die ihrerseits bis zu 2-fach gleich oder verschieden durch Fluor, Chlor oder durch geradkettiges oder verzweigtes

Alkyl mit bis zu 3 Kohlenstoffatomen substituiert sein können, und/oder gegebenenfalls durch eine Gruppe der Formel $-(O)_cSO_2-R^{12}$ substituiert sind, worin

c eine Zahl 0 oder 1 bedeutet, und

$R^{12}$ geradkettiges oder verzweigtes Alkyl mit bis zu 3 Kohlenstoffatomen oder Phenyl bedeutet,

b eine Zahl 0 oder 1 bedeutet,

T einen heterocyclischen Rest der Formel

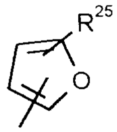 , 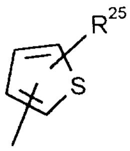 , 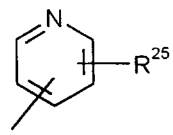 , 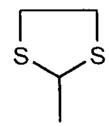 , 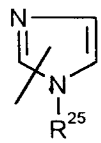 ,

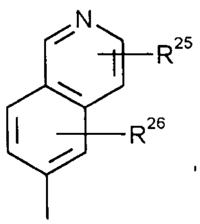 , 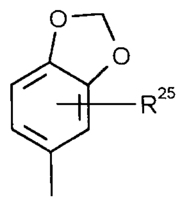 , 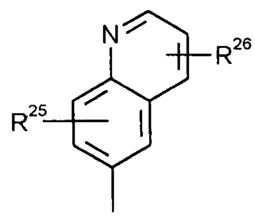 ,

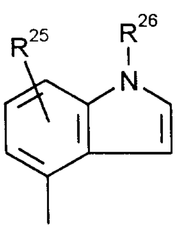 , 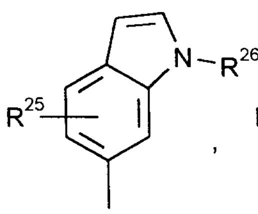 , 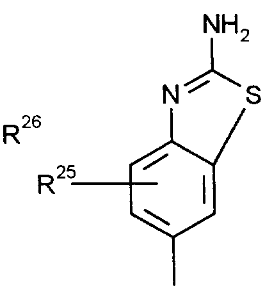 ,

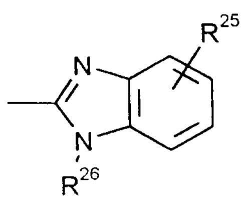 , 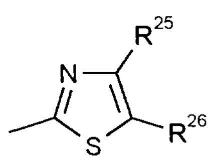 , 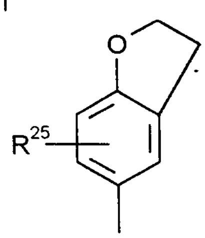 ,

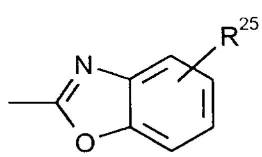

oder

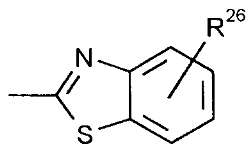

bedeutet
worin

$R^{25}$ und $R^{26}$ gleich oder verschieden sind und Wasserstoff, geradkettiges oder verzweigtes Alkyl mit bis zu 4 Kohlenstoffatomen oder Amino bedeuten,

V die oben angegebene Bedeutung von L oder T hat, oder
einen Rest der Formel

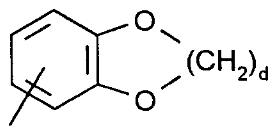

bedeutet,
worin

d eine Zahl 1 oder 2 bedeutet,

$R^6$ einen Rest der Formel $-(CH_2)_d-R^{13}$ bedeutet,
worin

e eine Zahl 0 oder 1 bedeutet,

$R^{13}$ Hydroxy, Carboxy oder geradkettiges oder verzweigtes Alkoxycarbonyl mit bis zu 3 Kohlenstoffatomen bedeutet,
wobei

$R^7$ Wasserstoff, Cyano, Trifluormethyl, Vinyl, geradkettiges oder verzweigtes Alkyl mit bis zu 5 Kohlenstoffatomen bedeutet, das gegebenenfalls bis zu 2-fach gleich oder verschieden durch geradkettiges oder verzweigtes Alkoxy mit bis zu 3 Kohlenstoffatomen substituiert ist, oder Alkoxy mit bis zu 3 Kohlenstoffatomen bedeutet, oder
eine Gruppe der Formel $-CO-NH-(CH_2)_f-NR^{14}R^{15}$ bedeutet,
worin

f eine Zahl 1, 2 oder 3 bedeutet,

$R^{14}$ und $R^{15}$ gleich oder verschieden sind und
Wasserstoff, Phenyl oder geradkettiges oder verzweigtes Alkyl mit bis zu 3 Kohlenstoffatomen bedeuten,

$R^8$ Wasserstoff, Carboxy, geradkettiges oder verzweigtes Alkoxycarbonyl mit bis zu 3 Kohlenstoffatomen oder geradkettiges oder verzweigtes Alkyl mit bis zu 4 Kohlenstoffatomen bedeutet, das gegebenenfalls durch Hydroxy, Carboxy oder durch geradkettiges oder verzweigtes Alkoxycarbonyl mit jeweils bis zu 3 Kohlenstoffatomen oder Phenyl substituiert ist,

$R^9$ und $R^{10}$ gleich oder verschieden sind und Wasserstoff, Cyclopropyl, Cyclopentyl, Cyclohexyl oder Phenyl bedeuten, oder geradkettiges oder verzweigtes Alkyl mit bis zu 5 Kohlenstoffatomen bedeuten, das gegebenenfalls durch Phenyl, Cyclopropyl, Cyclopentyl, Cyclohexyl, Hydroxy, Carboxy, durch geradkettiges oder verzweigtes Alkoxy oder Alkoxycarbonyl mit jeweils bis zu 3 Kohlenstoffatomen oder durch eine Gruppe der Formel $-NR^{16}R^{17}$ substituiert ist
worin

$R^{16}$ und $R^{17}$ gleich oder verschieden sind und Wasserstoff, Phenyl oder geradkettiges oder verzweigtes Alkyl mit bis zu 3 Kohlenstoffatomen bedeuten,

oder

$R^9$ und $R^{10}$ gemeinsam mit dem Stickstoffatom einen Heterocyclus der Formel

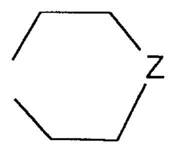

bilden,
worin

Z ein Sauerstoffatom oder die Gruppe $-NR^{18}$ oder -CH bedeutet,
worin

$R^{18}$ Wasserstoff, Acetyl, tert.-Butoxycarbonyl oder einen Rest der Formel $-SO_2R^{19}$ bedeutet,
worin

$R^{19}$ geradkettiges oder verzweigtes Alkyl mit bis zu 3 Kohlenstoffatomen, Benzyl oder Phenyl bedeutet, das gegebenenfalls durch Phenyl oder Tolyl substiuiert ist,

W geradkettiges oder verzweigtes Alkyl von 2 bis 7 Kohlenstoffatomen bedeutet, das 1- bis 3-fach gleich oder verschieden durch Hydroxy, Pyridyl, Norbornyl oder Phenyl substituiert ist, das seinerseits durch Hydroxy oder Benzyloxy substituiert sein kann, oder durch eine Gruppe der Formel $-OR^{20}$ oder $-NR^{21}R^{22}$ substituiert ist,
worin

$R^{20}$ geradkettiges oder verzweigtes Alkyl mit bis zu 4 Kohlenstoffatomen bedeutet und

$R^{21}$ und $R^{22}$ gleich oder verschieden sind und die oben angegebene Bedeutung von $R^{16}$ und $R^{17}$ haben,

X ein Sauerstoff- oder Schwefelatom bedeutet,

Y Formyl oder die Gruppe $-CHR^{23}R^{24}$ bedeutet,
worin

$R^{23}$ Wasserstoff bedeutet,

$R^{24}$ Hydroxy oder geradkettiges oder verzweigtes Alkoxy oder Acyl mit jeweils bis zu 3 Kohlenstoffatomen bedeutet,
oder

$R^{23}$ und $R^{24}$ gleich oder verschieden sind und geradkettiges oder verzweigtes Alkoxy mit bis zu 3 Kohlenstoffatomen bedeuten,

$R^{11}$ einen Rest der Formel

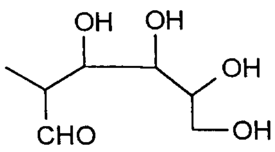 , 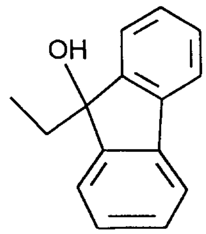 , 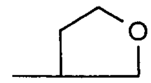

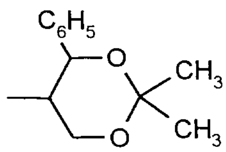 , 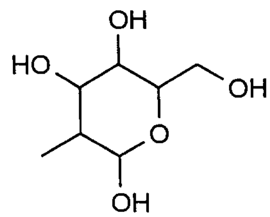

oder
Phenyl bedeutet, das gegebenenfalls bis zu 2-fach gleich oder verschieden durch Fluor, Chlor, Brom, Carboxy oder geradkettiges oder verzweigtes Alkoxycarbonyl mit bis zu 3 Kohlenstoffatomen substituiert ist,
oder
geradkettiges oder verzweigtes Alkyl mit bis zu 5 Kohlenstoffatomen bedeutet, das gegebenenfalls bis zu 2-fach gleich oder verschieden durch Hydroxy, Carboxy, Fluor, Chlor, Brom oder durch geradkettiges oder verzweigtes Alkoxycarbonyl mit bis zu 3 Kohlenstoffatomen substituiert ist,

und deren Salze,
wobei
2-[4-(Chinolin-2-yl-methoxy)phenyl]-2-cyclopentyl-essigsäuremethyloxycarbonylmethylamid,
2-[4-(Chinolin-2-yl-methoxy)phenyl]-2-cyclopentyl-essigsäurecarboxymethylamid,
N-Methyl-2-[3-isobutyl-4-(chinolin-2-yl-methoxy)phenyl]2-cycloheptylacetamid,
N-Methyl-2-[4-(chinolin-2-yl-methoxy)phenyl]-2-cycloheptyl-essigsäureamid,
N-Ethyl-2-[4-(chinolin-2-yl-methoxy)phenyl]-2-cyclopentyl-essigsäureamid und
N-Ethyl-2-[4-(chinolin-2-yl-methoxy)phenyl]-2-cyclohexyl-essigsäureamid ausgenommen sind.

Außerdem wurden Verfahren zur Herstellung der erfindungsgemäßen Verbindungen der allgemeinen Formel (I) gefunden, dadurch gekennzeichnet, daß man
[A] im Fall, daß E nicht für den Rest der Formel

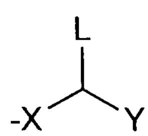

steht,
Carbonsäuren der allgemeinen Formel (II)

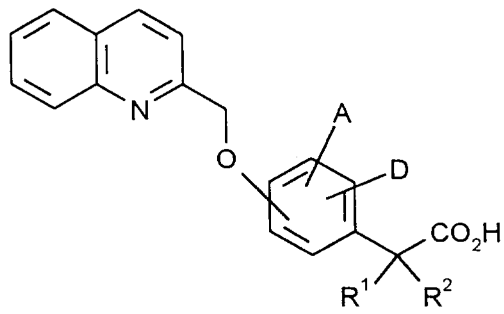

(II)

in welcher

A, D, $R^1$ und $R^2$ die angegebene Bedeutung haben,

gegebenenfalls unter vorgeschalteter Aktivierung der Carbonsäurefunktion, mit Glycinolen oder deren Ester der allgemeinen Formel (III)

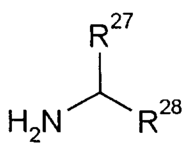

(III)

in welcher

$R^{27}$ den oben angegebenen Bedeutungsumfang von L, V, W und $R^8$ umfaßt

und

$R^{28}$ für die $-CH_2-OH$ oder für $(C_1-C_8)$-Alkoxycarbonyl steht,

in inerten Lösemitteln, in Anwesenheit einer Base und gegebenenfalls eines Hilfsstoffes umsetzt, und
[B] im Fall, daß E für den Rest der Gruppe

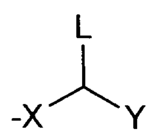

steht,
die Carbonsäuren der allgemeinen Formel (II), nach vorgeschalteter Aktivierung der Carbonsäurefunktion, mit Verbindungen der allgemeinen Formel (IV)

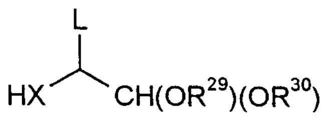

(IV)

in welcher

X und L die oben angegebene Bedeutung haben,

$R^{29}$ und $R^{30}$ gleich oder verschieden sind und $C_1$-$C_6$-Alkyl bedeuten,
in inerten Lösemitteln, gegebenenfalls in Anwesenheit einer Base und/oder Hilfsmittels, umsetzt,

und im Fall Y = CHO eine Oxidation anschließt,
und in Abhängigkeit der jeweiligen Definition des oben aufgeführten Substituenten E gegebenenfalls eine Acylierung, Reduktion, Verseifung, Amidierung, Alkylierung, Sulfoamidierung und/oder Eliminierung nach üblichen Methoden durchführt.

Das erfindungsgemäße Verfahren kann durch folgendes Formelschema beispielhaft erläutert werden:

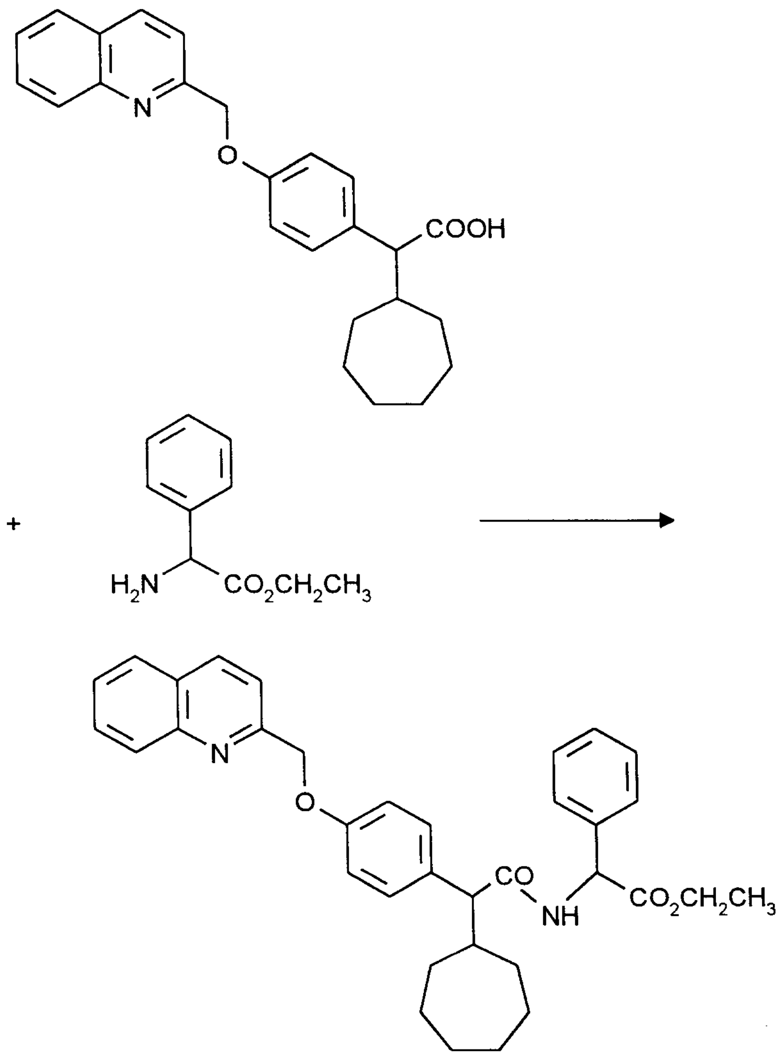

Als Lösemittel eignen sich hierbei inerte organische Lösemittel, die sich unter den Reaktionsbedingungen nicht verändern. Hierzu gehören Ether, wie Diethylether oder Tetrahydrofuran, Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohenwasserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt sind Dichlormethan, Tetrahydrofuran, Aceton oder Dimethylformamid.

Als Basen eignen sich die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliumethanolat, oder Natrium- oder Kaliummethanolat, oder organische Amine wie Triethylamin, Picolin oder N-Methylpiperidin, oder Amide wie Natriumamid oder Lithiumdiisopropylamid, oder metallorganische Verbindungen wie Butyllithium oder Phenyllithium. Bevorzugt sind Natrium- und Kaliumcarbonat und Triethylamin.

Die Base wird in einer Menge von 0,6 mol bis 5 mol, bevorzugt von 0,7 mol bis 2 mol, bezogen auf 1 mol der Verbindung der allgemeinen Formel (II) eingesetzt.

Die Reaktion wird im allgemeinen in einem Temperaturbereich von 0°C bis 150°C, bevorzugt von +20°C bis +110°C durchgeführt.

Die Umsetzung kann bei normalen, erhöhtem oder bei erniedrigtem Druck durchgeführt werden (z.B. 0,5 bis 5 bar). Im allgemeinen arbeitet man bei Normaldruck.

Zur Aktivierung der Carbonsäurefunktion eignen sich im allgemeinen Basen und/oder Dehydratisierungsreagenzien wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexfluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Die Aktivierung der Carbonsäurefunktion erfolgt im allgemeinen in einem Temperaturbereich von 0°C bis 150°C, bevorzugt von 0°C bis 80°C und gegebenenfalls unter Schutzgasatmosphäre.

Die Alkylierung erfolgt im allgemeinen mit Alkylierungsmitteln wie beispielsweise ($C_1$-$C_8$)-Alkylhalogeniden, Sulfonsäurestern oder substituierten oder unsubstituierten ($C_1$-$C_8$)-Dialkyl- oder ($C_1$-$C_8$)-Diarylsulfonate, vorzugsweise Methyliodid oder Dimethylsulfat.

Die Alkylierung erfolgt im allgemeinen in einem der oben aufgeführten Lösemitteln, vorzugsweise in Dimethylformamid in einem Temperaturbereich von 0°C bis +70°C, vorzugsweise von 0°C bis +30°C und unter Normaldruck.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung kann gegebenenfalls auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters, eingesetzt. Besonders bevorzugt verwendet man molare Mengen der Reaktanden.

Die Verseifung von tert.-Butylestern erfolgt im allgemeinen mit Säuren, wie beispielsweise Salzsäure oder Trifluoressigsäure, in Anwesenheit eines der oben angegebenen Lösemitteln und/oder Wasser oder deren Gemische, vorzugsweise mit Dioxan oder Tetrahydrofuran.

Die Oxidation im Fall Y = CHO erfolgt im allgemeinen in einem der oben aufgeführten Ether, vorzugsweise Dioxan und in Anwesenheit einer Säure. Hierzu gehören bevorzugt anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit $C_1$-$C_4$-Alkylresten oder Arylresten wie beispielsweise Methansulfonsäure, Ethansulfonsäure, Benzolsulfonsäure oder Toluolsulfonsäure. Bevorzugt ist Salzsäure.

Die Oxidation wird im allgemeinen in einem Temperaturbereich von 0°C bis +100°C, bevorzugt von +20°C bis +80°C durchgeführt.

Im allgemeinen wird die Oxidation bei Normaldruck durchgeführt. Es ist aber auch möglich, bei Unterdruck oder bei Überdruck zu arbeiten (z.B. von 0,5 bis 5 bar).

Die Amidierung und die Sulfonamidierung erfolgen im allgemeinen in einem der oben aufgeführten Lösemitteln, vorzugsweise in Tetrahydrofuran oder Dichlormethan.

Die Amidierung und die Sulfonamidierung können gegebenenfalls über die aktivierte Stufe der Säurehalogenide, die aus den entsprechenden Säuren durch Umsetzung mit Thionylchlorid, Phosphortrichlorid, Phosphorpentachlorid, Phosphortribromid oder Oxalylchlorid hergestellt werden können, verlaufen.

Die Amidierung und die Sulfonamidierung erfolgt im allgemeinen in einem Temperaturbereich von -20°C bis +80°C, vorzugsweise von -10°C bis +30°C und unter Normaldruck.

Als Basen eignen sich dafür neben den oben aufgeführten Basen vorzugsweise Triethylamin und/oder Dimethylaminopyridin, DBU oder DABCO.

Die Base wird in einer Menge von 0,5 mol bis 10 mol, bevorzugt von 1 mol bis 2mol, bezogen auf 1 mol der entsprechenden Säure oder Esters, eingesetzt.

Als säurebindende Mittel für die Sulfonamidierung können Alkali- oder Erdalkalicarbonate wie Natriumcarbonat, Kaliumcarbonat, Alkali- oder Erdalkalihydroxide wie beispielsweise Natrium- oder Kaliumhydroxid, oder organische Basen wie Pyridin, Triethylamin, N-Methylpiperidin, oder bicyclische Amidine wie 1,5-Diazabicyclo[3.4.0]-noneni-5 (DBN) oder 1,5-Diazabicyclo[3.4.0]undeceni-5 (DBU) eingesetzt werden. Bevorzugt ist Kaliumcarbonat.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexfluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid oder N-Hydroxysuccinimid.

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Als Lösemittel eignen sich hierbei alle inerten Lösemittel, die sich unter den Reaktionsbedingungen nicht verändern. Hierzug gehören bevorzugt Alkohole wie Methanol, Ethanol, Propanol oder Isopropanol, oder Ether wie Diethylenglykoldimethylether oder Amide wie Hexamethylphosphorsäuretriamid oder Dimethylformamid, oder Essigsäure. Ebenso ist es möglich, Gemische der genannten Lösemittel zu verwenden.

Die Reduktionen können im allgemeinen durch Wasserstoff in Wasser oder in inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle, Platin, oder mit Hydriden oder Boranen in inerten Lösemitteln, gegebenenfalls in Anwesenheit eines Katalysators durchgeführt werden.

Bevorzugt wird die Reaktion mit Hydriden, wie komplexen Borhydriden oder Aluminiumhydriden durchgeführt. Besonders bevorzugt werden hierbei Natriumborhydrid, Lithiumaluminiumhydrid oder Natriumcyanoborhydrid eingesetzt.

Die Reaktion wird im allgemeinen in einem Temperaturbereich von 0°C bis 150°C, bevorzugt von +20°C bis +110°C durchgeführt.

Die Umsetzung kann bei normalen, erhöhtem oder bei erniedrigtem Druck durchgeführt werden (z.B. 0,5 bis 5 bar). Im allgemeinen arbeitet man bei Normaldruck.

Die Acylierung erfolgt im allgemeinen in einem der oben aufgeführten Lösemittel, vorzugsweise Methylenchlorid und in Anwesenheit eines typischen Acylierungsmittels, wie beispielsweise Acetylchlorid.

Die Acylierung wird im allgemeinen in einem Temperaturbereich von 0°C bis 150°C, bevorzugt von +20°C bis +110°C durchgeführt.

Die Acyclierung kann bei normalen, erhöhtem oder bei erniedrigtem Druck durchgeführt werden (z.B. 0,5 bis 5 bar). Im allgemeinen arbeitet man bei Normaldruck.

Die Eliminierung erfolgt im allgemeinen in einem der oben aufgeführten Lösemitteln, in Anwesenheit einer Base und Hilfsmittels. Bevorzugt sind Dimethylformamid, Methylchlorid und Triethylamin.

Die Eliminierung wird im allgemeinen in einem Temperaturbereich von 0°C bis 150°C, bevorzugt von +20°C bis +110°C durchgeführt.

Die Eliminierung kann bei normalen, erhöhtem oder bei erniedrigtem Druck durchgeführt werden (z.B. 0,5 bis 5 bar). Im allgemeinen arbeitet man bei Normaldruck.

Die Verbindungen der allgemeinen Formel (II) sind bekannt (vgl. US 4 929 629, 4 970 215, 5 091 392, 5 126 354, EP 414 078, 529 450) oder können hergestellt werden, indem man

Verbindungen der allgemeinen Formel (V)

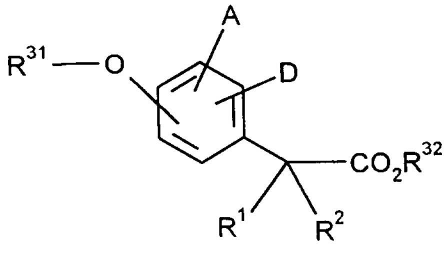

(V)

in welcher

A, D, $R^1$ und $R^2$ die oben angegebene Bedeutung haben,

$R^{31}$ für eine typische Hydroxyschutzgruppe, bevorzugt für Benzyl oder tert.-Butyl steht,

und

$R^{32}$ für Wasserstoff oder für ($C_1$-$C_4$)-Alkyl steht,

nach Abspaltung dieser Schutzgruppe nach üblichen Methoden mit Verbindungen der allgemeinen Formel (VI)

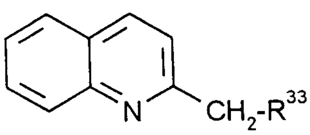

(VI)

in welcher

$R^{33}$ für Halogen, vorzugsweise Brom, steht,

in inerten Lösemitteln, gegebenenfalls in Anwesenheit einer Base, umsetzt,
und im Fall der Säuren die Ester verseift.

Lösemittel für die Veretherung können inerte organische Lösemittel sein, die sich unter den Reaktionsbedingungen nicht verändern. Hierzu gehören vorzugsweise Ether wie beispielsweise Dioxan, Tetrahydrofuran oder Diethylether, Halogenkohlenwasserstoffe wie Dichlormethan, Trichlormethan, Tetrachlormethan, 1,2-Dichlorethan oder Trichlorethylen, Kohlenwasserstoffe wie Benzol, Xylol, Toluol, Hexan, Cyclohexan oder Erdölfraktionen, Nitromethan, Dimethylformamid, Acetonitril, Aceton oder Hexamethylphosphorsäuretriamid. Ebenso ist es möglich, Gemische dieser Lösemittel einzusetzen.

Als Basen für die Veretherung können anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder organische Amine (Trialkyl($C_1$-$C_6$)-amine) wie Triethylamin, oder Heterocyclen wie Pyridin, Methylpiperidin, Piperidin oder Morpholin.

Es ist auch möglich, als Basen Alkalimetalle wie Natrium und deren Hydride, wie Natriumhydrid, einzusetzen.

Die Veretherung erfolgt im allgemeinen in einem Temperaturbereich von 0°C bis +150°C, bevorzugt von +10°C bis +100°C.

Die Veretherung wird im allgemeinen bei Normaldruck durchgeführt. Es ist aber auch möglich, das Verfahren bei Unterdruck oder Überdruck durchzuführen (z.B. in einem Bereich von 0,5 bis 5 bar).

Im allgemeinen setzt man 0,5 bis 5, bevorzugt 1 bis 2 mol Halogenid, bezogen auf 1 mol des Reaktionspartners, ein. Die Base wird im allgemeinen in einer Menge von 0,5 bis 5 mol, bevorzugt von 1 bis 3 mol, bezogen auf das Halogenid, eingesetzt.

Die Verseifung der Carbonsäureester erfolgt nach üblichen Methoden, indem man die Ester in inerten Lösemitteln mit üblichen Basen behandelt.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die Verseifung Wasser oder die für eine Verseifing üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C, durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol, bezogen auf 1 mol des Esters, eingesetzt. Besonders bevorzugt verwendet man molare Mengen der Reaktanden.

Die Verbindungen der allgmeinen Formel (III) und (IV) sind bekannt.

Die Verbindungen der allgemeinen Formeln (V) und (VI) sind bekannt.

Die erfindungsgemäßen Verbindungen der allgemeinen Formel (I) haben ein nicht vorhersehbares pharmakologisches Wirkspektrum.

Sie können als Wirkstoffe in Arzneimitteln zur Reduzierung von Veränderungen an Gefäßwänden Verwendung finden und zur Behandlung von Koronaren Herzerkrankungen, Herzinsuffizienz, Störungen der Hirnleistung, ischämischen Gehirnerkrankungen, Apoplex, Durchblutungsstörungen, Mikrozirkulationsstörungen und Thrombosen.

Weiterhin spielt bei der Okklusion von Gefäßen die Proliferation glatter Muskelzellen eine ausschlaggebende Rolle. Die erfindungsgemäßen Verbindungen sind geeignet, diese Proliferation zu inhibieren und damit atherosklerotische Prozesse zu verhindern.

Die erfindungsgemäßen Verbindungen zeichnen sich durch eine Senkung der ApoB-100-assoziierten Lipoproteinen (VLDL und seiner Abbauprodukte, wie z.B. (LDL), des ApoB-100 der Triglyceriden und des Cholesterins aus. Damit besitzen sie wertvolle, im Vergleich um Stand der Technik überlegene pharmakologische Eigenschaften.

Überraschenderweise besteht die Wirkung der erfindungsgemäßen Verbindungen zunächst in einer Verminderung oder vollständigen Inhibierung der Bildung und/oder der Freisetzung von ApoB-100-assoziierten Lipoproteinen aus Leberzellen, was eine Senkung des VLDL-Plasmaspiegels zur Folge hat. Diese VLDL-Senkung muß mit einer Senkung der Plasmaspiegel von ApoB-100, LDL, Triglyceriden und von Cholesterin einhergehen; es werden also gleichzeitig mehrere der obengenannten Risikofaktoren gesenkt, die an Gefäßwandveränderungen beteiligt sind.

Die erfindungsgemäßen Verbindungen können deshalb zur Präventation und Behandlung von Atherosklerose, der Fettsucht, Pankreatitis und der Obstipation eingesetzt werden.

Die Erfindung betrifft außerdem die Kombination von heterocyclischem substituierten Oxy-phenyl-(phenyl)glycinolamiden der allgemeinen Formel (I) mit einem Glycosidase- und/oder Anylasehemmer zur Behandlung von familiären Hyperlipidämien, der Fettsucht (Adiposilas) und des Diabetes mellitus. Glucosidasen und/oder Arylasehemmer im Rahmen der Erfindung sind beispielsweise Acarbose, Adipisine, Voglibose (AO-128), Miglitol, Emiglitate, MDL-25637, Camiglibase (MDL-73945), Tendamistate, AI-3688, Trestatin, Pradimicin-Q und Salbostatine.

**1. Hemmung der Freisetzung ApoB-100-assoziierter Lipoproteine**

Der Test zum Nachweis der Hemmung der Freisetzung ApoB-100-assoziierter Lipoproteine aus Leberzellen erfolgte in vitro mit kultivierten Leberzellen, bevorzugt mit Zellen der humanen Linie HepG2. Diese Zellen werden unter Standardbedingungen in Medium für die Kultur eukariotischer Zellen gezüchtet, bevorzugt in RPMI 1640 mit 10% fötalem Kälberserum. HepG2-Zellen synthetisieren und sezernieren in den Kulturüberstand ApoB-100-assoziierte Lipoproteinpartikel, die im Prinzip ähnlich aufgebaut sind wie die VLDL- bzw. LDL-Partikel, die im Plasma zu finden sind.

Diese Partikel können mit einem Immunoassay für humanes LDL nachgewiesen werden. Dieser Immunoassay erfolgt mit Antikörpern, die im Kaninchen gegen humanes LDL unter Standardbedingungen induziert worden waren. Die anti-LDL-Antikörper (Kan-anti-LDL-Ak) wurden an einem Immunosorbens mit humanem LDL affinitätschromatographisch gereinigt. Diese gereinigten Kan-anti-LDL-Ak werden an die Oberfläche von Plastik adsorbiert. Zweckmäßigerweise erfolgt diese Adsorbtion an die Plastikoberfläche von Mikrotitierplatten mit 96 Vertiefungen, bevorzugt an MaxiSorp-Platten (Nunc). Wenn im Überstand von Hep-G2-Zellen ApoB-100-assoziierte Partikel vorhanden sind, dann können diese an die insolubilisierten Kan-anti-LDL-Ak binden, und es entsteht ein Immunkomplex, der an die Plastikoberfläche gebunden ist. Nicht gebundene Proteine werden durch Waschen entfernt. Der sich an der Plastikoberfläche befindliche Immunkomplex wird mit monoklonalen Antikörpern nachgewiesen, die nach Standardbedingungen gegen humanes LDL induziert und gereinigt worden waren. Diese Antikörper wurden mit dem Enzym Peroxidase (Boehringer, Mannheim) konjugiert. Peroxidase setzt das farblose Substrat TMB (Kirkegaard u. Perry) in Gegenwart von $H_2O_2$ in ein gefärbtes Produkt um. Nach Ansäuerung des Reaktionsgemisches mit $H_2SO_4$ wird die spezifische Lichtadsorption bei 450 nm bestimmt, die ein Maß für die Menge von ApoB-100-assoziierten Partikeln ist, die von den HepG2-Zellen in den Kulturüberstand sezerniert worden waren.

Überraschenderweise hemmen die erfindungsgemäßen Verbindungen die Freisetzung der ApoB-100-assoziierten Partikel. Der $IC_{50}$-Wert gibt an, bei welcher Substanzkonzentration die Lichtadsorption im Vergleich zur Kontrolle (Lösemittelkontrolle ohne Substanz) um 50% inhibiert ist.

Tabelle

| Bsp.-Nr. | $IC_{50}$[nM] |
|---|---|
| 15 | 500 |
| 53 | 250 |
| 127 | 130 |
| 141 | 20 |

**2. Untersuchung zur Inhibition der Proliferation glatter Muskelzellen**

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. 50, 172, 1971]. Die Zellen werden in geeigneten Kulturschalen, in der Regel 96-Loch-Platten, ausgesät und für 2 - 3 Tage in Medium 199 mit 7,5% FCS und 7,5% NCS, 2 mM L-Glutamin und 15 mM HEPES, pH 7,4 in 5% $CO_2$ bei 37°C kultiviert. Danach werden die Zellen durch Serumentzug für 2 - 3 Tage synchronisiert und sodann mit Serum oder anderen Faktoren zum Wachstum angeregt. Gleichzeitig werden Testverbindungen zugesetzt. Nach 16 - 20 Stunden wird 1 µCi $^3$H-Thymidin zugefügt und nach weiteren 4 Stunden der Einbau dieser Substanz in die TCA-präzipitierbare DNA der Zellen bestimmt. Zur Bestimmung der $IC_{50}$-Werte wird die Wirkstoffkonzentration errechnet, die bei sequentieller Verdünnung des Wirkstoffes halbmaximale Hemmung der durch 10% FCS hervorgerufene Thymidininkorporation bewirkt.

**3. Bestimmung der VLDL-Sekretion in vivo am Hamster**

Der Effekt der Testsubstanzen auf die VLDL-Sekretion in vivo wird am Hamster untersucht. Hierzu werden Goldhamster nach Prämedikation mit Atropin (83 mg/kg s.c.) mit Ketavet (83 mg/kg s.c.) und Nembutal (50 mg/kg i.p.) narkotisiert. Wenn die Tiere reflexfrei geworden sind, wird die V. jugularis freipräpariert und kanüliert. Anschließend werden 0,25 ml/kg einer 20%igen Lösung von Triton WR-1339 in physiologischer Kochsalzlösung appliziert. Dieses Detergens hemmt die Lipoproteinlipase und führt so zu einem Anstieg des Triglyceridspiegels aufgrund eines ausbleibenden Katabolismus von sezernierten VLDL-Partikeln. Dieser Triglyceridanstieg kann als Maß für die VLDL-Sekretionsrate herangezogen werden. Den Tieren wird vor sowie ein und zwei Stunden nach Applikation des Detergens durch Punktion des retroorbitalen Venenplexus Blut entnommen. Das Blut wird zwei Stunden bei Raumtemperatur, anschließend über Nacht bei 4°C inkubiert, um die Gerinnung vollständig abzuschließen. Danach wird 5 Minuten bei 10.000 g zentrifugiert. Im so erhaltenen Serum wird die Triglyceridkonzentration mit Hilfe eines modifizierten kommerziell erhältlichen Enzymtests bestimmt (Merckotest® Triglyceride Nr. 14354). 100 µl Serum werden mit 100 µl Testreagenz in 96-Lochplatten versetzt und 10 Minuten bei Raumtemperatur inkubiert. Anschließend wird die optische Dichte bei einer Wellenlänge von 492 nM in einem automatischen Platten-Lesegerät bestimmt (SLT-Spectra). Serumproben mit einer zu hohen Triglyceridkonzentration werden mit physiologischer Kochsalzlösung verdünnt. Die in den Proben enthaltene Triglyceridkonzentration wird mit Hilfe einer parallel gemessenen Standardkurve bestimmt. Testsubstanzen werden in diesem Modell entweder unmittelbar vor Applikation des Detergens intravenös verabreicht oder vor Einleitung der Narkose oral oder subkutan.

**4. Hemmung der intestinalen Triglyceridabsorption in vivo (Ratten)**

Die Substanzen, die auf ihre triglyceridabsorptionshemmende Wirkung in vivo untersucht werden sollen, werden männlichen Wistar-Ratten mit einem Körpergewicht zwischen 170 und 230 g oral verabreicht. Zu diesem Zweck werden die Tiere 18 Stunden vor der Substanzapplikation in Gruppen zu 6 Tieren eingeteilt und anschließend wird ihnen das Futter entzogen. Trinkwasser steht den Tieren ad libitum zur Verfügung. Die Tiere der Kontrollgruppe erhalten eine wäßrige Traganth-Suspension bzw. eine Traganth-Suspension, die Olivenöl enthält. Die Traganth-Olivenöl-Suspension wird mit dem Ultra-Turrax hergestellt. Die zu untersuchenden Substanzen werden in einer entsprechenden Traganth-Olivenöl-Suspension ebenfalls mit dem Ultra-Turrax, direkt vor der Substanzapplikation suspendiert.

Jeder Ratte wird vor der Schlundsondenapplikation zur Bestimmung des basalen Serumtriglyceridgehaltes Blut durch Punktion des retroorbitalen Venenplexus entnommen. Anschließend werden die Traganth-Suspensionen, die Traganth-Olivenöl-Suspensionen ohne Substanz (Kontrolltiere) bzw. die Substanzen, suspendiert in einer entsprechenden Traganth-Olivenöl-Suspension, den nüchternen Tieren mit einer Schlundsonde verabreicht. Die weiteren Blutentnahmen

zur Bestimmung des postprandialen Serumtriglyceridanstiegs erfolgen in der Regel 1, 2 und 3 Stunden nach der Schlundsondenapplikation.

Die Blutproben werden zentrifugiert und nach Gewinnung des Serums die Triglyceride photometrisch mit einem EPOS-Analyzer 5060 (Eppendorf Gerätebau, Netheler & Hinz GmbH, Hamburg) bestimmt. Die Bestimmung der Triglyceride erfolgt vollenzymatisch mit einem handelsüblichen UV-Test.

Der postprandiale Serumtriglyceridanstieg wird durch Substraktion des Triglyceridvorwertes jeden Tieres von seinen korrespondierenden postprandialen Triglyceridkonzentrationen (1, 2 und 3 Stunden nach Applikation) ermittelt.

Die Differenzen (in mmol/l) zu jedem Zeitpunkt (1, 2 und 3 Stunden) werden in den Gruppen gemittelt, und die Mittelwerte des Serumtriglyceridanstiegs (ΔTG) der substanzbehandelten Tiere mit den Tieren verglichen, die nur die Traganth-Öl-Suspension erhielten.

Die Differenzen (in mmol/l) zu jedem Zeitpunkt (1, 2 und 3 Stunden) werden in den Gruppen gemittelt, und die Mittelwerte des Serumtriglyceridanstiegs (ΔTG) der substanzbehandelten Tiere mit den Tieren verglichen, die nur die Traganth-Öl-Suspension erhielten.

Ebenso wird der Serumtriglyceridverlauf der Kontrolltiere, die nur Traganth erhielten, berechnet. Der Substanzeffekt zu jedem Zeitpunkt (1, 2 oder 3 Stunden) wird wie folgt ermittelt und in Δ% von der ölbelasteten Kontrolle angegeben.

$$\Delta\%\ \text{Triglycerid} = \frac{\Delta TG_{\text{Substanz}} - \Delta TG_{\text{Traganthkontrolle}}}{\Delta TG_{\text{Ölbelastung}} - \Delta TG_{\text{Traganthkontrolle}}} \times 100.$$

Effekt von 10 mg Prüfsubstanz / kg KG p.o. auf den Triglyceridanstieg (Δ%) 2 h nach einer Triglyceridbelastung im Serum nüchterner Ratten. Der Serumtriglyceridanstieg fettbelasteter Kontrolltiere bezogen auf den Serumtriglyceridspiegel von Traganth-Kontrolltieren entspricht 100%. n = 6 Tiere pro Gruppe.

Die statistische Auswertung erfolgt mit Student's t-Test nach vorheriger Überprüfung der Varianzen auf Homogenität.

Substanzen, die zu einem Zeitpunkt den postprandialen Serumtriglyceridanstieg, verglichen mit dem der unbehandelten Kontrollgruppe, statistisch signifikant ($p < 0,05$) um mindestens 30 % vermindern, werden als pharmakologisch wirksam angesehen.

**5. Hemmung der VLDL-Sekretion in vivo (Ratte)**

Die Wirkung der Testsubstanzen auf die VLDL-Sekretion wird ebenfalls an der Ratte untersucht. Dazu wird Ratten 500 mg/kg Körpergewicht (2,5 mg/kg) Triton WR-1339, gelöst in physiologischer Kochsalzlösung, intravenös in die Schwanzvene appliziert. Triton WR-1339 inhibiert die Lipoproteinlipase und führt somit durch Hemmung des VLDL-Katabolismus zu einem Anstieg des Triglycerid- und Cholsterinspiegels. Diese Anstiege können als Maß für die VLDL-Sekretionsrate herangezogen werden.

Den Tieren wird vor sowie eine und zwei Stunden nach Applikation des Detergens durch Punktion des retroorbitalen Venenplexus Blut entnommen. Das Blut wird zur Gerinnung 1 h bei Raumtemperatur inkubiert und das Serum durch Zentrifugation mit 10 000 g für 20 s gewonnen. Anschließend werden die Triglyceride mittels eines handelsüblichen gekoppelten Enzymtests (Sigma Diagnostics®, Nr. 339) bei einer Wellenlänge von 540 nm photometrisch bestimmt. Die Messung erfolgt mit Hilfe eines ebenfalls gekoppelten Enzymtests (Boehringer Mannheim®, Nr. 1442350) bei einer Wellenlänge von 546 nm. Proben mit Triglycerid- bzw. Cholesterinkonzentrationen, die den Meßbereich der Methoden überschreiten, werden mit physiologischer Kochsalzlösung verdünnt. Die Ermittlung der jeweiligen Serumkonzentrationen erfolgt anhand parallel gemessener Standardreihen. Testsubstanzen werden unmittelbar nach der Tritoninjektion oral, intravenös oder subcutan appliziert.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

**Definition der Isomerentypen:**

| | |
|---|---|
| 4dia = | Gemisch der vier möglichen Diastereomeren bei zwei Asymmetriezentren im Molekül |
| dia A = | Diastereomer mit dem größeren $R_f$-Wert |
| dia B = | Diastereomer mit dem kleineren $R_f$-Wert |
| ent = | Enantiomer |
| 2 ent dia = | Gemisch zweier enantiomerenreiner Diastereomere |
| ent dia A = | enantiomerenreines Diastereomer mit dem größeren $R_f$-Wert |
| ent dia B = | enantiomerenreines Diastereomer mit dem kleineren $R_f$-Wert |
| R = | R-Enantiomer |
| rac = | Racemat |
| rac dia A = | racemisches Diastereomer mit dem größeren $R_f$-Wert |
| rac dia B = | racemisches Diastereomer mit dem kleineren $R_f$-Wert |
| S = | S-Enantiomer |

**Verwendete Abkürzungen:**

| | |
|---|---|
| Ac = | Acetyl |
| Bn = | Benzyl |
| Bz = | Benzoyl |
| iBu = | iso Butyl |
| nBu = | normal Butyl |
| sBu = | sekundär Butyl |
| tBu = | tert. Butyl |
| cDec = | cyclo Decyl |
| DMF = | N,N-Dimethylformamid |
| DMSO = | Dimethylsulfoxid |
| cDodec = | cyclo-Dodecyl |
| Et = | Ethyl |
| cHept = | cyclo-Heptyl |
| cHex = | cyclo-Hexyl |
| HOBT = | 1-Hydroxy-1H-benzotriazol |
| Me = | Methyl |
| Mes = | Mesyl |
| cNon = | cyclo-Nonyl |
| cOct = | cyclo-Octyl |
| cPent = | cyclo-Pentyl |
| nPent = | normal Pentyl |
| Ph = | Phenyl |
| cPr = | cyclo-Propyl |
| nPr = | normal Propyl |

| | |
|---|---|
| iPr = | iso-Propyl |
| THF = | Tetrahydrofuran |
| TMS = | Tetramethylsilan |
| pTol = | para-Tolyl |
| pTos = | para-Tosyl |
| cUndec = | cyclo-Undecyl |

**Benutzte Solvenzgemische:**

| Bezeichnung | Laufmittel | Verhältnis |
|---|---|---|
| A | Dichlormethan : Essigester | 1 : 1 |
| B | Dichlormethan : Methanol | 10 : 1 |
| C | Dichlormethan : Methanol | 20 : 1 |
| D | Dichlormethan : Methanol | 50 : 1 |
| E | Dichlormethan : Methanol | 25 : 1 |
| F | Dichlormethan : Ethanol | 20 : 1 |
| G | Dichlormethan : Methanol | 100 : 1 |
| H | Petrolether : Essigester | 2 : 1 |
| I | Toluol : Essigester | 4 : 1 |
| J | Petrolether : Aceton | 2 : 1 |
| K | Dichlormethan : Essigester | 5 : 1 |
| L | Petrolether : Aceton | 1 : 1 |
| M | Dichlormethan : Essigester | 4 : 1 |
| N | Toluol : Essigester | 1 : 1 |
| O | Petrolether : Essigester | 1 : 1 |
| P | Petrolether : Aceton | 3 : 1 |
| Q | Toluol : Essigester | 2 : 3 |
| R | Toluol : Essigester | 2 : 1 |
| S | Dichlormethan : Essigester | 10 : 1 |
| T | Petrolether : Essigester | 5 : 1 |
| U | Dichlormethan : Methanol | 5 : 1 |
| V | Petrolether : Essigester | 4 : 1 |
| W | Essigester | |

| Bezeichnung | Laufmittel | Verhältnis |
|---|---|---|
| X | Petrolether : Essigester | 3 : 2 |
| Y | Dichlormethan : Ethanol | 50 : 1 |
| Z | Dichlormethan : Methanol | 19 : 1 |
| AA | Toluol : Ethanol | 30 : 1 |
| AB | Toluol : Ethanol | 15 : 1 |
| AC | Dichlormethan:Methanol: Essigsäure | 90 : 10 : 1 |
| AD | Dichlormethan:Methanol:konzentriertem wäßrigen Ammoniak | 90 : 10 : 1 |
| AE | Petrolether : Essigester | 3 : 7 |
| AF | Petrolether : Essigester | 7 : 3 |
| AG | Petrolether : Essigester | 1 : 2 |
| AH | Petrolether : Essigester | 2 : 3 |
| AI | Petrolether : Essigester | 1 : 4 |
| AJ | Dichlormethan : Methanol | 9 : 1 |
| AK | Toluol : Aceton | 10 : 1 |
| AL | Toluol : Aceton | 40 : 1 |

Zubereitungsvorschrift für das DC-Laufmittel BABA:

87,9 ml einer wäßrigen 0,06667 molaren Kaliumdihydrogenphosphatlösung und 12,1 ml einer wäßrigen 0,06667 molaren Dinatriumhydrogenphosphatlösung werden gemischt. 60 ml der so zubereiteten Lösung werden mit 200 ml n-Butylacetat, 36 ml n-Butanol und 100 ml Eisessig geschüttelt und die wäßrige Phase abgetrennt. Die organische Phase ist das Laufmittel BABA.

## Ausgangsverbindungen

### Beispiel I

2-Amino-2-[2'-(1',3'-dithiolano)]essigsäuremethylester Hydrochlorid

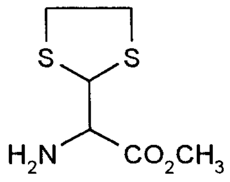

x HCl (racemisch)

0,5 g (2,8 mmol) 2-Amino-2-[2'-(1',3'-dithiolano)]essigsäure (Synthese: M.P. Mertes und A. A. Ramsey J. Med. Chem. 12, 342 (1969).) werden in 10 ml Methanol unter Rückfluß mit 0,4 ml (5,6 mmol) Thionylchlorid umgesetzt. Nach 8 Stunden kühlt man ab, dampft im Vakuum am Rotationsverdampfer bis zur Trocknene ein und entfernt dann Restlösemittel und Reagenzübersschüsse im Hochvakuum über Phosphorpentoxid und festem Natriumhydroxid.
Ausbeute: 0,62 g
$R_f$ = 0,32 (BABA)
charakteristische $^1$H-NMR-Signale ($CD_3OD$, 200 MHz, das Lösemittelsignal als Standard bei 3,25 ppm): $\delta$ = 4,36 (d, 1H); 5,17 (d, 1H) ppm.

Analog Beispiel I werden folgende Verbindungen erhalten:

**Tabelle I:**

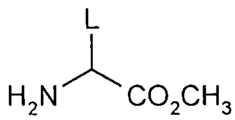 x HCl

| Bsp.-Nr. | L/Hydrochlorid | $R_f$ / Solvens | Literatur/Vertreiber des Ausgangsmaterials |
|---|---|---|---|
| II | 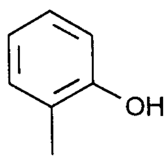 | 0,39 / BABA | Edukt : 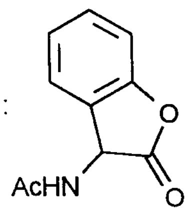 DE 21 62 717 |
| III | 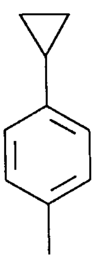 | 0,01/AH | |
| IV | 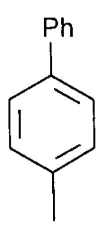 | 0,01/AH | |

| Bsp.-Nr. | L/Hydrochlorid | $R_f$ / Solvens | Literatur/Vertreiber des Ausgangsmaterials |
|---|---|---|---|
| V | 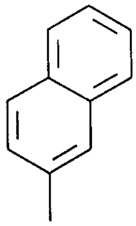 | 0,01/AH | Edukt = 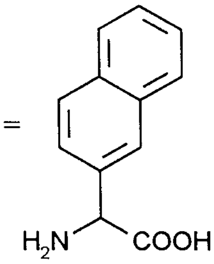 US 4 474 780 |
| VI | 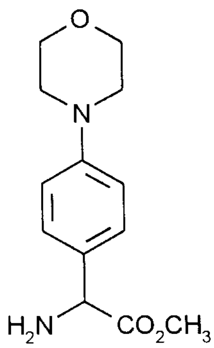 | 0,21 / E | Edukt = 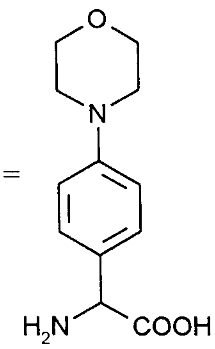 DE 28 36 613 |
| VII | 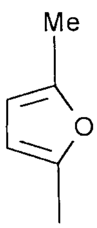 | 0,27 / BABA | Edukt = 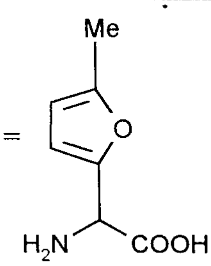 R.M. Williams et al., J. Org. Chem. 55, 3723 (1990). |

| Bsp.-Nr. | L/Hydrochlorid | $R_f$ / Solvens | Literatur/Vertreiber des Ausgangsmaterials |
|---|---|---|---|
| VIII | 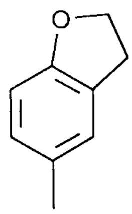 | 0,46 / BABA | Edukt = 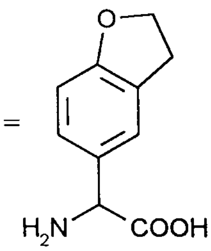 US 4 138 397 |
| IX | 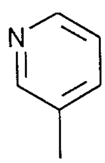 | 0,17 / BABA | Edukt = 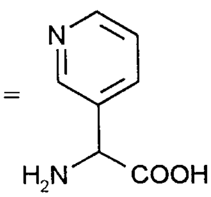 DE 25 40 804 |
| X | 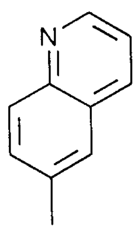 | 0,20 / BABA | Edukt = 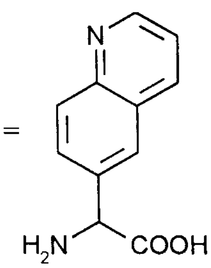 US 4 734 407 |

| Bsp.-Nr. | L/Hydrochlorid | $R_f$ / Solvens | Literatur/Vertreiber des Ausgangsmaterials |
|---|---|---|---|
| XI | 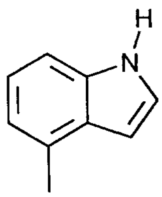 | 0,45 / BABA | Edukt = 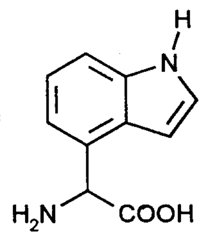 US 4 734 407 |
| XII | 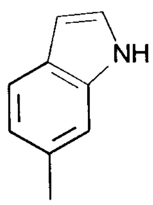 | 0,58 / BABA | Edukt = 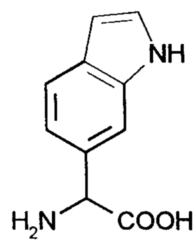 G. Schmidt et al. in "Recent Advances in Chemotherapy" Ed. D.Adam , H. Lode und E.Rubinstein, S. 2428 f, Futuramed Publishers München, 1992. |
| XIII | 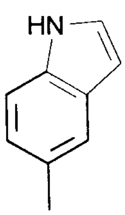 | 0,58 / BABA | Edukt = 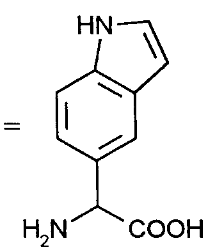 US 4 734 407 |

| Bsp.-Nr. | L/Hydrochlorid | $R_f$ / Solvens | Literatur/Vertreiber des Ausgangsmaterials |
|---|---|---|---|
| XIV | 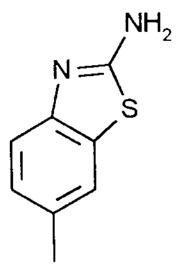 | 0,29 / BABA | Edukt = 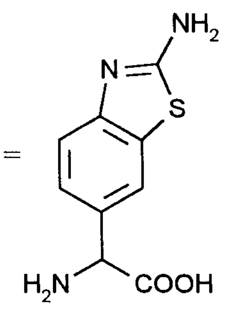 US 4 748 163 |
| XV | 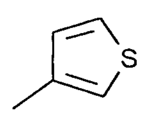 | 0,40 / BABA | Edukt = 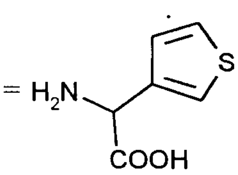 M. Hatanaka et al., J. Med. Chem. 16, 978 (1973). |

**Beispiel XVI**

3-Fluorphenylglycinol

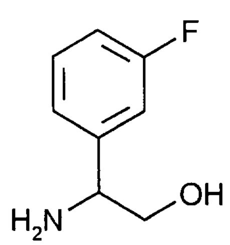

71 ml einer 1 M Lithiumalanatlösung in THF werden mit 18 ml wasserfreiem THF versetzt und zum Rückfluß erhitzt. Nach Entfernen des Heizbades setzt man 6,0 g 3-Fluorphenylglycin (E.L. Compere Jr., D.A. Weinstein, Synthesis 1977, 852) in kleinen Portionen zu, erhitzt eine weitere Stunde Zum Rückfluß und tropft dann vorsichtig eine Lösung von 1,27 g Kaliumhydroxid in 5,1 ml Wasser zu. Nach weiteren 15 Minuten unter Rückfluß wird der entstandene Niederschlag

heiß abgesaugt und mehrfach mit THF gewaschen. Das Filtrat wird weitgehend eingeengt (Vakuum/Rotationsverdampfer), mit Essigester aufgenommen, mit Natriumsulfat getrocknet, wiederum eingedampft und mit Petrolether verrührt, wobei sich das Produkt langsam verfestigt. Nach scharfem Absaugen und Entfernen des Restlösemittels im Vakuum fallen 6,0 g Produkt an.
$R_f$ = 0,13 (B)
$^1$H-NMR ($CD_3OD$, 250 MHz, das Lösemittelsignal bei 3,25 ppm dient als Standard): δ = 3,48 (dd, 1H); 3,62 (dd, 1H); 3,89 (dd, 1H); 6,93 (m, 1H); 7,05 - 7,14 (m, 2H); 7,27 (m, 1H) ppm.
D,L-3-Fluorphenylglycin ist außerdem bei K & K käuflich.

Analog Beispiel XVI werden die folgenden Verbindungen erhalten:

**Tabelle II:**

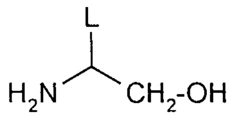

| Bsp.-Nr. | L | $R_f$ / Solvens | Vertreiber des Ausgangsmaterials |
|---|---|---|---|
| XVII | 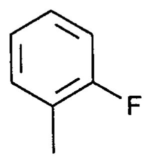 | 0,18/B | Aldrich |
| XVIII | 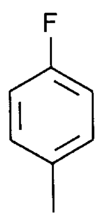 | 0,09/B | Fluka |
| XIX | 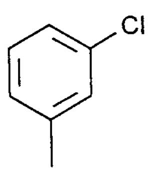 | 0,23 / B | K & K |
| XX | 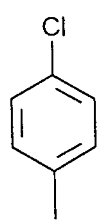 | 0,12 / B | K & K |

**Beispiel XXI**

E- und Z-(Isochinolin-1-yl)-phenyl-ketoxim

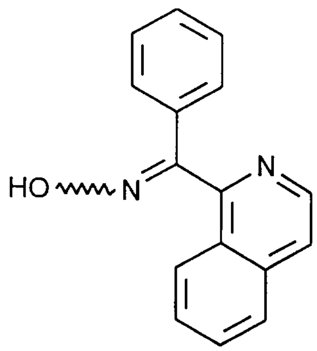

15,0 g 1-Benzoylisochinolin (J. Knabe und A. Frie, Arch. Pharm. 306, 648 (1973).) und 8,94 g Hydroxyammoniumchlorid werden in 50 ml Pyridin 4 Stunden unter Rückfluß gekocht. Die sich abkühlende Reaktionsmischung wird in 500 ml Wasser bei Raumtemperatur eingerührt. Nach 15 minütigem kräftigem Nachrühren saugt man den Niederschlag ab, wäscht ihn mehrfach mit Wasser, saugt zuletzt sehr scharf ab und trocknet ihn dann im Hochvakuum über Phosphorpentoxid bei ca. 80°C.
Ausbeute: 15,9 g
$R_f$ = 0,44 (C)
MS (Chemische Ionisation mit $NH_3$): m/z = 249 ($[M+H]^+$, 17%), 248 ($M^+$, 18%).

Analog der Herstellungsvorschrift von Beispiel XXI werden folgende Verbindungen hergestellt:

Tabelle III:

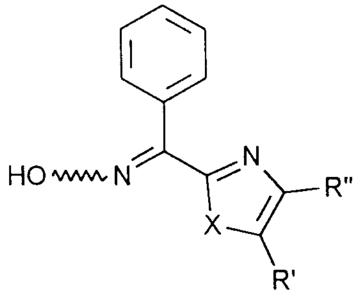

| Bsp.-Nr. | X | R' | R'' | $R_f$ (Solvenz) | Literatur für das Ausgangsmaterial |
|---|---|---|---|---|---|
| XXII | S | $CH_3$ | $CH_3$ | 0,73 / 0,53 (C) | Y. Oka et al., Chem. Pharm. Bull. 18, 527 (1970). |
| XXIII | NH | H | H | 0,30 / 0,26 (B) | A. Sonn et al., Chem. Ber. 66, 1900 (1933) |

**Beispiel XXIV**

1-(α-Aminobenzyl)-isochinolin

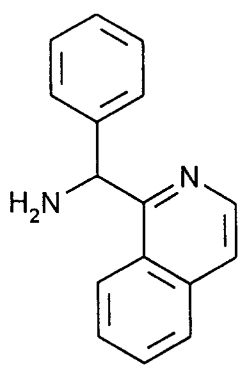

15,72 g der Verbindung aus Beispiel XXI und 6,05 g Ammoniumacetat werden in 236 ml konzentrierter wäßriger Ammoniaklösung, 157 ml Wasser und 157 ml Ethanol suspendiert und darauf unter Rückfluß über eine Stunde portionsweise mit insgesamt 22,7 g Zinkstaub versetzt. Nach 3 Stunden unter Rückfluß kühlt man auf Raumtemperatur ab, stellt mit 10%iger wäßriger Natronlauge (10 g festes NaOH auf 90 ml Wasser) einen pH von 14 ein und extrahiert mehrfach mit

Ether. Die organischen Phasen werden mit Natriumsulfat getrocknet, eingedampft und im Hochvakuum vom Restlösemittel befreit.
Ausbeute: 12,4 g
$R_f$ = 0,13 (B)
charakteristisches $^1$H-NMR-Signal ($CDCl_3$, 250 MHz, TMS): δ = 5,95 (s, 1H, CH-$NH_2$) ppm.

In Analogie zur Herstellung von Beispiel XXIV lassen sich die folgenden Verbindungen darstellen:

Tabelle IV:

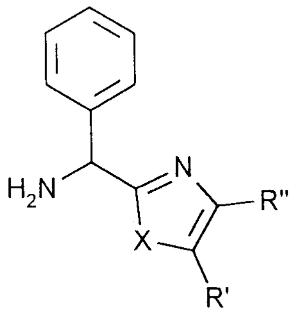

| Bsp.-Nr. | X | R' | R'' | $R_f$ (Solvenz) | Bsp.-Nr. des Ausgangsmaterials |
|---|---|---|---|---|---|
| XXV | S | $CH_3$ | $CH_3$ | 0,38 (C) | XXII |
| XXVI | NH | H | H | 0,13 (B) | XXIII |

Analog Beispiel XVI werden folgende Verbindungen erhalten:

**Tabelle V:**

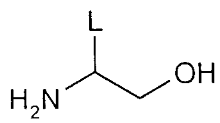

| Bsp.-Nr. | L | $R_f$ (Solvenz) | Literatur/Vertreiber des Ausgangsmaterials |
|---|---|---|---|
| XXVII | 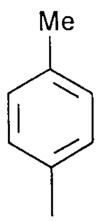 | 0,10 (B) | Edukt = 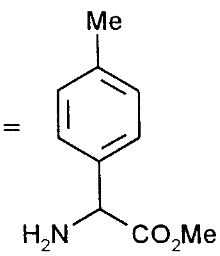 F. Rose-Munch et al., J. Organomet. Chem. 415, 223 (1991). |
| XXVIII | 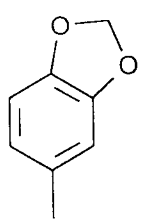 | 0,01 (0) | Edukt = 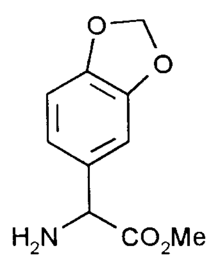 G.H. Hakimelahi und G. Just, Can. J. Chem. 57, 1932 (1979). |
| XXIX | 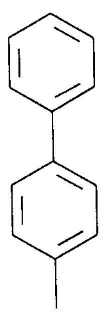 | 0,19 (U) | Edukt = Bsp. Nr. IV |

**Beispiel XXX**

(R, S)-2-Benzyl-4-hydroxy-buttersäureamid

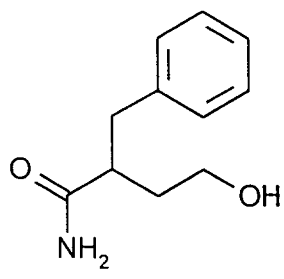

12,0 g (R,S)-2-4-hydroxy-benzyl-γ-butyrolacton (Z. Jedlinski et al., J. Org. Chem. 52, 4601 (1987).) werden in einer gesättigten Lösung von Ammoniak in Methanol bei Raumtemperatur umgesetzt. Nach 20 Stunden dampft man das Lösemittel mit dem überschüssigen Reagenz im Vakuum ab, nimmt wiederum in Methanol auf und dampft ein, um das überschüssige Ammoniak völlig zu entfernen. Der erhaltene Feststoff wird im Exsikkator über Phosphorpentoxid nachgetrocknet.
Ausbeute: 13,5 g
$R_f$ = 0,39 (B)
MS (EI): m/z = 193 (5%, $M^+$), 176 (25%, $M^+$-$NH_3$); 162 (9%, $M^+$-$CH_2OH$); 148 (56%, $M^+$-$NH_3$-CO); 91 (100%, $C_6H_5CH_2^+$).

**Beispiel XXXI**

4-Amino-(R,S)-3-benzyl-butanol-1

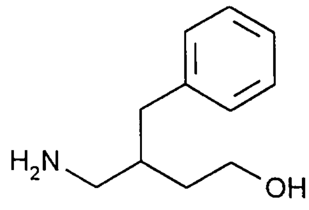

11,6 g der Verbindung aus Beispiel XXX werden in 100 ml THF (wasserfrei) mit 4,55 g Lithiumalanat bei Raumtemperatur zur Reaktion gebracht. Nach 20 Stunden bricht man die Umsetzung durch Zugabe von 45 ml gesättigter wäßriger Natriumchloridlösung ab, rührt eine Stunde kräftig nach und saugt die Mischung über Kieselguhr ab. Das Eluat wird mit Toluol aufgenommen (Zur völligen Auflösung ist gegebenenfalls eine Zugabe von Ethanol notwendig.) und durch Abdampfen, Wiederaufnehmen in Toluol und erneutes Abdampfen das Wasser mit dem organischen Lösemittel herausgeschleppt.
Ausbeute: 11,3 g
$R_f$ = 0,23 (Dichlormethan : Methanol : Eisessig = 5:1:1)
Das rohe Produkt wird ohne Aufreinigung weiter umgesetzt.

Die Synthese der Edukt-Carbonsäuren sind bekannt, vgl. hierzu beispielsweise (US 4 929 629, US 4 970 215, EP 509 359, US 4 929 626, US 5 091 392, 5 126 354, EP 414 078, EP 529 450).

**Beispiel XXXII und Beispiel XXXIII**

2-(R)- und 2-(S)-2-{4-[(Chinolin-2-yl)methoxy]phenyl}-2-cyclopentyl-essigsäure-(R)-phenylglycinolamid

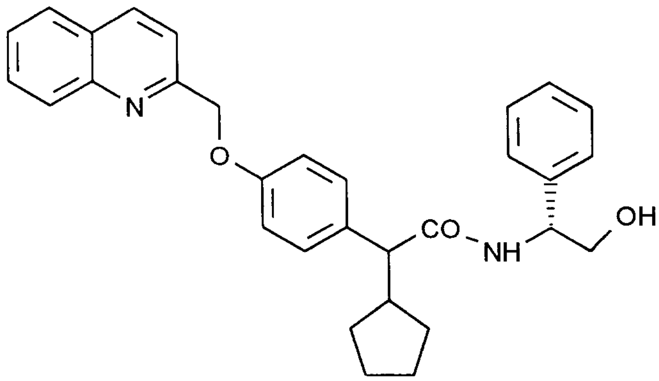

2,5 g (6,9 mmol) racemische 2-{4-[(Chinolin-2-yl)methoxy]phenyl}-2-cyclopentyl-essigsäure (Synthese: US 4 970 215) werden in 25 ml wasserfreiem N,N-Dimethylformamid gelöst, mit 2,88 ml (20,8 mmol) Triethylamin sowie 604,7 µl (7,6 mmol) Mesylchlorid versetzt und unter Argon als Schutzgas 3 Std. bei -60°C nachgerührt. Darauf setzt man 1,14 g (8,3 mmol) (R)-Phenylglycinol (käuflich bei Aldrich) und 0,84 g (6,9 mmol) 4-(N,N-Dimethylamino)-pyridin gelöst in 20 ml wasserfreiem N,N-Dimethylformamid zu und rührt unter langsamer Erwärmung auf Raumtemperatur insgesamt 16 h nach. Das Reaktionsgemisch wird mit Essigester und Wasser versetzt und die wäßrige Phase mit 1 M Salzsäure auf einen pH-Wert von ca. 2 gestellt. Die organische Phase wird mehrfach mit verdünnter Salzsäure (pH ~ 2) extrahiert, mit Wasser gewaschen und dann mehrfach mit 0,1 M wäßriger Natronlauge extrahiert. Wiederum wird die organische Phase mit Wasser gewaschen, anschließend mit wasserfreiem Magnesiumsulfat getrocknet und eingedampft. Zur Auftrennung der Komponenten wird eine Säulenchromatographie durchgeführt (Kieselgel 60, Merck 40-63 µm, Laufmittel:Petrolether:Essigester von 5:1 bis 1:1)

**Beispiel XXXII:**

$R_f$ = 0,12 (K)
Ausbeute: 1,1 g

**Beispiel XXXIII:**

$R_f$ = 0,08 (K)
Ausbeute: 1,0 g
Die absolute Konfiguration der enantiomerenreinen Carbonsäuren (Ausgangsmaterial zu den Beispielen XXXII und XXXIII) ist bekannt (EP 509 359), sodaß die absolute Konfiguration der Produkte (Beispiel XXXII und XXXIII) daraus abgeleitet werden kann. Die 1H-NMR-Spektren der beiden diastereomeren Produkte (200 MHz, d6-DMSO, TMS für Beispiel XXXII / 250 MHz, d6-DMSO, TMS für Beispiel XXXIII) weisen im Aromatenbereich signifikante Unterschiede auf: So liegen die H-Signale des Phenylrestes des Phenylglycinolanids aus Beispiel XXXII bei ca. 7,1 ppm und im Falle von Beispiel XXXIII bei ca. 7,3 ppm. Dieser Befund ist auf viele Derivate diesen Typs übertragbar.

**Beispiel XXXIV**

2-(S)-2-{4-[(Chinolin-2-yl)methoxy]phenyl}-2-cycloheptyl-essigsäure-(R)-phenylglycinolamid

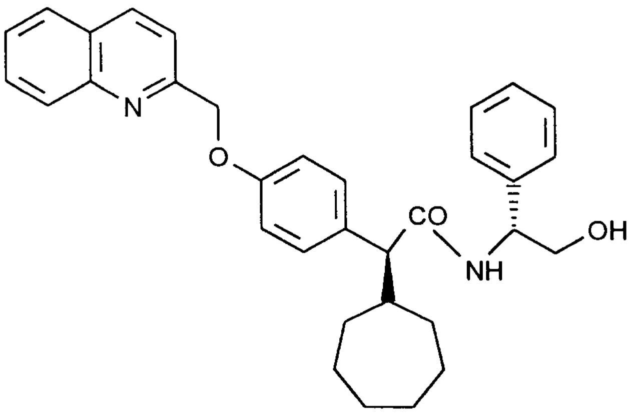

Die Titelverbindung wird in Analogie zur Vorschrift der Beispiele XXXII und XXXIII hergestellt.
Fp. = 176°C

**Beispiel XXXV**

(2-Hydroxy-phenyl)-glycinol-Hydrochlorid

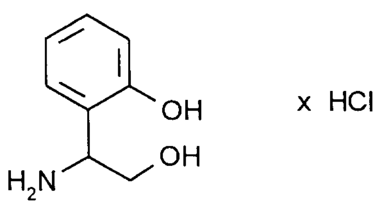

2,18 g (10 mmol) (2-Hydroxy-phenyl)-glycin-methylester-hydrochlorid werden in 20 ml trockenem THF gelöst und bei Raumtemperatur (25°C) 18 Stunden mit 3,34 g (33 mmol) Triethylamin und 2,39 g (22 mmol) Trimethylchlorsilan umgesetzt. Der entstandene Niederschlag wird abgesaugt, mit trockenem THF gewaschen und das Filtrat bei 25°C mit Lithiumalanat (0,76 g/38,0 mmol) umgesetzt. Darauf saugt man überschüssiges Reagenz ab, wäscht mit trockenem THF nach, versetzt mit Wasser und verdünnt mit Ether (pH ~ 10). Die wäßrige Phase wird mit 2 M Salzsäure auf pH = 2 gestellt, mit Ether gewaschen und lyophilisiert.
Ausbeute 1,20 g (6,3 mmol)
Rf = 0,38 (BABA)
In Analogie zur Vorschrift des Beispiels XXXV werden die in Tabelle VI aufgeführten Verbindungen hergestellt.

**Tabelle VI**

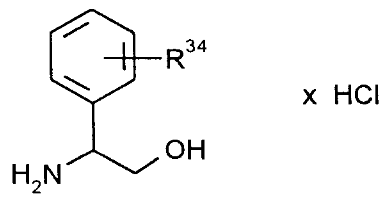

| Bsp.-Nr. | R34 | Rf (Laufmittel) |
|---|---|---|
| XXXVI | 3-OH | 0,23 (U) |
| XXXVII | 4-OH | 0,34 (BABA) |

Die Verbindungen der Tabelle VII werden analog der Vorschrift von Beispiel-Nr. I hergestellt:

**Tabelle VII**

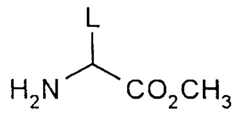

x HCl

| Bsp.-Nr. | L | Fp. [°C] Rf (Solvens) |
|---|---|---|
| XXXVIII | 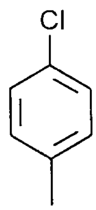 | 0,01 (AH) |
| XXXIX | 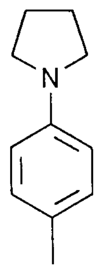 | 0,00 (AH) |
| XL | 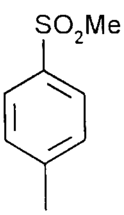 | 0,01 (AH) |
| XLI | 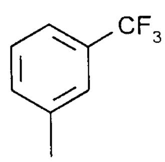 | 0,01 (AH) |
| XLII | 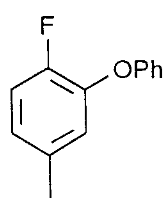 | 0,01 (AH) |
| XLIII | 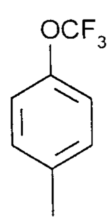 | 0,01 (AH) |

| Bsp.-Nr. | L | Fp. [°C] Rf (Solvens) |
|---|---|---|
| XLIV | 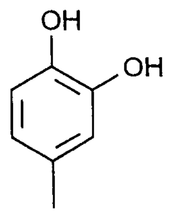 | 0,01 (AH) |

## Herstellungsbeispiele

### Beispiel 1

(2S)-2-[4-(Chinolin-2-yl-methoxy)-phenyl]-2-cyclopentyl-essigsäure-(R)-(O-acetyl)-phenylglycinolamid

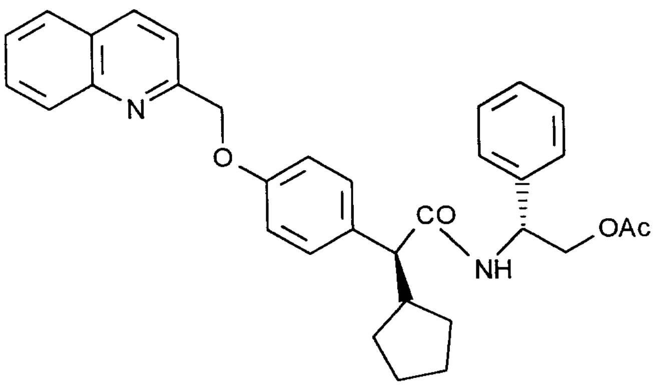

30 mg der Verbindung aus Beispiel XXXII werden in 20 ml Dichlormethan bei Raumtemperatur mit 13,4 µl Acetylchlorid und 15,1 µl Pyridin zur Reaktion gebracht. Nach 20 Stunden wird der Ansatz auf 1 M Salzsäure gegossen und die organische Phase darauf mit einer Pufferlösung von pH = 2 (Pufferlösung gebrauchsfertig, pH = 2,00, Citrat-Salzsäure bezogen auf SMR von NIST, Merck, Best.-Nr. 9433.1000) gewaschen. Die organische Phase wird mit Magnesiumsulfat getrocknet, eingedampft und im Hochvakuum vom Restlösemittel befreit.
Ausbeute: 29 mg.
Rf = 0,47 (F)

**Beispiel 2**

(2S)-2-[4-(Chinolin-2-yl-methoxy)phenyl]-2-cyclopentyl-essigsäure-(S)-(O-acetyl)-phenyl-glycinol-amid

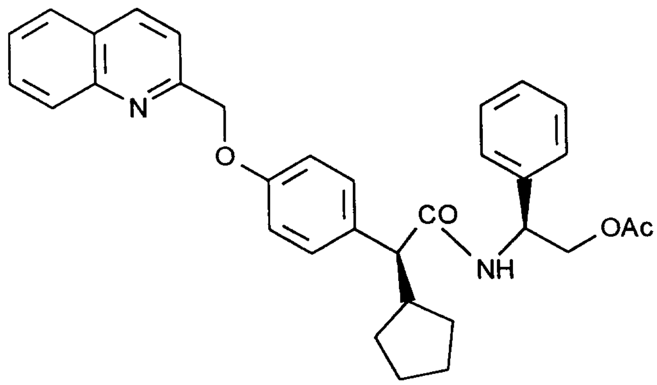

In Analogie zur Vorschrift des Beispiels 1 wird (2S)-2-[4-(Chinolin-2-yl-methoxy)phenyl]-2-cyclopentyl-essigsäure-(S)-phenylglycinol-amid (US 4 970 215) zur Titelverbindung umgesetzt.

Fp.: 143°C

Analog Beispiel XXXII und XXXIII lassen sich die Verbindungen aus den Tabellen 1 und 2 herstellen:

Tabelle 1:

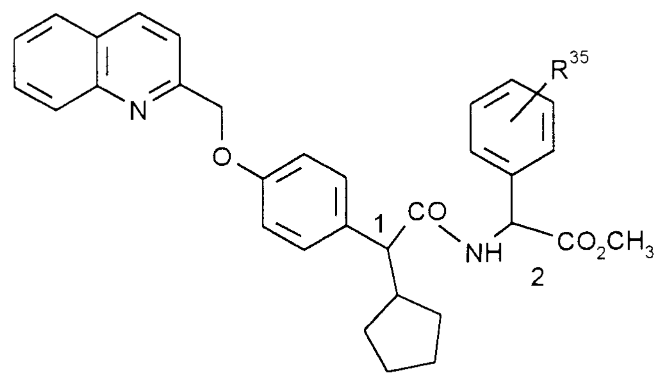

| Bsp.-Nr. | 1 | 2 | $R^{35}$ | $R_f$ / Solvens | Beispiel-Nr. / Literatur für das Eduktamin |
|---|---|---|---|---|---|
| 3 | rac | rac | 2-OH | 0,25 / D | Beispiel II |
| 4 | rac | rac | 3-OH | 0,19 / D | DE 22 04 117 |
| 5 | rac | rac | 4-OH | 0,18 / D | EP 530 879 |
| 6 | rac | rac | 2-OMe | 0,31 / D | S.J. Daum et al., Syn. Commun. 11, 1 (1981). |
| 7 | rac | rac | 3-OMe | 0,15 / G | DE 23 09 180 |

| Bsp.-Nr. | 1 | 2 | $R^{35}$ | $R_f$ / Solvens | Beispiel-Nr. / Literatur für das Eduktamin |
|---|---|---|---|---|---|
| 8 | rac | rac | 4-OMe | 0,35 / D | H.E. Baumgarten et al., J. Org. Chem. 31, 3708 (1966). |
| 9 | rac | rac | 4-tBu | 0,36 / H | WO 91 / 08 704 |
| 10 | rac | rac | 4-cPr | 0,41 / I | Beispiel III |
| 11 | rac | rac | 4-Ph | 0,53 / J | Beispiel IV |
| 12 | rac | rac | 3,4- 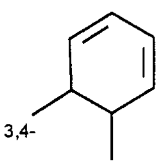 | 0,44 / I | Beispiel V |
| 13 | rac | rac | 4- 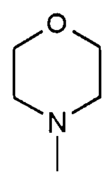 | 0,68 / E | Beispiel VI |

**Tabelle 2:**

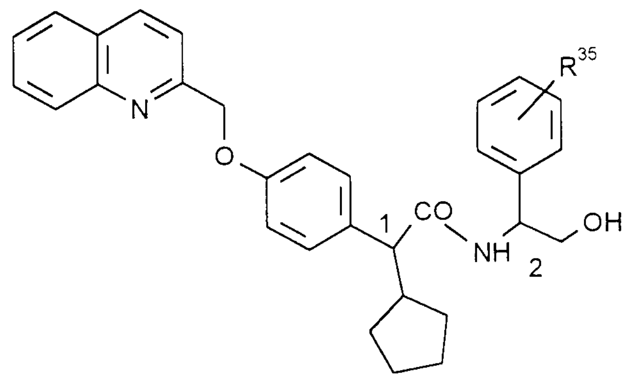

| Bsp.-Nr. | 1 | 2 | $R^{35}$ | Rf / Solvens | Bsp. Nr. des Eduktes |
|---|---|---|---|---|---|
| 14 | rac | rac | 2-F | 0,61 / A | Beispiel XVII |
| 15 | rac | rac | 3-F | 0,57 / A | Beispiel XVI |
| 16 | rac | rac | 4-F | 0,50 / A | Beispiel XVIII |
| 17 | rac | rac | 3-Cl | 0,61 / B | Beispiel XIX |
| 18 | rac | rac | 4-Cl | 0,72 / B | Beispiel XX |
| 19 | rac | rac | 3,4-OCH2O- | 0,48 / A | Beispiel XXVIII |
| 20 | rac | rac | 4-Me | 0,60 / A | Beispiel XXVII |

**Beispiel 21**

2-{4-[Chinolin-2-yl-methoxy]phenyl}-2-cyclopentyl-essigsäure-[4-tertiär-butyl-phenyl]glycinol-amid

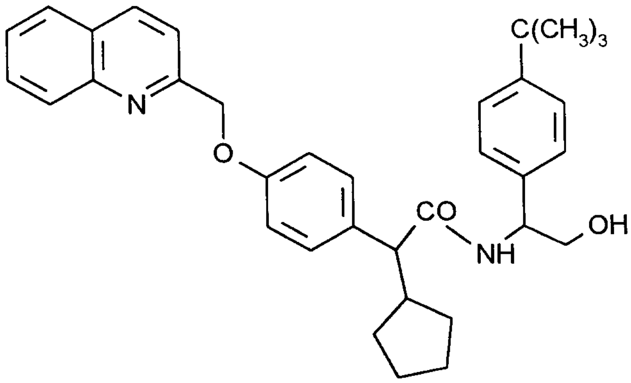

0,78 mg der Verbindung aus Beispiel 9 wird bei Raumtemperatur in 10 ml wasserfreiem THF mit 3,05 ml einer 1 M Lösung von Lithiumalanat in THF umgesetzt. Nach 100 Minuten gießt man auf Essigester / Wasser, wäscht die organische Phase mit gesättigter wäßriger Natriumchloridlösung, trocknet die organische Phase mit Magnesiumsulfat und dampft zur Trockne ein. Das erhaltene Rohprodukt wird säulenchromatographisch gereinigt (Kieselgel 60, Merck 40-63 µm, als Laufmittel dient zuerst Dichlormethan und dann ein Gemisch von Dichlormethan:Methanol von zuerst 200:1, dann 100:1, 50:1 bis 20:1).
Ausbeute: 0,38 g
$R_f$ = 0,46 (E)
In Analogie zur Vorschrift des Beispiels 21 werden die Verbindungen aus der Tabelle 3 hergestellt:

Tabelle 3:

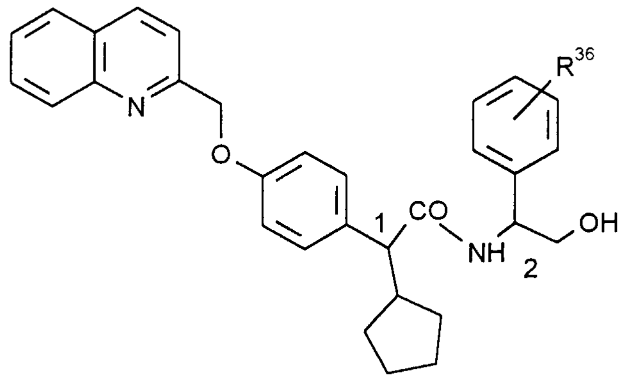

| Bsp.-Nr. | 1 | 2 | $R^{36}$ | $R_f$ / Solvens | Bsp. Nr. der Edukte |
|---|---|---|---|---|---|
| 22 | rac | rac | 4-cPr | 0,33 / E | Beispiel 10 |
| 23 | rac | rac | 4-Ph | 0,27 / E | Beispiel 11 |
| 24 | rac | rac | 3,4- 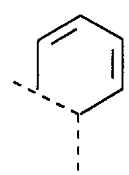 | 0,42 / E | Beispiel 12 |

| Bsp.-Nr. | 1 | 2 | $R^{36}$ | $R_f$ / Solvens | Bsp. Nr. der Edukte |
|---|---|---|---|---|---|
| 25 | rac | rac | 4- 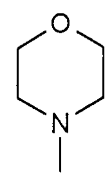 | 0,63 / B | Beispiel 13 |
| 26 | rac dia A | | 4-Ph | 0,36 / F | Beispiel 11 |
| 27 | rac dia B | | 4-Ph | 0,36 / F | Beispiel 11 |
| 28 | rac | rac | 2-OH | 0,40 / C | Beispiel 3 |
| 29 | rac | rac | 3-OH | 0,40 / B | Beispiel 4 |
| 30 | rac | rac | 4-OH | 0,34 / B | Beispiel 5 |
| 31 | rac | rac | 2-OMe | 0,09 / D | Beispiel 6 |
| 32 | rac dia A | | 3-OMe | 0,29 / C | Bespiel 7 |
| 33 | rac | rac | 3-OMe | 0,29 / C | Beispiel 7 |
| 34 | rac | rac | 4-OMe | 0,36 / C | Beispiel 8 |

In Analogie zu den Vorschriften der Beispiele XXXII und XXXIII werden die in der Tabelle 4 aufgeführten Verbindungen hergestellt.

**Tabelle 4:**

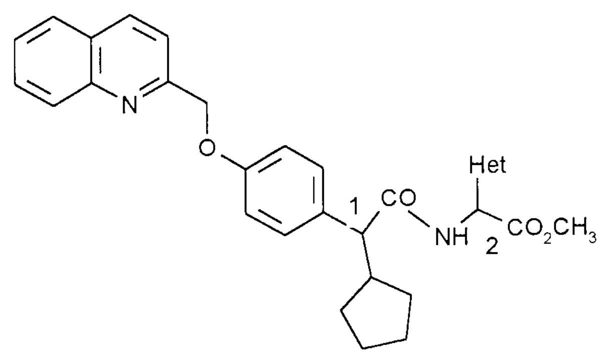

| Bsp.-Nr. | 1 | 2 | Het | $R_f$ / Solvens | Beispiel-Nr. / Literatur für das Eduktamin |
|---|---|---|---|---|---|
| 35 | rac | rac | 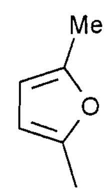 | 0,97 / K | Beispiel VII |

Tabelle 4 - Fortsetzung

| **Bsp.-Nr.** | **1** | **2** | **Het** | **$R_f$ / Solvens** | **Beispiel-Nr. / Literatur für das Eduktamin** |
|---|---|---|---|---|---|
| 36 | rac | rac | 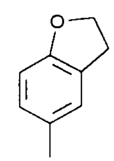 | 0,40 / O | Beispiel VIII |
| 37 | rac | rac | 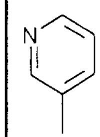 | 0,30 / K | Beispiel IX |
| 38 | rac | rac | 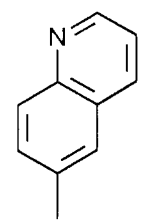 | 0,24 / P | Beispiel X |

Tabelle 4 - Fortsetzung

| Bsp.-Nr. | 1 | 2 | Het | $R_f$ / Solvens | Beispiel-Nr. / Literatur für das Eduktamin |
|---|---|---|---|---|---|
| 39 | rac | rac | 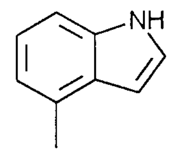 | 0,67 / C | Beispiel XI |
| 40 | rac | rac | 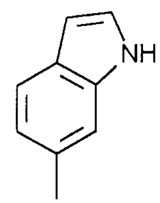 | 0,43 / B | Beispiel XII |

Tabelle 4 - Fortsetzung

| **Bsp.-Nr.** | **1** | **2** | **Het** | **$R_f$ / Solvens** | **Beispiel-Nr. / Literatur für das Eduktamin** |
|---|---|---|---|---|---|
| 41 | rac | rac | 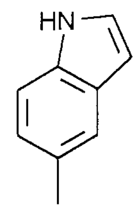 | 0,38 / C | Beispiel XIII |
| 42 | rac | rac | 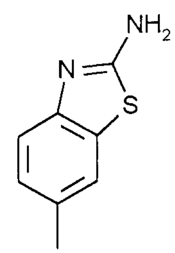 | 0,30 / Q | Beispiel XIV |

Tabelle 4 - Fortsetzung

| Bsp.-Nr. | 1 | 2 | Het | $R_f$ / Solvens | Beispiel-Nr. / Literatur für das Eduktamin |
|---|---|---|---|---|---|
| 43 | rac | rac | 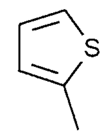 | 0,65 / K | DE 22 04 117 |
| 44 | rac | rac | 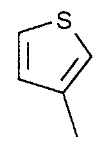 | 0,60 / K | Beispiel XV |
| 45 | rac | rac | 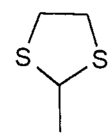 | 0,50 / R | Beispiel I |

Analog der Vorschriften der Beispiele XXXII, XXXIII und 21 werden die in der Tabelle 5 aufgeführten Verbindungen hergestellt:

**Tabelle 5:**

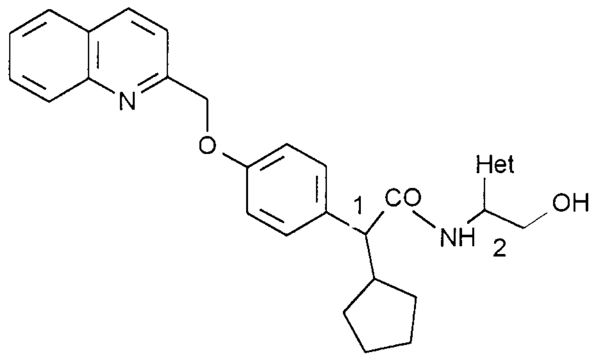

| Bsp.-Nr. | 1 | 2 | Het | $R_f$ / Solvens | Bsp.-Nr. Edukt | Herstellung analog Bsp.-Nr. |
|---|---|---|---|---|---|---|
| 46 | rac | rac | 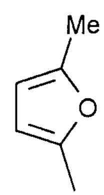 | 0,2 / K | Beispiel 35 | 21 |

| Bsp.-Nr. | 1 | 2 | Het | $R_f$ / Solvens | Bsp.-Nr. Edukt | Herstellung analog Bsp.-Nr. |
|---|---|---|---|---|---|---|
| 47 | rac | rac | 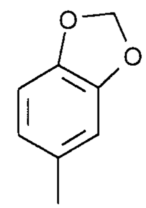 | 0,48 / A | Beispiel XXVIII | XXXII, XXXIII |
| 48 | rac | rac | 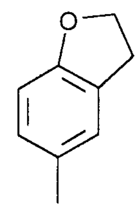 | 0,22 / E | Beispiel 36 | 21 |
| 49 | rac | rac | 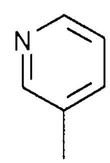 | 0,33 / B | Beispiel 37 | 21 |

| Bsp.-Nr. | 1 | 2 | Het_ | $R_f$ / Solvens | Bsp.-Nr. Edukt | Herstellung analog Bsp.-Nr. |
|---|---|---|---|---|---|---|
| 50 | rac | rac | 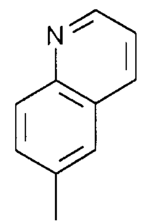 | 0,2 / L | Beispiel 38 | 21 |
| 51 | rac | rac | 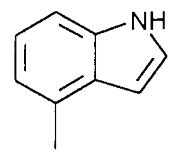 | 0,2 / C | Beispiel 39 | 21 |
| 52 | rac | rac | 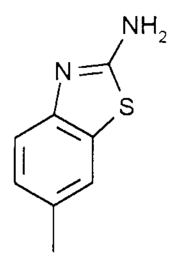 | 0,39 / B | Beispiel 42 | 21 |

| Bsp.-Nr. | 1 | 2 | Het | $R_f$ / Solvens | Bsp.-Nr. Edukt | Herstellung analog Bsp.-Nr. |
|---|---|---|---|---|---|---|
| 53 | rac | rac | 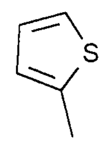 | 0,14 / K | Beispiel 43 | 21 |
| 54 | rac | rac | 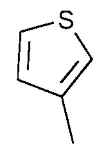 | 0,1 / M | Beispiel 44 | 21 |
| 55 | rac | rac | 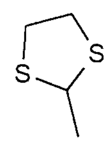 | 0,36 / N | Beispiel 45 | 21 |

Analog zur Vorschrift der Beispiele XXXII, XXXIII und 21 werden die in den Tabellen 6 und 7 aufgeführten Verbindungen hergestellt:

**Tabelle 6:**

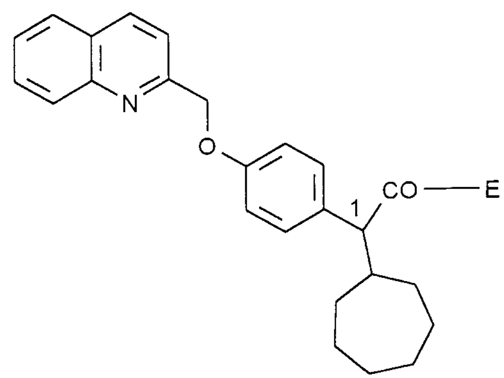

| Bsp.-Nr. | 1 | E | $R_f$ / Solvens | Vertreiber / Literatur / Bsp.-Nr. des Ausgangsmaterials | Analog Vorschrift in Beispiel-Nr. |
|---|---|---|---|---|---|
| 56 | S | (rac) 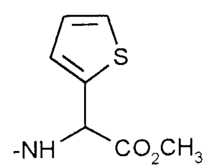 | 0,35 / H | DE 22 04 117 | Beispiel XXXII/XXXIII |

| Bsp.-Nr. | 1 | E | $R_f$ / Solvens | Vertreiber / Literatur / Bsp.-Nr. des Ausgangsmaterials | Analog Vorschrift in Beispiel-Nr. |
|---|---|---|---|---|---|
| 57 | S | (rac) 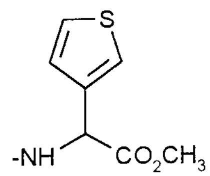 | 0,33 / S | Beispiel XV | Beispiel XXXII/XXXIII |
| 58 | S | (rac) 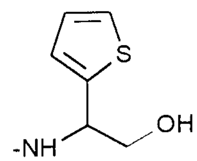 | 0,2 / 0,14 / M | Beispiel 56 | Beispiel 21 |
| 59 | S | (rac) 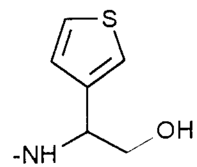 | 0,21 / 0,14 / M | Beispiel 57 | Beispiel 21 |

| Bsp.-Nr. | 1 | E | $R_f$ / Solvens | Vertreiber / Literatur / Bsp.-Nr. des Ausgangsmaterials | Analog Vorschrift in Beispiel-Nr. |
|---|---|---|---|---|---|
| 60 | S | (rac) 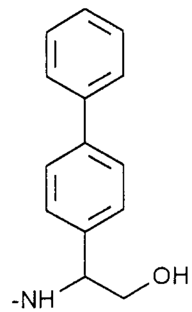 | 0,3 / 0,25 / D | Beispiel XXIX | Beispiel XXXII/XXXIII |
| 61 | S | (dia A) 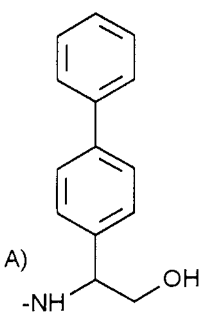 | 0,30 / D | Beispiel XXIX | Beispiel XXXII/XXXIII |

| Bsp.-Nr. | 1 | E | $R_f$ / Solvens | Vertreiber / Literatur / Bsp.-Nr. des Ausgangsmaterials | Analog Vorschrift in Beispiel-Nr. |
|---|---|---|---|---|---|
| 62 | S | 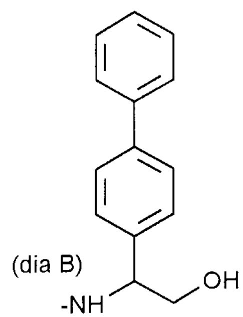 (dia B) | 0,25 / D | Beispiel XXIX | Beispiel XXXII/XXXIII |

Tabelle 7:

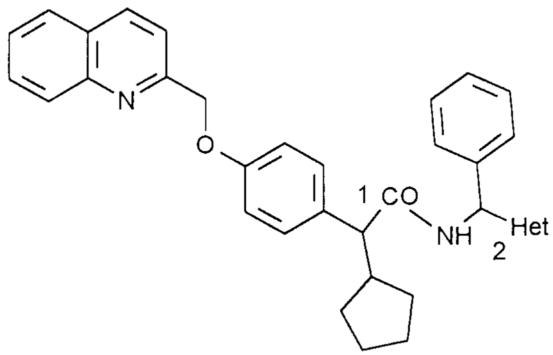

| Bsp.-Nr. | 1 | 2 | Het | $R_f$ / Solvens | Bsp.-Nr. / Literatur für das Eduktamin |
|---|---|---|---|---|---|
| 63 | rac | rac | 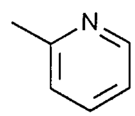 | 0,31 / J | E. Niemers et al., Synthesis 1976, 593. |
| 64 | rac | rac | 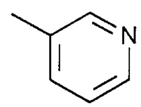 | 0,18 / J | E. Niemers et al., Synthesis 1976, 593. |
| 65 | rac | rac | 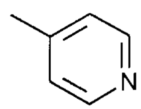 | 0,18 / J | E. Niemers et al., Synthesis 1976, 593. |

| Bsp.-Nr. | 1 | 2 | Het | $R_f$ / Solvens | Bsp.-Nr. / Literatur für das Eduktamin |
|---|---|---|---|---|---|
| 66 | rac | rac | 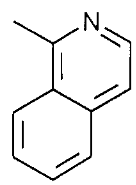 | 0,37 / J | Beispiel XXIV |
| 67 | rac | rac | 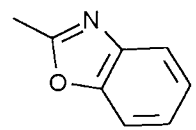 | 0,59 / M | T.P. Sycheva et al., Chem. Heterocycl. Compounds 1966, 529. |
| 68 | rac | rac | 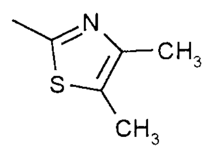 | 0,65 / B | Beispiel XXV |
| 69 | rac | rac | 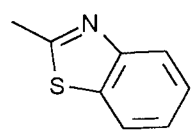 | 0,61 / M | UUS 44 25 338 |

| Bsp.-Nr. | 1 | 2 | Het | $R_f$ / Solvens | Bsp.-Nr. / Literatur für das Eduktamin |
|---|---|---|---|---|---|
| 70 | rac | rac | 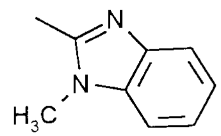 | 0,61 / B | V.M. Aruyzina et al., Chem. Heterocycl. Compounds 1966, 460. |
| 71 | rac | rac | 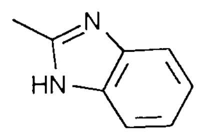 | 0,17 / K | Samia M. Rida et al., Pharmazie 41, 563 (1986). |
| 72 | rac | rac | 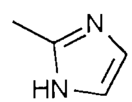 | 0,25 / A | Beispiel XXVI |

**Beispiel 73**

2-[4-(Chinolin-2-yl-methoxy)phenyl]-2-cycloheptyl-essigsäure-(phenylglycinethylester)-amid

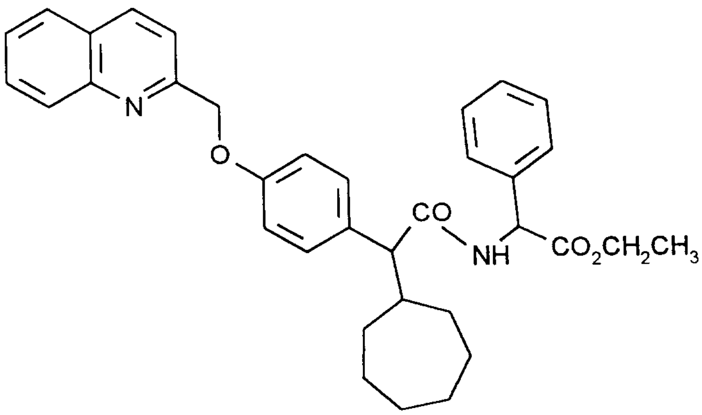

Analog der Herstellungsvorschrift der Beispiele XXXII und XXXIII werden (R,S)-2-[4-(Chinolin-2-yl-methoxy)phenyl]-2-cycloheptyl-essigsäure (US 4 970 215) und D,L-Phenylglycinethylester (Maybridge) miteinander umgesetzt.
$R_f$ = 0,12 (T)

**Beispiel 74**

2-[4-(Chinolin-2-yl-methoxy)phenyl]-2-cycloheptyl-essigsäure-(phenylglycin)-amid

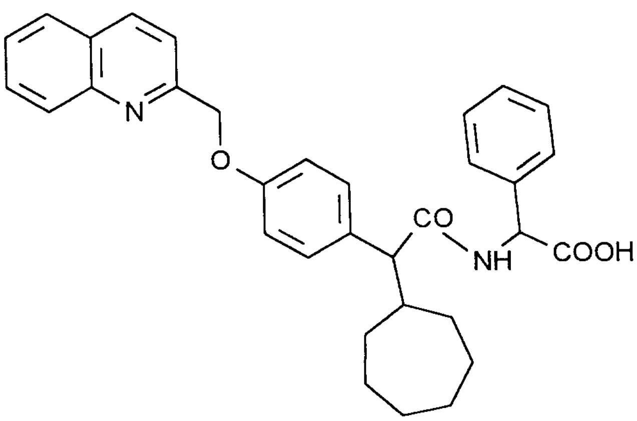

18,2 g der Verbindung aus Beispiel 73 werden unter Rückfluß mit 160 ml wäßriger 2M Natronlauge in 40 ml Ethanol umgesetzt. Nach 2,5 Stunden wird der Ethanolanteil größtenteils im Vakuum entfernt. Die partiell eingeengte Reaktionsmischung wird mit Wasser verdünnt und mehrfach mit Ether extrahiert. Darauf zieht man im Vakuum den Restanteil organischen Lösemittels aus der wäßrigen Phase ab, stellt bei 0°C mit 2 M Salzsaure einen pH-Wert von 2 ein und saugt den anfallenden Niederschlag ab. Der Niederschlag wird mehrmals mit Wasser gewaschen und nach letztmaligem Absaugen im Vakuum über Phosphorpentoxid und festem Natriumhydroxid getrocknet.
Ausbeute: 11,8 g
$R_f$ = 0,21 (B)

**Beispiel 75**

2-[4-(Chinolin-2-yl-methoxy)-phenyl]-2-cycloheptyl-essigsäure-N-(phenylglycin-amid)-amid

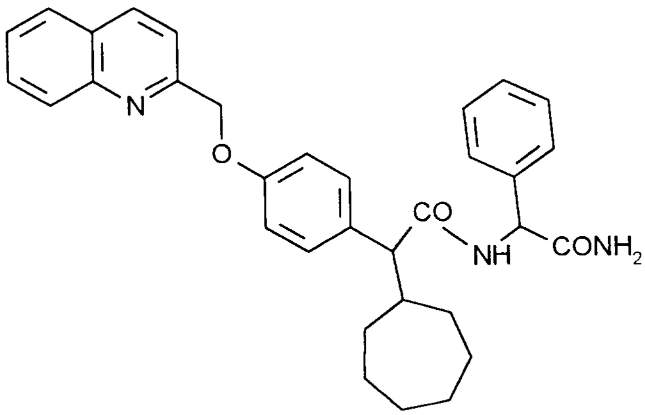

200 mg der Verbindung aus Beispiel 73 wird in einer gesättigten Lösung von Ammoniak in Methanol gelöst und einen Tag unter Rückfluß zur Reaktion gebracht. Das Reaktionsgemisch wird im Vakuum eingedampft und zuletzt im Hochvakuum über Phosphorpentoxid getrocknet.
Ausbeute: 190 mg
$R_f$ = 0,28 (C)

In Analogie zu den in der Tabelle 8 aufgeführten Vorschriften werden die in Tabelle 8 aufgeführten Verbindungen hergestellt:

**Tabelle 8:**

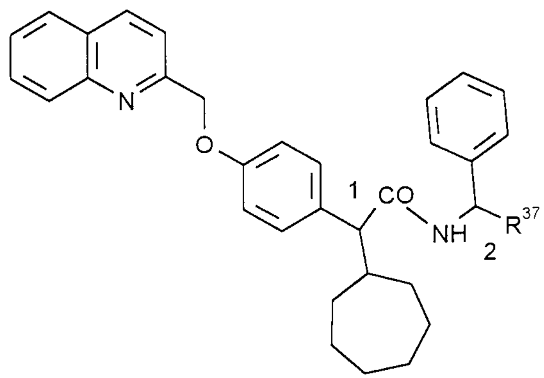

| Bsp.-Nr. | 1 | 2 | $R^{37}$ | $R_f$ / Solvens | Literatur / Bsp.-Nr. des Ausgangsmaterials | Herstellung analog Bsp.-Nr. |
|---|---|---|---|---|---|---|
| 76 | rac | rac | $-CONHCH_3$ | 0,39 / C | Beispiel 73 | 75 ** |
| 77 | rac | rac | $-CON(CH_3)_2$ | 0,33 / D | Beispiel 74 | XXXII / XXXIII |
| 78 | rac | rac | -CONHnBu | 0,15 / D | Beispiel 73 | 75 ** |
| 79 | rac | rac | 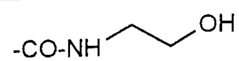 | 0,55 / B | Beispiel 73 | 75 ** |

| Bsp.-Nr. | 1 | 2 | $R^{37}$ | $R_f$ / Solvens | Literatur / Bsp.-Nr. des Ausgangsmaterials | Herstellung analog Bsp.-Nr. |
|---|---|---|---|---|---|---|
| 80 | rac | rac | 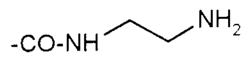 | 0,3 / U | Beispiel 73 | 75 *** |
| 81 | rac | rac | 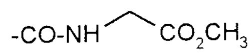 | 0,53 / C | Beispiel 74 | XXXII / XXXIII |
| 82 | rac | rac | 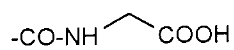 | 0,2 / B | Beispiel 81 | 74 |
| 83 | rac | rac | 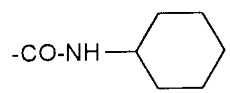 | 0,38 / C | Beispiel 74 | XXXII / XXXIII |
| 84 | rac | rac | 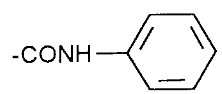 | 0,28 / D | Beispiel 74 | XXXII / XXXIII |
| 85 | rac | rac | 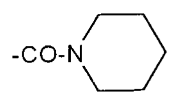 | 0,18 /D | Beispiel 74 | XXXII / XXXIII |
| 86 | rac | rac | 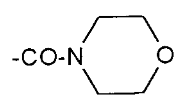 | 0,41 / D | Beispiel 74 | XXXII / XXXIII |

| Bsp.-Nr. | 1 | 2 | $R^{37}$ | $R_f$ / Solvens | Literatur / Bsp.-Nr. des Ausgangsmaterials | Herstellung analog Bsp.-Nr. |
|---|---|---|---|---|---|---|
| 87 | rac | rac | 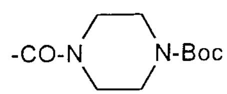 | 0,34 / D | Beispiel 74 * | XXXII / XXXIII |

* = Das Eduktamin (tert.Butyl 1-Piperazincarboxylat) ist bei Aldrich käuflich.

** = Das Reaktionslösemittel ist Ethanol.

*** = Das umzusetzende Amin ist das Reaktionslösemittel.

**Beispiel 88**

2-[4-(Chinolin-2-yl-methoxy)phenyl]-2-cycloheptyl-essigsäure-n-[α-(piperazin-1-yl-carbonyl)benzyl]amid

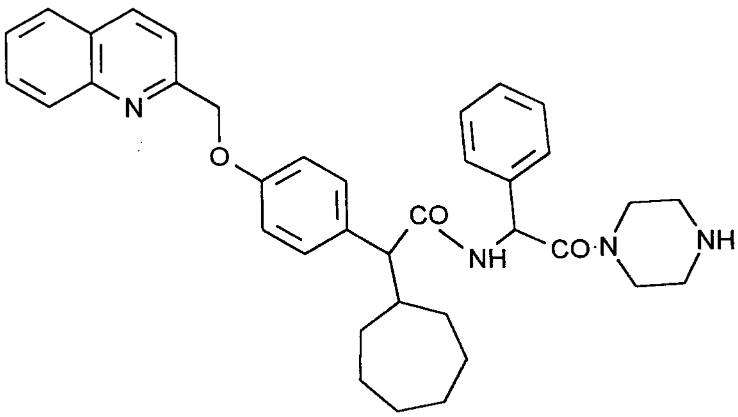

0,63 der Verbindung aus Beispiel 87 werden in 5 ml Essigester gelöst und bei Raumtemperatur mit 8 ml 4 M Salzsäure umgesetzt. Nach 23 Stunden setzt man Essigester zu und extrahiert die organische Phase mit 2 M Salzsäure. Die saure wäßrige Phase wird im Vakuum von organischem Restlösemittel befreit und bei ca. 10°C mit 10%iger Natronlauge (10 g festes NaOH auf 90 ml Wasser) auf pH = 14 gestellt. Der anfallende Niederschlag wird abgesaugt, mit Wasser gewaschen und im Vakuum über Phosphorpentoxid getrocknet.
Ausbeute: 0,48 g.
$R_f$ = 0,19 (C)

**Beispiel 89**

2-[4-(Chinolin-2-yl-methoxy)phenyl]-2-cycloheptyl-essigsäure-N-[α-(4-N-acetyl-piperazin-1-yl-carbonyl)benzyl]amid

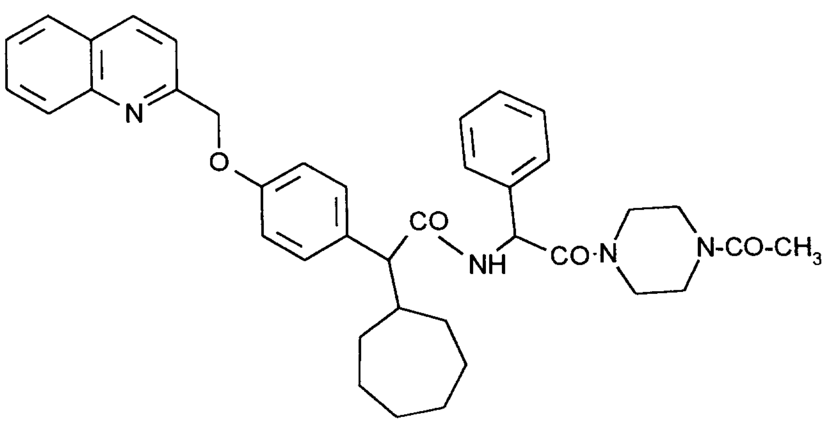

150 mg der Verbindung aus Beispiel 88 werden bei Raumtemperatur in 4 ml Dichlormethan mit 0,04 ml Triethylamin und 0,018 ml Acetylchlorid zur Reaktion gebracht. Nach 1,5 Stunden extrahiert man mit einem Puffer von pH = 4, schüttelt die wäßrige Phase mit Dichlormethan und dampft die organische Phase nach Natriumsulfattrocknung ein. Das rohe Produkt wird durch Säulenfiltration (Kieselgel 60, Merck 63-200 µm, Dichlormethan) gereinigt.

Ausbeute: 160 mg
$R_f$ = 0,36 (Dichlormethan : Methanol = 20:1)

**Beispiel 90**

2-[4-(Chinolin-2-yl-methoxy)-phenyl]-2-cycloheptyl-essigsäure-N-[α-(4N-methansulfonyl-piperazin-1-yl-carbonyl)benzyl]amid

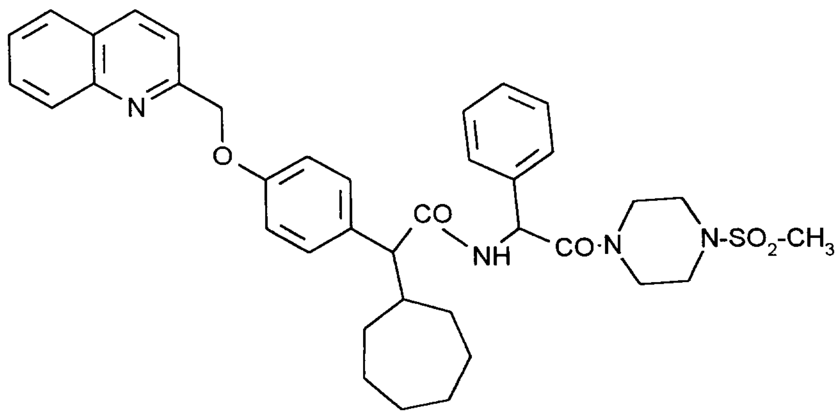

Analog der Herstellung des Beispiels 89 wird die Verbindung aus Beispiel 88 mit Methansulfonylchlorid zur Reaktion gebracht.
$R_f$ = 0,82 (C)

Die Verbindungen aus der Tabelle 9 werden in Analogie zu den Vorschriften der Beispiele XXXII, XXXIII, 74 und 75 hergestellt:

**Tabelle 9:**

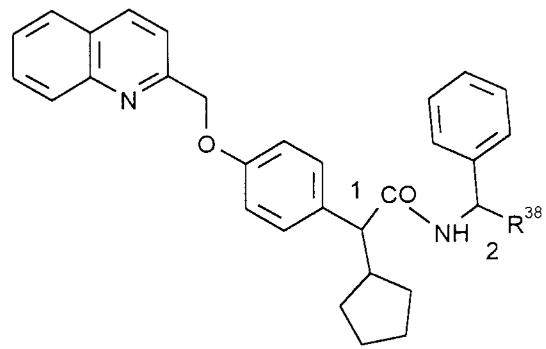

| Bsp.-Nr. | 1 | 2 | $R^{38}$ | $R_f$ / Solvens / Fp. | Vertreiber / Literatur der Eduktamine | Herstellung analog Bsp.-Nr. |
|---|---|---|---|---|---|---|
| 91 | S | R | $-CO_2CH_3$ | 0,43 / X | Nipkayaku | XXXII / XXXIII |
| 92 | S | R | -COOH | Fp.= 194-196°C | Beispiel 91 | Beispiel 74 |
| 93 | rac | rac | $-CO_2C_2H_5$ | 0,5 / D | Maybridge | XXXII / XXXIII |
| 94 | S | R | $-CH_3$ | Fp. = 146°C | Aldrich | XXXII / XXXIII |
| 95 | rac | rac | $-C_2H_5$ | 0,29 / D | Norse | XXXII / XXXIII |
| 96 | S | rac | $-CONH_2$ | 0,26 / Z | Beispiel 91 | Beispiel 75 |

| Bsp.-Nr. | 1 | 2 | $R^{38}$ | $R_f$ / Solvens / Fp. | Vertreiber / Literatur der Eduktamine | Herstellung analog Bsp.-Nr. |
|---|---|---|---|---|---|---|
| 97 | rac | rac | $-CONHCH_3$ | 0,49 / C | Beispiel 93 | Beispiel 75 * |
| 98 | rac | rac | 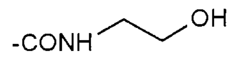 | 0,56 / B | Beispiel 93 | Beispiel 75 * |
| 99 | rac | rac | -CN | 0,34 / D | Aldrich | XXXII / XXXIII |
| 100 | rac | rac | $-CF_3$ | 0,22 / T | J.R. McCarthy et al., Tetrahedron Lett. 31, 5547 (1990). | XXXII / XXXIII |
| 101 | rac | rac | 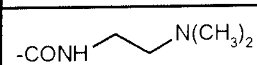 | 0,49 / B | Beispiel 93 | Beispiel 75 * |

* = Das Reaktionslösemittel ist Ethanol.

**Beispiel 102**

(2S)-2-[4-(Chinolin-2-yl-methoxy)-phenyl]-2-cycloheptyl-essigsäure-N-(1-phenyl-vinyl)amid

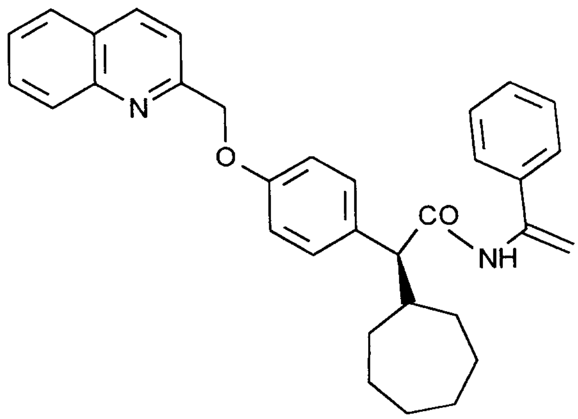

2,0 g der Verbindung aus Beispiel XXXIV werden bei -30°C in 20 ml wasserfreiem DMF mit 0,545 ml Triethylamin und 0,304 ml Mesylchlorid umgesetzt. Nach 2 Stunden setzt man weitere 1,09 ml Triethylamin zu und erwärmt auf Raumtemperatur. Nach 20 stündigem Nachrühren stellt man mit 2 M Salzsäure auf pH = 2 und extrahiert mit einem Ether-Essigester-Gemisch. Die organischen Phasen werden mit Magnesiumsulfat getrocknet und eingedamft. Das anfallende rohe Material wird säulenchromatographisch aufgereinigt (Kieselgel 60, Merck 40-63 µm, Petrolether:Essigester zuerst 5:1 dann 2:1).
Ausbeute: 1,3 g
$R_f$ = 0,20 (T)
charakteristische $^1$H-NMR-Signale ($CDCl_3$, 200 MHz, TMS): δ = 4,55 (dd, 1H);
5,16 (dd, 1H) (vinylische Protonen) ppm.

Die Verbindungen der folgenden Tabelle 10 werden analog der Vorschrift zu Beispiel 102 hergestellt:

**Tabelle 10:**

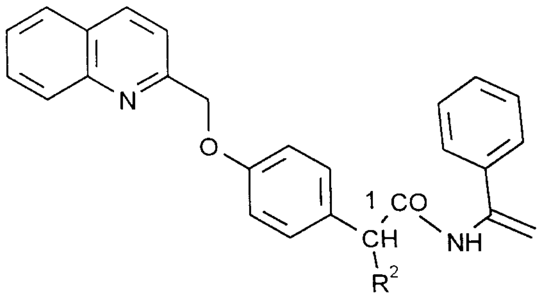

| Bsp.-Nr. | 1 | $R^2$ | $R_f$ / Solvens |
|---|---|---|---|
| 103 | S | 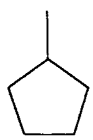 | 0,84 / O |
| 104 | rac | 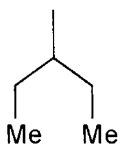 | 0,90 / B |
| 105 | rac | $-CH_3$ | 0,70 / A |
| 106 | rac | 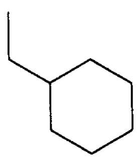 | 0,97 / B |
| 107 | rac | 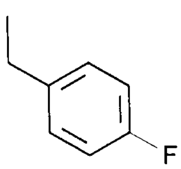 | 0,98 / B |

Die Synthese der Verbindungen aus Tabelle 11 verläuft analog der Vorschriften der Beispiele XXXII, XXXIII und 74.

**Tabelle 11:**

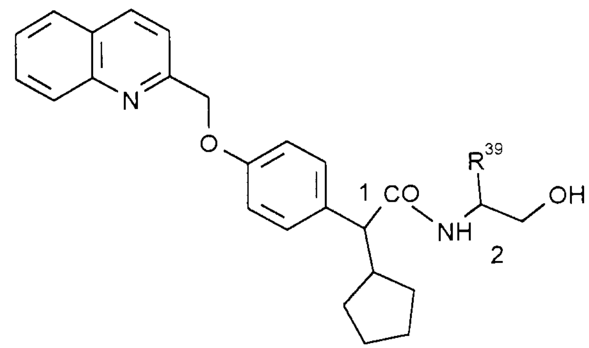

| Bsp.-Nr. | 1 | 2 | $R^{39}$ | $R_f$ (Solvens) / Fp. | Beispiel-Nr. / Vertreiber der Eduktamine | Herstellung analog Bsp.-Nr. |
|---|---|---|---|---|---|---|
| 108 | S | / | H | Fp.= 143°C | Aldrich | XXXII / XXXIII |
| 109 | S | R | Et | Fp.= 167-170°C (Toluol) | Aldrich | XXXII / XXXIII |
| 110 | S | R | iBu | Fp.= 155°C (Toluol) | Aldrich | XXXII / XXXIII |
| 111 | S | / | $CH_2OH$ | 0,20 (W) | Aldrich | XXXII / XXXIII |
| 112 | S | S | $CO_2Me$ | Fp.= 173-175°C | Aldrich | XXXII / XXXIII |
| 113 | S | S | COOH | Fp.= 183-185°C | Beispiel 112 | Beispiel 74 |

Die Herstellung der Verbindungen aus Tabelle 12 verläuft analog der Vorschrift zu den Beispielen XXXII und XXXIII.

**Tabelle 12:**

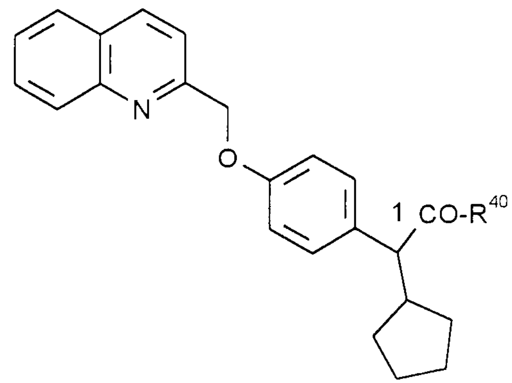

| Bsp.-Nr. | 1 | $R^{40}$ | $R_f$ / Solvens | Bsp.-Nr. / Literatur / Vertreiber der Eduktamine |
|---|---|---|---|---|
| 114 | rac | 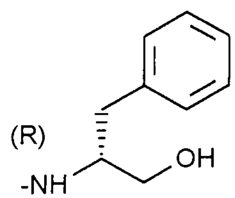 | 0,26 / C | Aldrich |

| Bsp.-Nr. | 1 | $R^{40}$ | $R_f$ / Solvens | Bsp.-Nr. / Literatur / Vertreiber der Eduktamine |
|---|---|---|---|---|
| 115 | S | 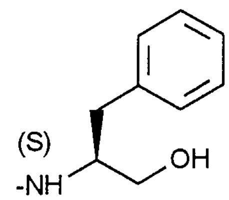 | Fp.= 158°C (Ethanol) | Aldrich |
| 116 | R | 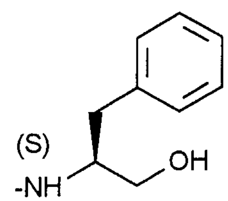 | Fp.= 132°C (Toluol/Essigester) | Aldrich |
| 117 | rac | 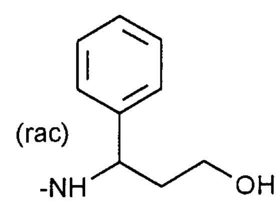 | 0,73 / B | DE 26 28 469 |

| Bsp.-Nr. | 1 | $R^{40}$ | $R_f$ / Solvens | Bsp.-Nr. / Literatur / Vertreiber der Eduktamine |
|---|---|---|---|---|
| 118 | rac | (rac) 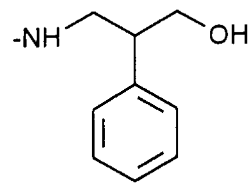 | 0,77 / B | DE 26 28 469 |
| 119 | rac | (rac) 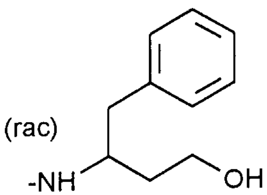 | 0,51 / C | DE 26 28 469 |
| 120 | rac | (rac) 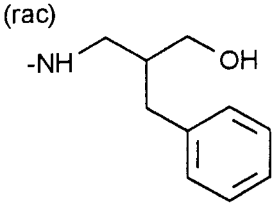 | 0,21 / D | H.V. Secor et al., J. Org. Chem. 43, 2539 (1978). |

| Bsp.-Nr. | 1 | $R^{40}$ | $R_f$ / Solvens | Bsp.-Nr. / Literatur / Vertreiber der Eduktamine |
|---|---|---|---|---|
| 121 | rac | (rac) 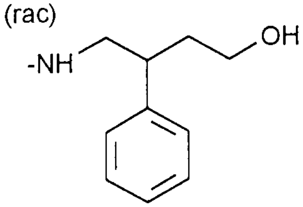 | 0,13 / D | Khim. Farm. Zh. 25, 60 (1991) |
| 122 | rac | (rac) 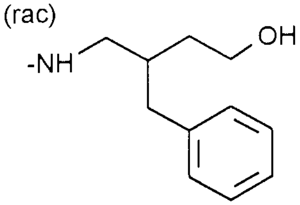 | 0,94 / B | Beispiel XXXI |
| 123 | rac | (rac) 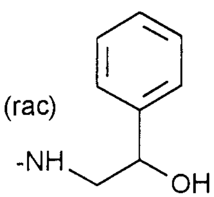 | 0,76 / B | Sigma |

| Bsp.-Nr. | 1 | $R^{40}$ | $R_f$ / Solvens | Bsp.-Nr. / Literatur / Vertreiber der Eduktamine |
|---|---|---|---|---|
| 124 | rac | (rac) 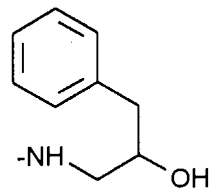 | 0,15 / D | WO 91 / 18 897 |
| 125 | rac | (rac) 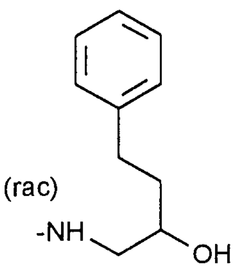 | 0,19 / C | DE 36 43 012 |

Analog der Vorschrift zu den Beispielen XXXII und XXXIII lassen sich die Verbindungen aus Tabelle 13 herstellen:

**Tabelle 13:**

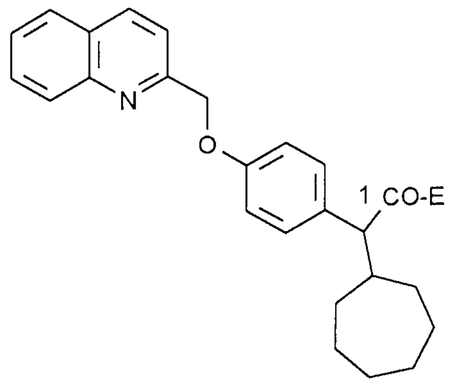

| Bsp.-Nr. | 1 | E | $R_f$ / Solvens | Literatur / Vertreiber der Eduktamine |
|---|---|---|---|---|
| 126 | S | 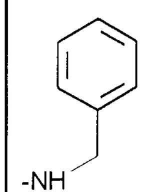 | 0,51 / D | Aldrich |

| Bsp.-Nr. | 1 | E | $R_f$ / Solvens | Literatur / Vertreiber der Eduktamine |
|---|---|---|---|---|
| 127 | S | 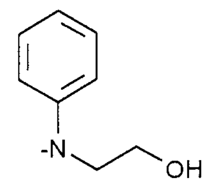 | 0,80 / V | Aldrich |
| 128 | S | 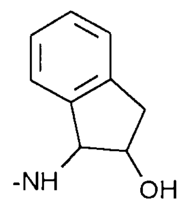 | 0,13 / D | P & B |
| 129 | S | 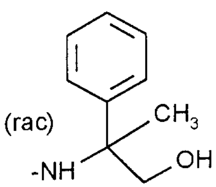 (rac) | 0,51 / O | EP 514 267 |

In Analogie zu den Vorschriften der Beispiele XXXII und XXXIII lassen sich die Verbindungen aus Tabelle 14 herstellen:

**Tabelle 14:**

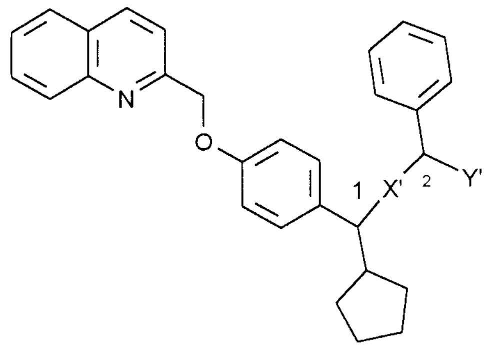

| Bsp.-Nr. | 1 | 2 | X' | Y' | $R_f$ / Solvens | Literatur der Ausgangsverbindungen |
|---|---|---|---|---|---|---|
| 130 | rac | rac | $CON(CH_3)$ | $CH_2OH$ | 0,89 / B | GB 14 34 826. |
| 131 | rac | rac | CONH | $CH_2OCH_3$ | 0,17 / G | A.I. Meyers et al., J. Org. Chem. 43, 892 (1978). |
| 132 | rac | rac | $CON(CH_3)$ | $CH_2OCH_3$ | 0,19 / G | B.E. Rossiter et al., Tetrahedron 49, 965 (1993). |
| 133 | rac | rac | COO | $CH(OC_2H_5)_2$ | 0,68 / D | P. Tinapp, Arch. Pharm. 310, 89 (1977). |
| 134 | rac dia A | | CO-S- | $CH_2OH$ | 0,42 / H | Y. Gareau et al., Tetrahedron Lett. 34, 3363 (1993). |
| 135 | rac dia B | | CO-S- | $CH_2OH$ | 0,39 / H | Y. Gareau et al., Tetrahedron Lett. 34, 3363 (1993). |

**Beispiel 136**

2-[4-(Chinolin-2-yl-methoxy)-phenyl]-2-cyclopentyl-essigsäure-(α-formyl)benzylester

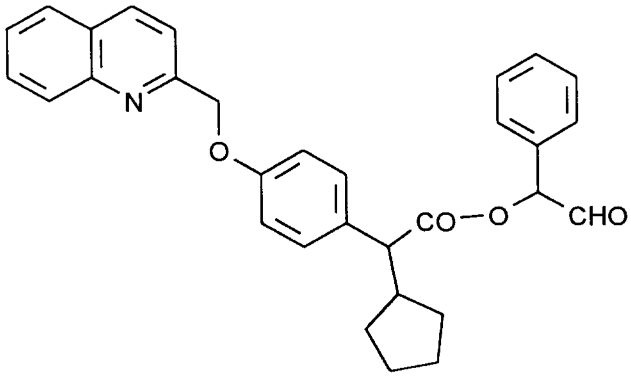

3,5 g der Verbindung aus Beispiel 133 werden in 60 ml Dioxan gelöst und bei Raumtemperatur 4 Stunden mit 20 ml konzentrierter Salzsäure umgesetzt. Darauf wird mit einem Gemisch aus Ether und wäßriger Natriumcarbonatlösung extrahiert. Die organische Phase wird mit Wasser gewaschen, mit Magnesiumsulfat getrocknet und eingedampft. Das Rohprodukt wird säulenchromatographisch gereinigt (Kieselgel 60, Merck, 40-63 µm, Petrolether:Essigester = zuerst 4:1, dann 2:1, zuletzt 1:1).
Ausbeute: 1,7 g
$R_f$ = 0,10 (V)

**Beispiel 137**

2-[4-(Chinolin-2-yl-methoxy)phenyl]-2-cyclopentyl-essigsäure-(α-hydroxymethyl)benzylester

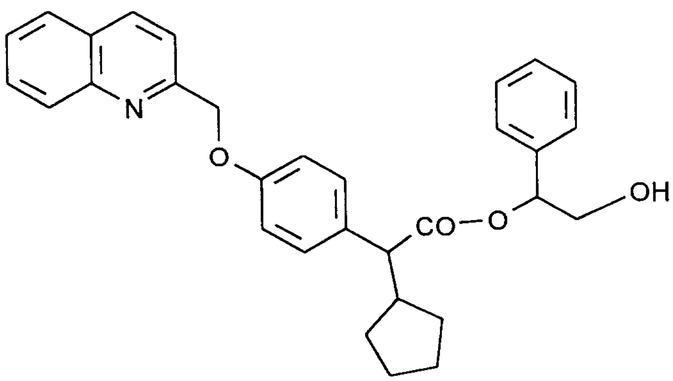

0,46 g der Verbindung aus Beispiel 136 werden in 20 ml Ethanol bei Raumtemperatur mit 40 mg Natriumboranat zur Reaktion gebracht. Nach 30 Minuten gießt man auf Wasser und extrahiert mit Essigester. Die organischen Phasen werden mit Magnesiumsulfat getrocknet und im Vakuum vom Lösemittel befreit. Das erhaltene Rohprodukt wird säulenchromatographisch gereinigt (Kieselgel 60, Merck, 40-63 µm, Petrolether:Essigester = 7:1, später 3:1).
Ausbeute: 0,12 g
$R_f$ = 0,41 (H)

**Beispiel 138**

2-[4-(Chinolin-2-yl-methoxy)phenyl]-2-cyclopentyl-essigsäure-($\alpha$-acetoxymethyl)thiobenzylester

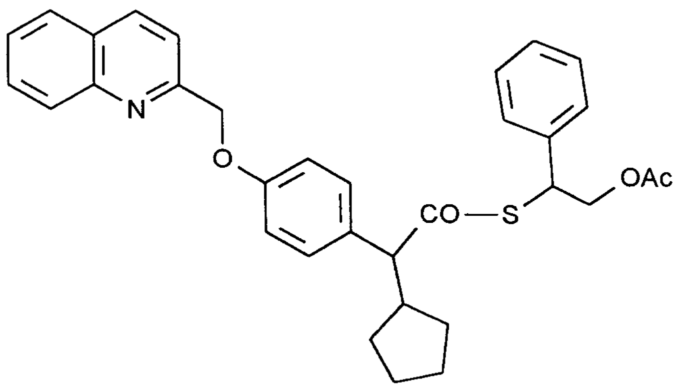

Analog der Vorschrift für Beispiel 89 läßt sich die Verbindung (Diastereomerengemisch) aus Beispiel 134 und 135 in die Titelverbindung überführen.
$R_f$ = 0,28 (T)

In Analogie zu den Vorschriften der Beispiele XXXII und XXXIII werden die in der Tabelle 15 aufgeführten Verbindungen hergestellt:

**Tabelle 15:**

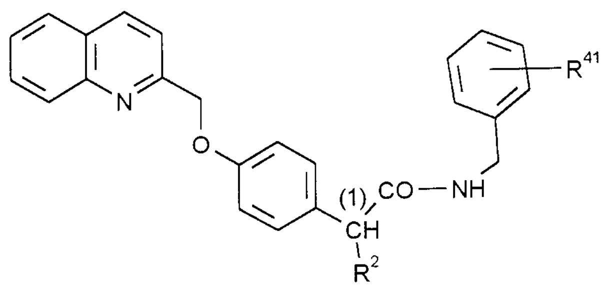

| Bsp.-Nr. | abs. Konfig. (1) | $R^2$ | $R^{41}$ | $R_f$ (Solvens) / Festpunkt | Vertreiber der Benzylamine |
|---|---|---|---|---|---|
| 139 | S | 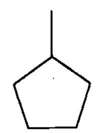 | H | Fp.= 138°C | Aldrich |
| 140 | R | 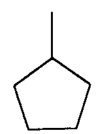 | H | Fp.= 134°C | Aldrich |

| Bsp.-Nr. | abs. Konfig. (1) | $R^2$ | $R^{41}$ | $R_f$ (Solvens) / Festpunkt | Vertreiber der Benzylamine |
|---|---|---|---|---|---|
| 141 | S | 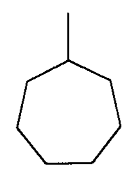 | H | 0,41 (D) | Aldrich |
| 142 | R | 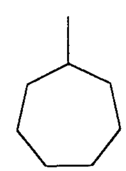 | H | 0,41 (D) | Aldrich |
| 143 | S | 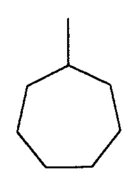 | 2-Cl | 0,31 (Y) | Aldrich |

| Bsp.-Nr. | abs. Konfig. (1) | $R^2$ | $R^{41}$ | $R_f$ (Solvens) / Festpunkt | Vertreiber der Benzylamine |
|---|---|---|---|---|---|
| 144 | S | 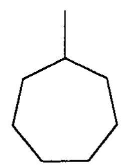 | 3-Cl | 0,26 (Y) | Maybridge |
| 145 | S | 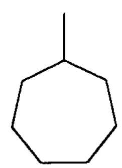 | 4-Cl | 0,30 (Y) | Aldrich |
| 146 | S | 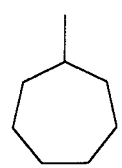 | 4-F | 0,32 (Y) | Aldrich |

**Beispiel 147**

(2R)-[4-(Chinolin-2-yl-methoxy)-phenyl]-2-cyclopentyl-essigsäure-(S)-(O-acetyl)-phenyl-glycinol-amid

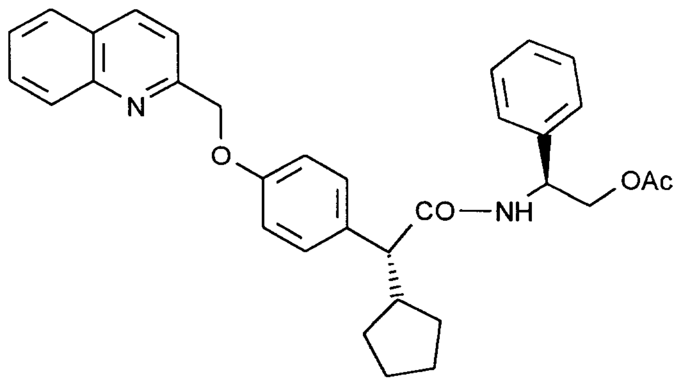

In Analogie zur Vorschrift des Beispiels 1 wird (2R)-2[4-(Chinolin-2-yl-methoxy)-phenyl]-2-cyclopentyl-essigsäure-(S)-phenylglycinol-amid [US 4 970 215] zur Titelverbindung umgesetzt.
$R_f$ = 0,47 (Ethanol:Dichlormethan = 1:20)
Fp. = 173°C

Die Verbindungen der Tabelle 16 werden analog zu den Vorschriften der Beispiele XXXII und XXXIII hergestellt:

**Tabelle 16**

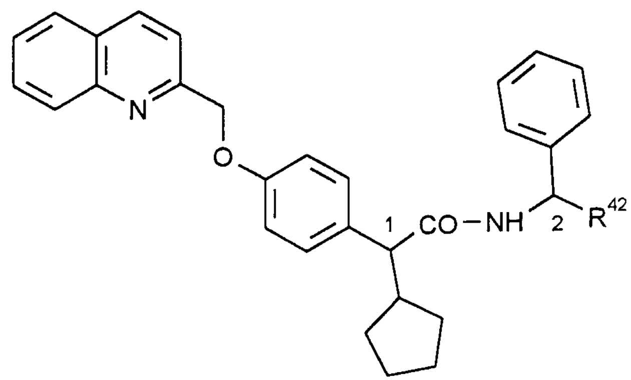

| Bsp.-Nr. | 1 | 2 | $R^{42}$ | Fließpunkt [°C] | Ausgangsmaterial (Amin)<br>a) Literatur<br>b) Vertreiberfirma |
|---|---|---|---|---|---|
| 148 | R | S | Me | Fp. = 157°C | b) Aldrich |
| 149 | R | S | $CH_2OMe$ | Fp. = 147°C | a) A.I. Meyers et al., J. Org. Chem. 43, 892 (1978). |
| 150 | S | S | $CH_2OMe$ | Fp. = 146°C | a) A.I. Meyers et al., J. Org. Chem. 43, 892 (1978). |
| 151 | R | R | Me | Fp. = 145°C | b) Aldrich |
| 152 | S | R | Me | Fp. = 163°C | b) Aldrich |

Die Verbindungen der Tabelle 17 werden analog der zitierten Vorschriften hergestellt:

**Tabelle 17**

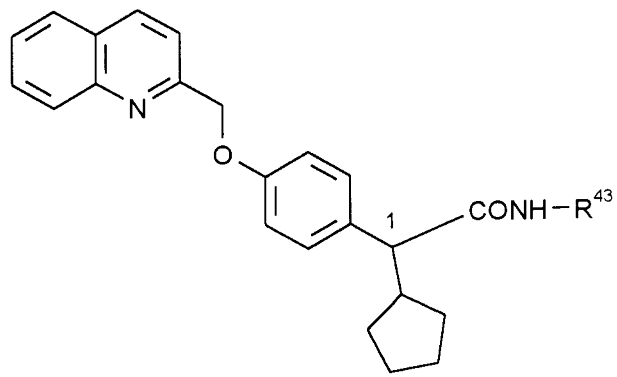

| Bsp.-Nr. | 1 | $R^{43}$ | $R_f$-Wert (Solvenz)<br>Fließpunkt [°C] | Ausgangsmaterial<br>a) Literatur<br>b) Vertreiberfirma*<br>c) Herstellung analog Bsp.-Nr. |
|---|---|---|---|---|
| 153 | R | Et | Fp. = 142°C | b) Aldrich |
| 154 | S | Et | Fp. = 145°C | b) Aldrich |
| 155 | R | $CH_2CH_2OH$ | Fp. = 143°C | b) Aldrich |
| 156 | rac | $CH_2CH_2OH$ | Fp. = 130°C | b) Aldrich |
| 157 | rac | $CH_2COOH$ | Fp. = 182-185°C | c) analog Bsp.-Nr. 74<br>(aus Bsp. Nr. 158) |

| Bsp.-Nr. | 1 | 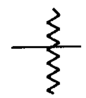 R43 | Rf-Wert (Solvenz) Fließpunkt [°C] | Ausgangsmaterial<br>a) Literatur<br>b) Vertreiberfirma*<br>c) Herstellung analog Bsp.-Nr. |
|---|---|---|---|---|
| 158 | rac | $CH_2CO_2Me$ | Fp. = 134-136°C | b) Aldrich |
| 159 | S | 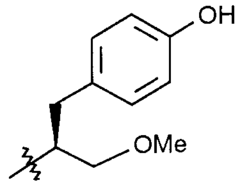 | Fp. = 176°C | |
| 160 | R | 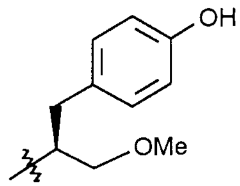 | Fp. = 123°C | |
| 161 | S | 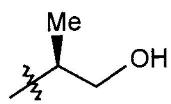 | Fp. = 134°C | b) Aldrich |
| 162 | R | 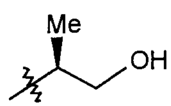 | Fp. = 184°C | b) Aldrich |

| Bsp.-Nr. | 1 | 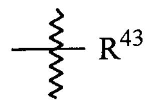 | $R_f$-Wert (Solvenz)<br>Fließpunkt [°C] | Ausgangsmaterial<br>a) Literatur<br>b) Vertreiberfirma*<br>c) Herstellung analog Bsp.-Nr. |
|---|---|---|---|---|
| 163 | R | 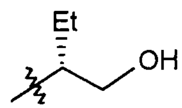 | Fp. = 168°C | b) Aldrich |
| 164 | R | 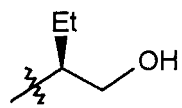 | Fp. = 170°C | b) Aldrich |
| 165 | S | 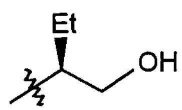 | Fp. = 142°C | b) Aldrich |
| 166 | R | 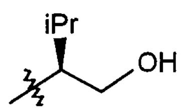 | Fp. = 158°C | b) Sigma |
| 167 | S | 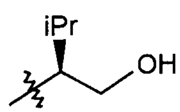 | Fp. = 186°C | b) Sigma |

| Bsp.-Nr. | 1 | 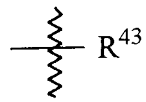 | $R_f$-Wert (Solvenz) Fließpunkt [°C] | Ausgangsmaterial a) Literatur b) Vertreiberfirma* c) Herstellung analog Bsp.-Nr. |
|---|---|---|---|---|
| 168 | S | 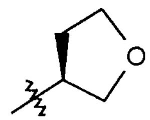 | Fp. = 160°C | b) D-Schuchardt |
| 169 | R | 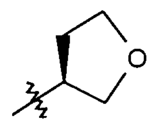 | Fp. = 137°C | b) D-Schuchardt |
| 170 | S | 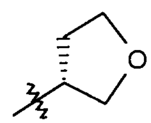 | Fp. = 139°C | b) D-Schuchardt |
| 171 | R | 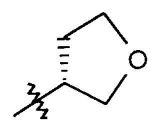 | Fp. = 160°C | b) D-Schuchardt |

| Bsp.-Nr. | 1 | 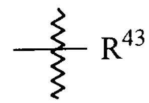 | $R_f$-Wert (Solvenz) Fließpunkt [°C] | Ausgangsmaterial a) Literatur b) Vertreiberfirma* c) Herstellung analog Bsp.-Nr. |
|---|---|---|---|---|
| 172 | S | 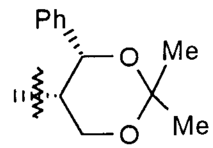 | $R_f$ = 0,48 (AA) | b) Sigma |
| 173 | R | 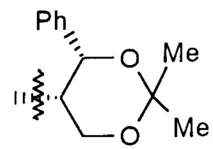 | $R_f$ = 0,43 (AA) | b) Sigma |
| 174 | R | 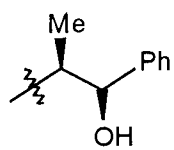 | Fp. = 233°C | b) Sigma |
| 175 | S | 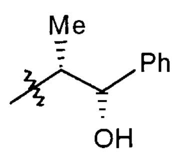 | Fp. = 234°C | b) Sigma |

| Bsp.-Nr. | 1 | 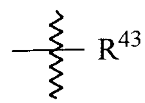 | $R_f$-Wert (Solvenz)<br>Fließpunkt [°C] | Ausgangsmaterial<br>a) Literatur<br>b) Vertreiberfirma*<br>c) Herstellung analog Bsp.-Nr. |
|---|---|---|---|---|
| 176 | R | 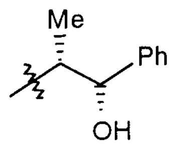 | Fp. = 219°C | b) Sigma |
| 177 | S | 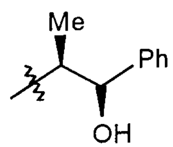 | Fp. = 214°C | b) Sigma |
| 178 | R | 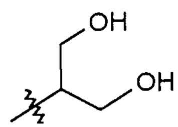 | $R_f$ = 0,12 (W)<br>Fp. = 161°C | b) Aldrich |
| 179 | S | 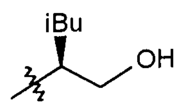 | Fp. = 152°C | b) Aldrich |
| 180 | R | 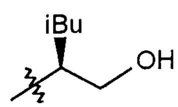 | Fp. = 154°C | b) Aldrich |

| Bsp.-Nr. | 1 | 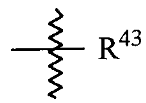 | $R_f$-Wert (Solvenz) Fließpunkt [°C] | Ausgangsmaterial a) Literatur b) Vertreiberfirma* c) Herstellung analog Bsp.-Nr. |
|---|---|---|---|---|
| 181 | R | 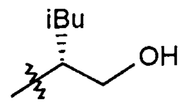 | Fp. = 155°C | b) Aldrich |
| 182 | S | 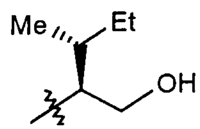 | Fp. = 164°C | b) Aldrich |
| 183 | R | 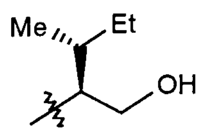 | Fp. = 134°C | b) Aldrich |
| 184 | R | (L)- 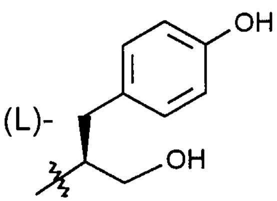 | Fp. = 200°C | b) Sigma |

| Bsp.-Nr. | 1 | 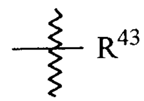 | $R_f$-Wert (Solvenz) Fließpunkt [°C] | Ausgangsmaterial<br>a) Literatur<br>b) Vertreiberfirma*<br>c) Herstellung analog Bsp.-Nr. |
|---|---|---|---|---|
| 185 | S | (L)- 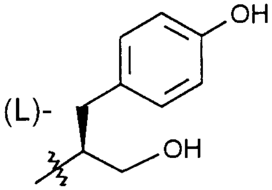 | Fp. = 190°C | b) Sigma |
| 186 | R | 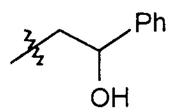 | Fp. = 168°C | b) Aldrich |
| 187 | R | 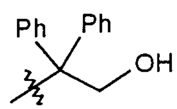 | $R_f$ = 0,37 (AF) | a) G. Bittner et al., Justus Liebigs Ann. Chem. 713, 1 (1968). |
| 188 | R | 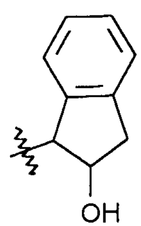 | $R_f$ = 0,18 (X) | b) P & B |

| Bsp.-Nr. | 1 | 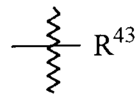 | $R_f$-Wert (Solvenz) Fließpunkt [°C] | Ausgangsmaterial<br>a) Literatur<br>b) Vertreiberfirma*<br>c) Herstellung analog Bsp.-Nr. |
|---|---|---|---|---|
| 189 | S | 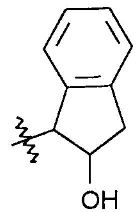 | $R_f$ = 0,45 (X) | b) P & B |
| 190 | R | 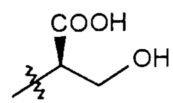 | $R_f$ = 0,01 (AH) | c) analog Bsp.-Nr. 74<br>(aus Bsp. Nr. 193) |
| 191 | S | 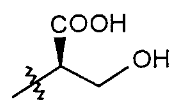 | $R_f$ = 0,02 (AH) | c) analog Bsp.-Nr. 74<br>(aus Bsp.-Nr. 192) |
| 192 | S | 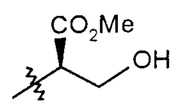 | $R_f$ = 0,14 (AH) | b) Chemalog |
| 193 | R | 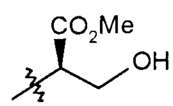 | $R_f$ = 0,21 (AH) | b) Chemalog |

| Bsp.-Nr. | 1 | 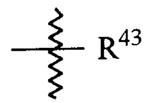 | $R_f$-Wert (Solvenz)<br>Fließpunkt [°C] | Ausgangsmaterial<br>a) Literatur<br>b) Vertreiberfirma*<br>c) Herstellung analog Bsp.-Nr. |
|---|---|---|---|---|
| 194 | R | 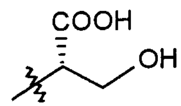 | $R_f$ = 0,01 (AE) | c) analog Bsp.-Nr. 74<br>(aus Bsp.-Nr. 195) |
| 195 | R | 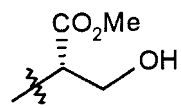 | $R_f$ = 0,21 (AH) | b) Chemalog |
| 196 | R | 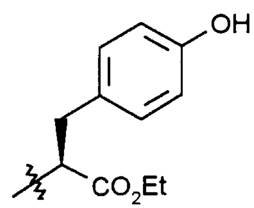 | $R_f$ = 0,38 (D) | b) Fluka |
| 197 | S | 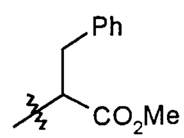 | $R_f$ = 0,50 (X) | b) Sigma |

| Bsp.-Nr. | 1 | 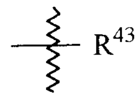 | $R_f$-Wert (Solvenz)<br>Fließpunkt [°C] | Ausgangsmaterial<br>a) Literatur<br>b) Vertreiberfirma*<br>c) Herstellung analog Bsp.-Nr. |
|---|---|---|---|---|
| 198 | S | 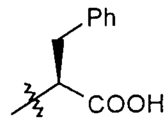 | $R_f$ = 0,02 (X) | c) analog Bsp.-Nr. 74 |
| 199 | R | 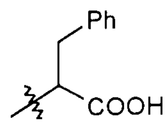 | $R_f$ = 0,02 (X) | c) analog Bsp.-Nr. 74 |
| 200 | S | 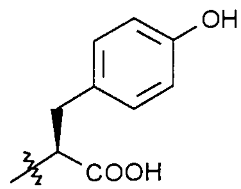 | $R_f$ = 0,01 (X) | c) analog Bsp.-Nr. 74 |
| 201 | R | 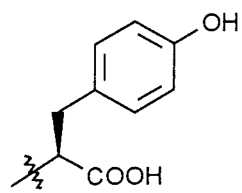 | $R_f$ = 0,01 (X) | c) analog Bsp.-Nr. 74 |

| Bsp.-Nr. | 1 | 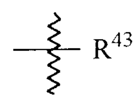 | $R_f$-Wert (Solvenz)<br>Fließpunkt [°C] | Ausgangsmaterial<br>a) Literatur<br>b) Vertreiberfirma*<br>c) Herstellung analog Bsp.-Nr. |
|---|---|---|---|---|
| 202 | R | 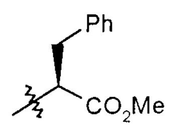 | $R_f$ = 0,50 (X) | b) Sigma |

* Die Angabe der Firma bezieht sich auf das Eduktamin; die Herstellung verläuft analog der Vorschrift zu den Beispielen XXXII und XXXIII.

Die Verbindungen der Tabelle 18 werden analog der Vorschriften von Beispiel-Nr. XXXII und XXXIII hergestellt:

**Tabelle 18**

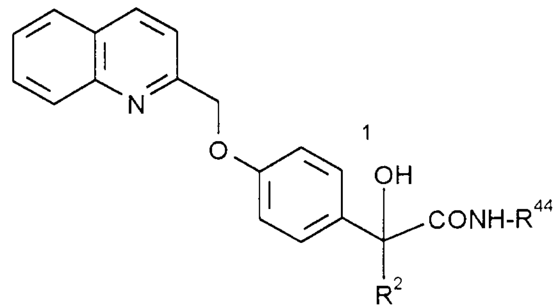

| Bsp.-Nr. | 1 | $R^2$ | 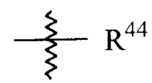 | $R_f$-Wert (Solvenz) Fließpunkt [°C] | Ausgangsmaterial a) Literatur b) Vertreiberfirma c) Herstellung analog Bsp.-Nr. |
|---|---|---|---|---|---|
| 203 | rac | cHept | 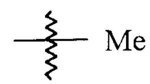 | Fp. = 163-166°C | b) Aldrich |
| 204 | rac | cHept | 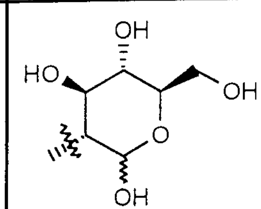 | $R_f$ = 0,13 (AC) | b) Lancaster |

| Bsp.-Nr. | 1 | $R^2$ | 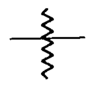 $R^{44}$ | $R_f$-Wert (Solvenz) Fließpunkt [°C] | Ausgangsmaterial<br>a) Literatur<br>b) Vertreiberfirma<br>c) Herstellung analog Bsp.-Nr. |
|---|---|---|---|---|---|
| 205 | rac | cHept | 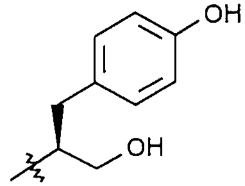 | $R_f$ = 0,46 (AE) | b) Sigma |
| 206 | rac | cHept | 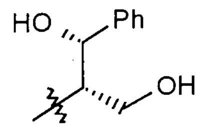 | $R_f$ = 0,37 (AE) | b) Aldrich |
| 207 | rac | cHept | 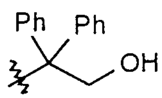 | $R_f$ = 0,85 (AD) | a) G. Bittner et al., Justus Liebigs Ann. Chem. 713, 1 (1968). |
| 208 | rac | cHept | 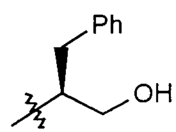 | $R_f$ = 0,57 (AE) | b) Aldrich |

| Bsp.-Nr. | 1 | $R^2$ | 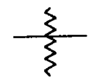 $R^{44}$ | $R_f$-Wert (Solvenz)<br>Fließpunkt [°C] | Ausgangsmaterial<br>a) Literatur<br>b) Vertreiberfirma<br>c) Herstellung analog Bsp.-Nr. |
|---|---|---|---|---|---|
| 209 | rac | cHept | 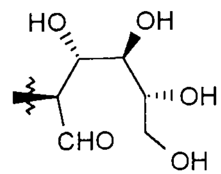 | $R_f$ = 0,03 (AC) | b) Aldrich |
| 210 | rac | cHept | 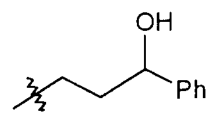 | $R_f$ = 0,61 (AG) | b) Columbia |
| 211 | rac | cHept | 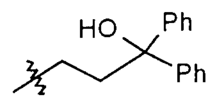 | $R_f$ = 0,28 (H) | a) DE 2 552 196 |
| 212 | rac | cHept | 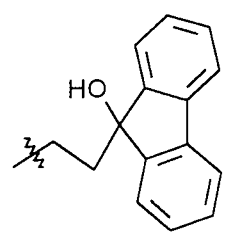 | $R_f$ = 0,62 (O) | a) GB 1 129 029 |

| Bsp.-Nr. | 1 | $R^2$ | 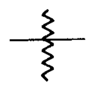 $R^{44}$ | $R_f$-Wert (Solvenz) Fließpunkt [°C] | Ausgangsmaterial<br>a) Literatur<br>b) Vertreiberfirma<br>c) Herstellung analog Bsp.-Nr. |
|---|---|---|---|---|---|
| 213 | rac | cHept | 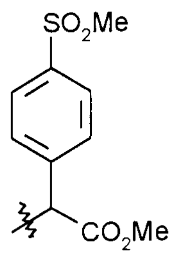 | $R_f$ = 0,64 (AF) | c) Bsp.-Nr. XL |

Die Verbindungen folgender Tabelle 19 werden analog der Vorschriften von Beispiel-Nr. XXXII, XXXIII und 74 hergestellt:

**Tabelle 19**

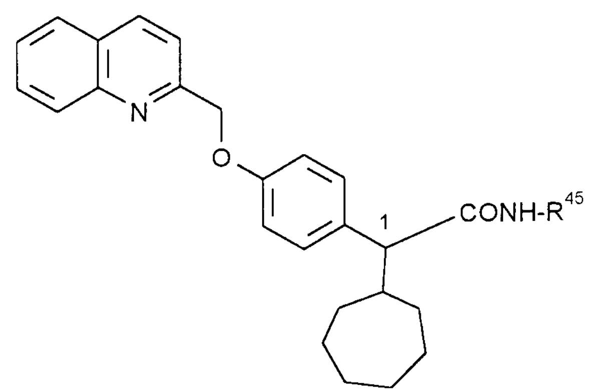

| Bsp.-Nr. | 1 | $R^{45}$ | $R_f$-Wert (Solvenz)<br>Fließpunkt [°C] | Ausgangsmaterial<br>a) Literatur<br>b) Vertreiberfirma<br>c) Herstellung analog Beispiel-Nr. |
|---|---|---|---|---|
| 214 | rac | $CH_2CH_2OH$ | Fp. = 91-94°C | b) Aldrich |
| 215 | R | 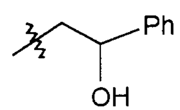 | $R_f$ = 0,44 (O) | b) Aldrich |
| 216 | R | 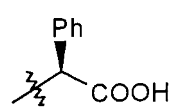 | $R_f$ = 0,11 (E) | c) analog Bsp.-Nr. 74 |

Die Verbindungen folgender Tabelle 20 werden analog der Vorschriften von Beispiel-Nr. XXXII, XXXIII und 74 hergestellt:

**Tabelle 20**

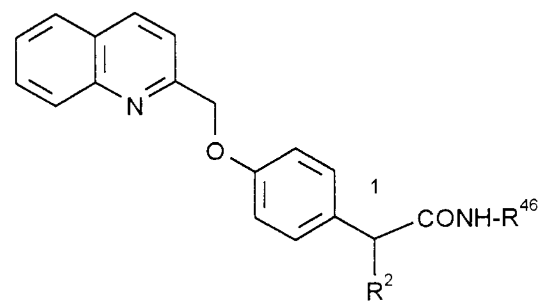

| Bsp.-Nr. | 1 | $R^2$ | 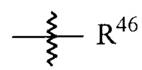 | Fließpunkt [°C] | Ausgangsmaterial<br>a) Literatur<br>b) Vertreiberfirma<br>c) Herstellung analog Bsp.-Nr. |
|---|---|---|---|---|---|
| 217 | rac | nBu | 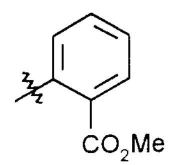 | Fp. = 174°C | b) Aldrich |
| 218 | rac | nBu | 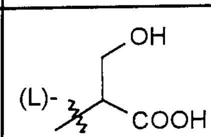 | Fp. = 165-170°C | c) analog Bsp.-Nr. 74<br>(aus Bsp.-Nr. 219) |

| Bsp.-Nr. | 1 | $R^2$ | 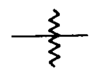 $R^{46}$ - | Fließpunkt [°C] | Ausgangsmaterial<br>a) Literatur<br>b) Vertreiberfirma<br>c) Herstellung analog Bsp.-Nr. |
|---|---|---|---|---|---|
| 219 | rac | nBu | 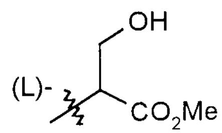 | Fp. = 157-159°C | b) Aldrich |
| 220 | rac | nBu | $CH_2COOH$ | Fp. = 173-175°C | c) analog Bsp.-Nr. 74<br>(aus Bsp.-Nr. 222) |
| 221 | rac | nBu | 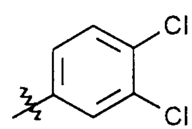 | Fp. = 187°C | b) Aldrich |
| 222 | rac | nBu | $CH_2CO_2Me$ | Fp. = 177°C | b) Aldrich |
| 223 | - | H | 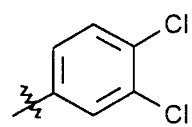 | Fp. = 193°C | b) Aldrich |

| Bsp.-Nr. | 1 | $R^2$ | 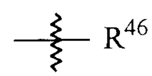 | Fließpunkt [°C] | Ausgangsmaterial<br>a) Literatur<br>b) Vertreiberfirma<br>c) Herstellung analog Bsp.-Nr. |
|---|---|---|---|---|---|
| 224 | - | H | 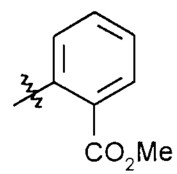 | Fp. = 187°C | b) Aldrich |

Die Verbindungen folgender Tabelle 21 werden analog der Vorschrift von Beispiel-Nr. XXXII, XXXIII und 90 hergestellt:

**Tabelle 21**

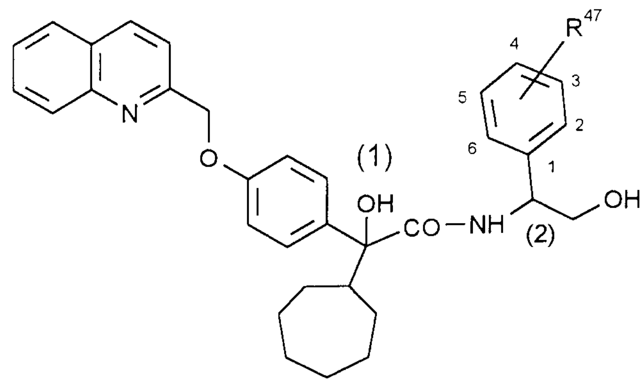

| Bsp.-Nr. | (1) | (2) | $R^{47}$ | $R_f$-Wert (Solvenz) | Ausgangsmaterial<br>a) Literatur<br>b) Vertreiberfirma<br>c) Herstellung analog Bsp.-Nr. |
|---|---|---|---|---|---|
| 225 | rac | rac | 4-Cl | $R_f$ = 0,52 (AD) | c) analog Bsp.-Nr. XXXII u. XXXIII (aus Bsp.-Nr. XX) |
| 226 | rac | rac | 3-Cl | $R_f$ = 0,69 (AE) | c) analog Bsp.-Nr. XXXII u. XXXIII (aus Bsp.-Nr. XIX) |

| Bsp.-Nr. | (1) | (2) | $R^{47}$ | $R_f$-Wert (Solvenz) | Ausgangsmaterial<br>a) Literatur<br>b) Vertreiberfirma<br>c) Herstellung analog Bsp.-Nr. |
|---|---|---|---|---|---|
| 227 | rac | rac | 4-Me | $R_f$ = 0,67 (AE) | c) analog Bsp.-Nr. XXXII u. XXXIII (aus Bsp.-Nr. XXVII) |
| 228 | rac | rac | 2-OH | $R_f$ = 0,32 (O) | c) analog Bsp.-Nr. XXXII u. XXXIII (aus Bsp.-Nr. XXXV) |
| 229 | rac | rac | 3-OH | $R_f$ = 0,40 (B) | c) analog Bsp.-Nr. XXXII u. XXXIII (aus Bsp.-Nr. XXXVI) |
| 230 | rac | rac | 3-$OSO_2Me$ | $R_f$ = 0,49 (B) | c) analog Bsp.-Nr. 90 (aus Bsp.-Nr. 229) |
| 231 | rac | dia B | 4-OH | $R_f$ = 0,51 (C) | c) analog Bsp.-Nr. XXXII u. XXXIII (aus Bsp.-Nr. XXXVII) |
| 232 | rac | dia A | 4-OH | $R_f$ = 0,60 (C) | c) analog Bsp.-Nr. XXXII u. XXXIII (aus Bsp.-Nr. XXXVII) |
| 233 | dia A | R | H | $R_f$ = 0,43 (C) | b) Aldrich |
| 234 | dia B | R | H | $R_f$ = 0,42 (C) | b) Aldrich |

Die Verbindungen folgender Tabelle 22 werden analog der Vorschrift von Beispiel-Nr. XXXII und XXXIII hergestellt:

**Tabelle 22**

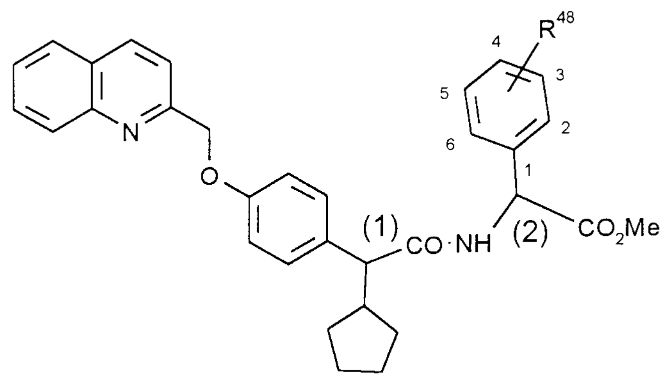

| Bsp.-Nr. | (1) | (2) | $R^{48}$ | $R_f$-Wert (Solvenz) | Ausgangsmaterial<br>a) Literatur<br>b) Vertreiberfirma<br>c) Herstellung aus Bsp.-Nr. |
|---|---|---|---|---|---|
| 235 | R | dia A | 4-cPr | $R_f$ = 0,24 (V) | c) III |
| 236 | R | dia B | 4-cPr | $R_f$ = 0,21 (V) | c) III |
| 237 | R | dia A | 4-tBu | $R_f$ = 0,25 (V) | a) WO 91 08 704 |
| 238 | R | dia B | 4-tBu | $R_f$ = 0,22 (V) | a) WO 91 08 704 |
| 239 | R | rac | 4-Ph | $R_f$ = 0,50 (X) | c) IV |
| 240 | R | R | 4-Cl | $R_f$ = 0,43 (AF) | c) XXXVIII |

| Bsp.-Nr. | (1) | (2) | $R^{48}$ | $R_f$-Wert (Solvenz) | Ausgangsmaterial<br>a) Literatur<br>b) Vertreiberfirma<br>c) Herstellung aus Bsp.-Nr. |
|---|---|---|---|---|---|
| 241 | R | S | 4-Cl | $R_f$ = 0,47 (X) | c) XXXVIII |
| 242 | R | dia A | 2-OH | $R_f$ = 0,36 (X) | c) II |
| 243 | R | dia B | 2-OH | $R_f$ = 0,28 (X) | c) II |
| 244 | R | rac | 3-OH | $R_f$ = 0,35 (X) | a) DE 22 04 117 |
| 245 | R | rac | 4-OH | $R_f$ = 0,23 (AF) | a) EP 530 879 |
| 246 | R | rac | 4-Me | $R_f$ = 0,53 (X) | a) F. Rose - Munch et al.,<br>J. Organomet. Chem. 415, 223 (1991). |
| 247 | R | rac | 4-$SO_2$Me | $R_f$ = 0,56 (AH) | c) XL |
| 248 | R | R | 4-OH | $R_f$ = 0,51 (AH) | a) EP 530 879 |
| 249 | R | rac | 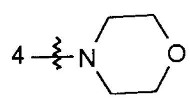 | $R_f$ = 0,23 (O) | c) VI |
| 250 | R | rac | 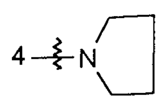 | $R_f$ = 0,22 (AF) | c) XXXIX |
| 251 | R | dia A | 3-$CF_3$ | $R_f$ = 0,25 (AF) | c) XLI |

| Bsp.-Nr. | (1) | (2) | $R^{48}$ | $R_f$-Wert (Solvenz) | Ausgangsmaterial<br>a) Literatur<br>b) Vertreiberfirma<br>c) Herstellung aus Bsp.-Nr. |
|---|---|---|---|---|---|
| 252 | R | dia B | 3-$CF_3$ | $R_f$ = 0,21 (AF) | c) XLI |
| 253 | R | dia A | 4-F, 3-OPh | $R_f$ = 0,26 (AF) | c) XLII |
| 254 | R | dia B | 4-F, 3-OPh | $R_f$ = 0,23 (AF) | c) XLII |
| 255 | R | S | H | $R_f$ = 0,55 (X) | b) Aldrich |
| 256 | S | S | H | $R_f$ = 0,53 (X) | b) Aldrich |
| 257 | R | dia A | 4-$OCF_3$ | $R_f$ = 0,33 (AF) | c) XLIII |
| 258 | R | dia B | 4-$OCF_3$ | $R_f$ = 0,28 (AF) | c) XLIII |
| 259 | R | R | H | $R_f$ = 0,50 (X) | b) Aldrich |
| 260 | R | rac | 3,4-$(OH)_2$ | $R_f$ = 0,29 (A) | c) XLIV |

Die Verbindungen folgender Tabelle 23 werden analog der Vorschrift von Beispiel-Nr. XXXII und XXXIII hergestellt:

**Tabelle 23**

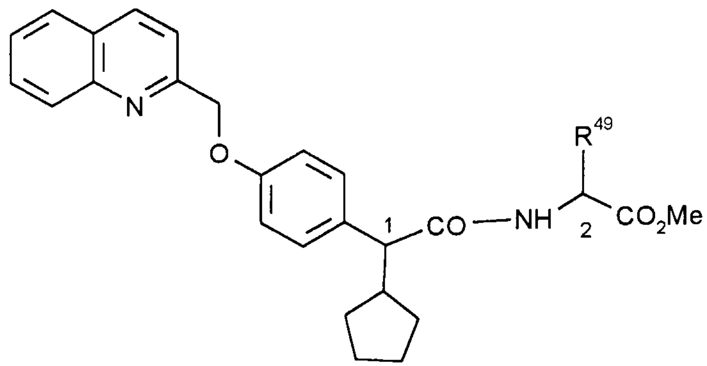

| Bsp.-Nr. | 1 | 2 | 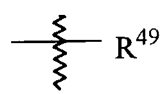 | $R_f$-Wert (Solvenz) | Ausgangsmaterial<br>a) Literatur<br>b) Vertreiberfirma<br>c) Herstellung aus Bsp.-Nr. |
|---|---|---|---|---|---|
| 261 | R | rac | 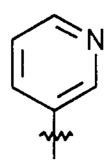 | $R_f$ = 0,31 (AE) | c) IX |

| Bsp.-Nr. | 1 | 2 | 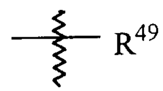 | $R_f$-Wert (Solvenz) | Ausgangsmaterial<br>a) Literatur<br>b) Vertreiberfirma<br>c) Herstellung aus Bsp.-Nr. |
|---|---|---|---|---|---|
| 262 | R | rac | 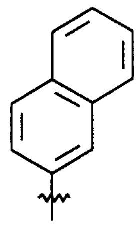 | $R_f$ = 0,66 (X) | c) V |
| 263 | R | rac | 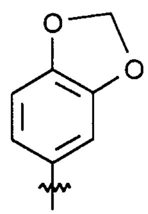 | $R_f$ = 0,43 (X) | a) G.H. Hakimelahi und G. Just, Can. J. Chem. 57, 1932 (1979). |
| 264 | S | rac | 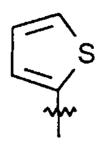 | $R_f$ = 0,38 (X) | a) DE 22 04 117 |

| Bsp.-Nr. | 1 | 2 | 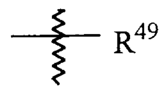 | $R_f$-Wert (Solvenz) | Ausgangsmaterial<br>a) Literatur<br>b) Vertreiberfirma<br>c) Herstellung aus Bsp.-Nr. |
|---|---|---|---|---|---|
| 265 | R | rac | 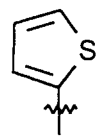 | $R_f$ = 0,26 (AF) | a) DE 22 04 117 |
| 266 | S | S | 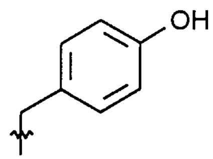 | $R_f$ = 0,34 (D) | b) Aldrich |

Die Verbindungen folgender Tabelle 24 werden analog der Vorschrift von Beispiel-Nr. XXXII, XXXIII und 21 hergestellt:

**Tabelle 24**

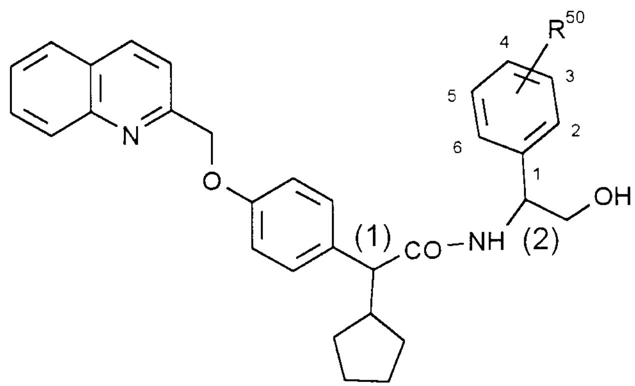

| Bsp.-Nr. | (1) | (2) | $R^{50}$ | $R_f$-Wert (Solvenz) | Ausgangsmaterial<br>a) Literatur<br>b) Vertreiberfirma<br>c) Herstellung analog Bsp.-Nr. |
|---|---|---|---|---|---|
| 267 | R | R | 4-Cl | $R_f$ = 0,35 (O) | c) analog Bsp.-Nr. 21<br>(aus Bsp.-Nr. 240) |
| 268 | R | rac | 4-Ph | $R_f$ = 0,22 (O) | c) analog Bsp.-Nr. 21<br>(aus Bsp.-Nr. 239) |
| 269 | R | S | 4-Cl | $R_f$ = 0,33 (O) | c) analog Bsp.-Nr. 21<br>(aus Bsp.-Nr. 241) |

| Bsp.-Nr. | (1) | (2) | $R^{50}$ | $R_f$-Wert (Solvenz) | Ausgangsmaterial<br>a) Literatur<br>b) Vertreiberfirma<br>c) Herstellung analog Bsp.-Nr. |
|---|---|---|---|---|---|
| 270 | R | dia B | 2-OH | $R_f$ = 0,28 (AH) | c) analog Bsp.-Nr. 21 (aus Bsp.-Nr. 243) |
| 271 | R | dia A | 2-OH | $R_f$ = 0,29 (AH) | c) analog Bsp.-Nr. 21 (aus Bsp.-Nr. 242) |
| 272 | R | dia B | 4-cPr | $R_f$ = 0,26 (O) | c) analog Bsp.-Nr. 21 (aus Bsp.-Nr. 236 ) |
| 273 | R | dia A | 4-cPr | $R_f$ = 0,26 (O) | c) analog Bsp.-Nr. 21 (aus Bsp.-Nr. 235) |
| 274 | R | rac | 4-OH | $R_f$ = 0,24 (AE) | c) analog Bsp.-Nr. 21 (aus Bsp.-Nr. 245) |
| 275 | R | dia A | 4-tBu | $R_f$ = 0,35 (O) | c) analog Bsp.-Nr. 21 (aus Bsp.-Nr. 237) |
| 276 | R | dia B | 4-tBu | $R_f$ = 0,32 (O) | c) analog Bsp.-Nr. 21 (aus Bsp.-Nr. 238) |
| 277 | R | R | 4-OH | $R_f$ = 0,46 (AI) | c) analog Bsp.-Nr. 21 (aus Bsp.-Nr. 248) |

| Bsp.-Nr. | (1) | (2) | $R^{50}$ | $R_f$-Wert (Solvenz) | Ausgangsmaterial<br>a) Literatur<br>b) Vertreiberfirma<br>c) Herstellung analog Bsp.-Nr. |
|---|---|---|---|---|---|
| 278 | R | rac | 4-$SO_2$Me | $R_f$ = 0,31 (AI) | c) analog Bsp.-Nr. 21<br>(aus Bsp.-Nr. 247) |
| 279 | R | rac | 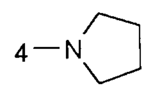 | $R_f$ = 0,33 (O) | c) analog Bsp.-Nr. 21<br>(aus Bsp.-Nr. 250) |
| 280 | S | dia B | 2-OH | $R_f$ = 0,50 (AH) | c) analog Bsp.-Nr. XXXII und XXXIII<br>(aus Bsp.-Nr. XXXV) |
| 281 | S | S | 4-Cl | $R_f$ = 0,21 (O) | c) analog Bsp.-Nr. XXXII und XXXIII |
| 282 | S | dia A | 2-OH | $R_f$ = 0,37 (AH) | c) analog Bsp.-Nr. XXXII und XXXIII<br>(aus Bsp.-Nr. XXXV) |
| 283 | R | dia A | 2-OH, 3-J | $R_f$ = 0,30 (O) | c) analog Bsp.-Nr. XXXII und XXXIII |
| 284 | R | dia B | 2-OH, 3-J | $R_f$ = 0,29 (O) | c) analog Bsp.-Nr. XXXII und XXXIII |
| 285 | S | R | 4-Cl | $R_f$ = 0,23 (O) | c) analog Bsp.-Nr. XXXII und XXXIII |
| 286 | S | rac | 4-F | $R_f$ = 0,34 (O) | c) analog Bsp.-Nr. XXXII und XXXIII<br>(aus Bsp.-Nr. XVIII) |
| 287 | S | rac | 3-OH | $R_f$ = 0,25 (AH) | c) analog Bsp.-Nr. XXXII und XXXIII<br>(aus Bsp.-Nr. XXXVI) |

| Bsp.-Nr. | (1) | (2) | $R^{50}$ | $R_f$-Wert (Solvenz) | Ausgangsmaterial<br>a) Literatur<br>b) Vertreiberfirma<br>c) Herstellung analog Bsp.-Nr. |
|---|---|---|---|---|---|
| 288 | S | rac | 2-F | $R_f$ = 0,37 (O) | c) analog Bsp.-Nr. XXXII und XXXIII (aus Bsp.-Nr. XVII) |
| 289 | R | rac | 2-F | $R_f$ = 0,39 (O) | c) analog Bsp.-Nr. XXXII und XXXIII (aus Bsp.-Nr. XVII) |
| 290 | R | rac | 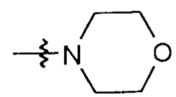 | $R_f$ = 0,13 (AE) | c) analog Bsp.-Nr. 21 (aus Bsp.-Nr. 249) |
| 291 | R | dia B | 3-$CF_3$ | $R_f$ = 0,39 (O) | c) analog Bsp.-Nr. 21 (aus Bsp.-Nr.251) |
| 292 | R | dia A | 3-$CF_3$ | $R_f$ = 0,28 (O) | c) analog Bsp.-Nr. 21 (aus Bsp.-Nr. 251) |
| 293 | R | dia A | 4-$OCF_3$ | $R_f$ = 0,13 (X) | c) analog Bsp.-Nr. 21 (aus Bsp.-Nr. 257) |
| 294 | R | dia B | 4-$OCF_3$ | $R_f$ = 0,29 (O) | c) analog Bsp.-Nr. 21 (aus Bsp.-Nr. 258) |
| 295 | R | dia B | 4-F, 3-OPh | $R_f$ = 0,13 (O) | c) analog Bsp.-Nr. 21 (aus Bsp.-Nr. 254) |

| Bsp.-Nr. | (1) | (2) | $R^{50}$ | $R_f$-Wert (Solvenz) | Ausgangsmaterial<br>a) Literatur<br>b) Vertreiberfirma<br>c) Herstellung analog Bsp.-Nr. |
|---|---|---|---|---|---|
| 296 | R | dia A | 4-F, 3-OPh | $R_f$ = 0,22 (O) | c) analog Bsp.-Nr. 21<br>(aus Bsp.-Nr. 253) |
| 297 | R | rac | 3-OH | $R_f$ = 0,26 (AE) | c) analog Bsp.-Nr. XXXII und XXXIII<br>(aus Bsp.-Nr. XXXVI) |
| 298 | R | rac | 4-Me | $R_f$ = 0,53 (O) | c) analog Bsp.-Nr. 21<br>(aus Bsp.-Nr. 246) |
| 299 | R | rac | 4-$CH_2$OH | $R_f$ = 0,27 (AE) | c) analog Bsp.-Nr. XXXII und XXXIII |

Die Verbindungen folgender Tabelle 25 werden analog der Vorschrift von Beispiel-Nr. 21 hergestellt:

**Tabelle 25**

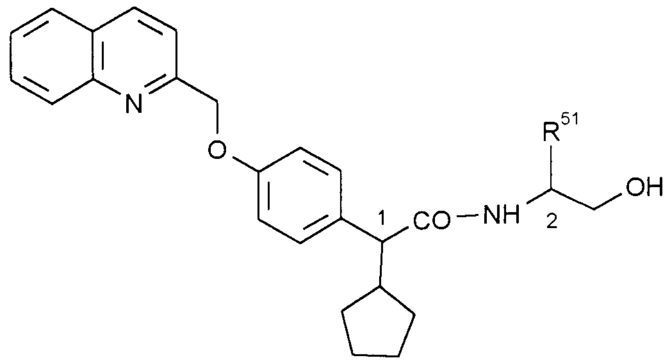

| Bsp.-Nr. | 1 | 2 | 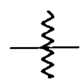 $R^{51}$ | $R_f$-Wert (Solvenz) | Ausgangsmaterial Herstellung aus Bsp.-Nr. |
|---|---|---|---|---|---|
| 300 | R | rac | 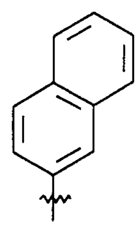 | $R_f$ = 0,20 (O) | 262 |

| Bsp.-Nr. | 1 | 2 | 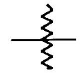 $R^{51}$ | $R_f$-Wert (Solvenz) | Ausgangsmaterial Herstellung aus Bsp.-Nr. |
|---|---|---|---|---|---|
| 301 | R | rac | 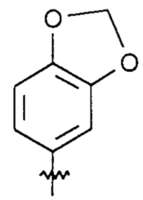 | $R_f$ = 0,32 (AH) | 263 |
| 302 | R | rac | 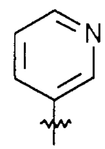 | $R_f$ = 0,06 (W) | 261 |
| 303 | R | S | 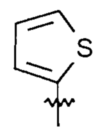 | $R_f$ = 0,40 (O) | 265 |
| 304 | S | rac | 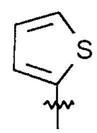 | $R_f$ = 0,28 / 0,38 (O) | 264 |

| Bsp.-Nr. | 1 | 2 | 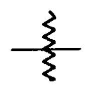 $R^{51}$ | $R_f$-Wert (Solvenz) | Ausgangsmaterial Herstellung aus Bsp.-Nr. |
|---|---|---|---|---|---|
| 305 | S | S | 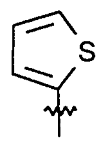 | $R_f$ = 0,36 (O) | 264 |
| 306 | R | R | 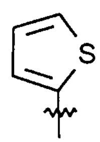 | $R_f$ = 0,32 (O) | 265 |

Die Verbindungen folgender Tabelle 26 werden analog der Vorschrift von Beispiel-Nr. XXXII, XXXIII und 21 hergestellt:

**Tabelle 26**

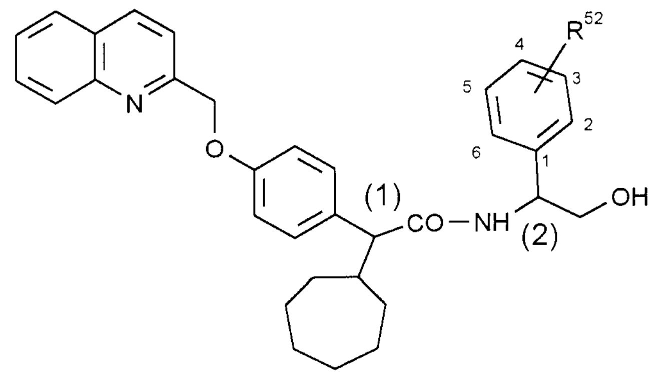

| Bsp.-Nr. | (1) | (2) | $R^{52}$ | $R_f$-Wert (Solvenz) Fließpunkt [°C] | Ausgangsmaterial<br>a) Literatur<br>b) Vertreiberfirma<br>c) Herstellung analog Bsp.-Nr. |
|---|---|---|---|---|---|
| 307 | R | dia A | 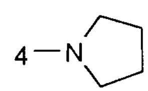 | $R_f$ = 0,26 (O) | c) analog Bsp.-Nr. XXXII und XXXIII |
| 308 | R | dia B | 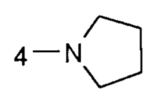 | $R_f$ = 0,21 (O) | c) analog Bsp.-Nr. XXXII und XXXIII |
| 309 | R | dia A | 4-$OCF_3$ | $R_f$ = 0,33 (O) | c) analog Bsp.-Nr. XXXII und XXXIII |

| Bsp.-Nr. | (1) | (2) | $R^{52}$ | $R_f$-Wert (Solvenz) Fließpunkt [°C] | Ausgangsmaterial a) Literatur b) Vertreiberfirma c) Herstellung analog Bsp.-Nr. |
|---|---|---|---|---|---|
| 310 | R | dia B | 4-$OCF_3$ | $R_f$ = 0,26 (O) | c) analog Bsp.-Nr. XXXII und XXXIII |
| 311 | R | dia A | 2-OH | $R_f$ = 0,39 (O) | c) analog Bsp.-Nr. XXXII und XXXIII (aus Bsp.-Nr. XXXV) |
| 312 | R | dia B | 2-OH | $R_f$ = 0,26 (O) | c) analog Bsp.-Nr. XXXII und XXXIII (aus Bsp.-Nr. XXXV) |
| 313 | R | S | 4-Cl | $R_f$ = 0,32 (O) | c) analog Bsp.-Nr. 21 |
| 314 | R | dia A | 4-Ph | $R_f$ = 0,40 (O) | c) analog Bsp.-Nr. XXXII und XXXIII (aus Bsp.-Nr. IV) |
| 315 | R | dia B | 4-Ph | $R_f$ = 0,35 (O) | c) analog Bsp.-Nr. XXXII und XXXIII (aus Bsp.-Nr. IV) |
| 316 | R | dia A | 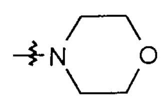 | $R_f$ = 0,09 (AH) | c) analog Bsp.-Nr. XXXII und XXXIII (aus Bsp.-Nr. VI) |
| 317 | R | dia B | 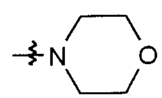 | $R_f$ = 0,09 (AH) | c) analog Bsp.-Nr. XXXII und XXXIII (aus Bsp.-Nr. VI) |
| 318 | R | dia A | 3-$CF_3$ | $R_f$ = 0,48 (O) | c) analog Bsp.-Nr. XXXII und XXXIII |

| Bsp.-Nr. | (1) | (2) | $R^{52}$ | $R_f$-Wert (Solvenz) Fließpunkt [°C] | Ausgangsmaterial a) Literatur b) Vertreiberfirma c) Herstellung analog Bsp.-Nr. |
|---|---|---|---|---|---|
| 319 | R | rac | 3-OH | $R_f$ = 0,21 (AH) | c) analog Bsp.-Nr. XXXII und XXXIII (aus Bsp.-Nr. XXXVI) |
| 320 | R | dia B | 3-$CF_3$ | $R_f$ = 0,41 (O) | c) analog Bsp.-Nr. XXXII und XXXIII |
| 321 | R | rac | 4-OH | $R_f$ = 0,21 (AH) | c) analog Bsp.-Nr. XXXII und XXXIII (aus Bsp.-Nr. XXXVII) |

Die Verbindungen folgender Tabelle 27 werden analog der Vorschrift von Beispiel-Nr. XXXII und XXXIII hergestellt:

**Tabelle 27**

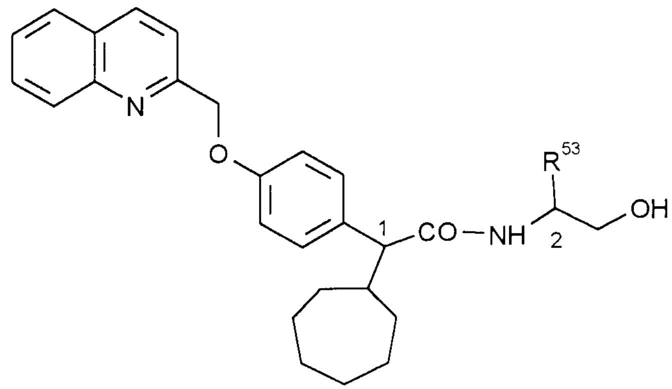

| Bsp.-Nr. | 1 | 2 | $R^{53}$ | $R_f$-Wert (Solvenz) | Ausgangsmaterial Herstellung aus Bsp.-Nr. |
|---|---|---|---|---|---|
| 322 | R | dia B | 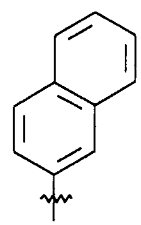 | $R_f$ = 0,36 (O) | V |
| 323 | R | dia A | 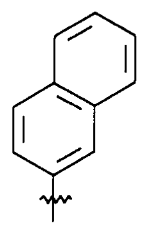 | $R_f$ = 0,40 (O) | V |

Die Verbindungen folgender Tabelle 28 werden analog der Vorschrift von Beispiel-Nr. 74 hergestellt:

**Tabelle 28**

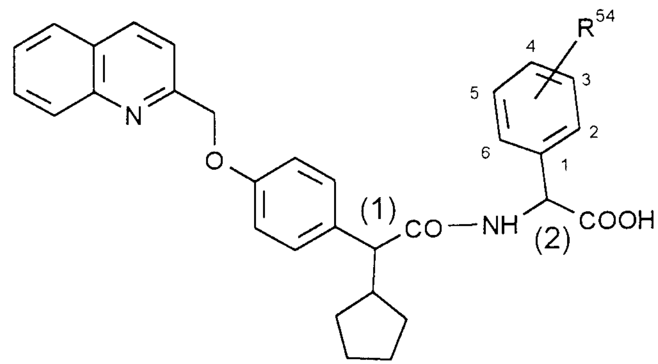

| Bsp.-Nr. | (1) | (2) | $R^{54}$ | $R_f$-Wert (Solvenz) | Ausgangsmaterial Herstellung aus Bsp.-Nr. |
|---|---|---|---|---|---|
| 324 | R | dia A | 3-$CF_3$ | $R_f$ = 0,02 (AH) | 251 |
| 325 | R | dia B | 3-$CF_3$ | $R_f$ = 0,02 (AH) | 252 |
| 326 | R | dia A | 4-$OCF_3$ | $R_f$ = 0,02 (AH) | 257 |
| 327 | R | dia B | 4-$OCF_3$ | $R_f$ = 0,02 (AH) | 258 |
| 328 | R | dia B | 4-F, 3-OPh | $R_f$ = 0,02 (AH) | 254 |
| 329 | R | S | H | $R_f$ = 0,01 (AH) | 255 |
| 330 | R | R | H | $R_f$ = 0,01 (AH) | 259 |

| Bsp.-Nr. | (1) | (2) | $R^{54}$ | $R_f$-Wert (Solvenz) | Ausgangsmaterial Herstellung aus Bsp.-Nr. |
|---|---|---|---|---|---|
| 331 | S | S | H | $R_f$ = 0,01 (AH) | 256 |
| 332 | R | dia A | 4-F, 3-OPh | $R_f$ = 0,02 (AH) | 253 |

Die Verbindungen folgender Tabelle 29 werden analog der Vorschrift von Beispiel-Nr. 76 hergestellt:

**Tabelle 29**

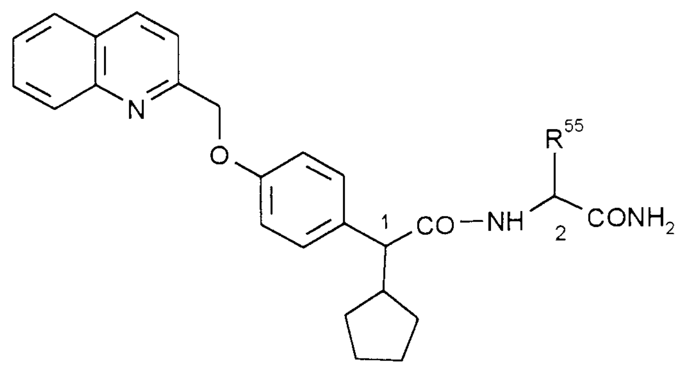

| Bsp.-Nr. | 1 | 2 | $R^{55}$ | $R_f$-Wert (Solvenz) | Ausgangsmaterial Herstellung aus Bsp.-Nr. |
|---|---|---|---|---|---|
| 333 | R | rac | 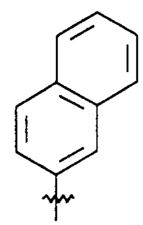 | $R_f$ = 0,69 (AJ) | 262 |

| Bsp.-Nr. | 1 | 2 | $R^{55}$ | $R_f$-Wert (Solvenz) | Ausgangsmaterial Herstellung aus Bsp.-Nr. |
|---|---|---|---|---|---|
| 334 | R | rac | 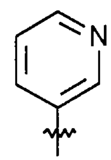 | $R_f$ = 0,55 (AE) | 261 |
| 335 | rac | rac | 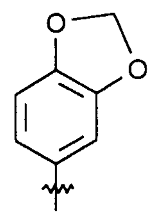 | $R_f$ = 0,31 (C) | 263 |
| 336 | S | rac | 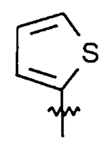 | $R_f$ = 0,56 (C) | 264 |
| 337 | R | rac | 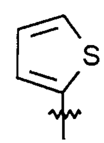 | $R_f$ = 0,53 (C) | 265 |

Die Verbindungen folgender Tabelle 30 werden analog der Vorschrift von Beispiel-Nr. XXXII und XXXIII hergestellt:

**Tabelle 30**

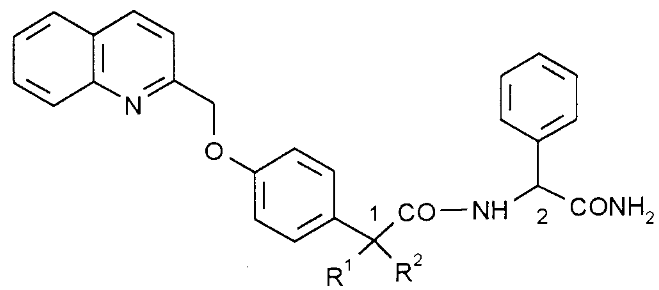

| Bsp.-Nr. | 1 | 2 | $R^1$ | $R^2$ | $R_f$-Wert (Solvenz) |
|---|---|---|---|---|---|
| 338 | rac | S | OH | cHex | $R_f$ = 0,52 (AE) |
| 339 | R | S | H | cPent | $R_f$ = 0,61 (AJ) |
| 340 | S | S | H | cPent | $R_f$ = 0,48 (AJ) |
| 341 | R | R | H | cPent | $R_f$ = 0,38 (Z) |
| 342 | rac | rac | OH | 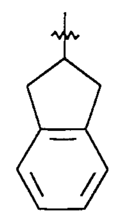 | $R_f$ = 0,48 (AE) |

| Bsp.-Nr. | 1 | 2 | $R^1$ | $R^2$ | $R_f$-Wert (Solvenz) |
|---|---|---|---|---|---|
| 343 | rac | rac | H | cDodec | $R_f$ = 0,34 (F) |
| 344 | rac | rac | H | cOct | $R_f$ = 0,30 (F) |

D,L-Phenylglycinamid ist bei Bader käuflich.

Die Verbindungen folgender Tabelle 31 werden analog der Vorschrift von Beispiel-Nr. 76 hergestellt:

**Tabelle 31**

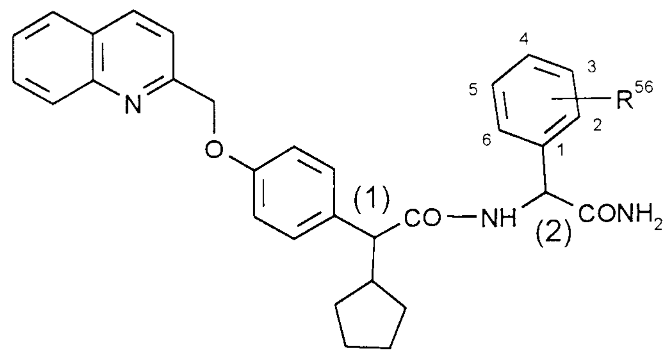

| Bsp.-Nr. | (1) | (2) | $R^{56}$ | $R_f$-Wert (Solvenz) | Ausgangsmaterial Herstellung aus Bsp.-Nr. |
|---|---|---|---|---|---|
| 345 | R | rac | 4-Cl | $R_f$ = 0,47 (AD) | 240 |
| 346 | R | S | 4-Cl | $R_f$ = 0,48 (AE) | 241 |
| 347 | S | S | 4-Cl | $R_f$ = 0,61 (AJ) | 240/241 |
| 348 | S | R | 4-Cl | $R_f$ = 0,45 (AD) | 240/241 |
| 349 | R | rac | 4-$OCF_3$ | $R_f$ = 0,35 (Z) | 257 |
| 350 | R | rac | 4-Ph | $R_f$ = 0,51 (AJ) | 239 |
| 351 | R | rac | 4-cPr | $R_f$ = 0,48 (AJ) | 235 |

| Bsp.-Nr. | (1) | (2) | $R^{56}$ | $R_f$-Wert (Solvenz) | Ausgangsmaterial Herstellung aus Bsp.-Nr. |
|---|---|---|---|---|---|
| 352 | R | rac | 4-cPr | $R_f$ = 0,47 (AJ) | 236 |
| 353 | R | rac | 4-tBu | $R_f$ = 0,55/0,50 (Z) | 237/238 |
| 354 | R | rac | 4-$OCF_3$ | $R_f$ = 0,28 (AJ) | 258 |
| 355 | R | rac | 4- 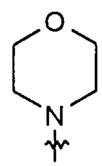 | $R_f$ = 0,33 (AE) | 249 |
| 356 | rac | rac | 2-OH | $R_f$ = 0,38 (AG) | 242/243 |
| 357 | rac | rac | 4-OH | $R_f$ = 0,30 (C) | 245 |
| 358 | rac dia A | | 4-OH | $R_f$ = 0,27 (C) | 245 |
| 359 | rac | rac | 3-OH | $R_f$ = 0,31 (C) | 244 |
| 360 | rac | rac | 4-F, 3-OPh | $R_f$ = 0,52 (C) | 253/254 |
| 361 | rac | rac | 4-$SO_2$Ph | $R_f$ = 0,47 (C) | 247 |
| 362 | R | rac | 3-$CF_3$ | $R_f$ = 0,33 (F) | 251/252 |

Die Verbindungen folgender Tabelle 32 werden analog der Vorschrift von Beispiel-Nr. XXXII, XXXIII und 76 hergestellt:

**Tabelle 32**

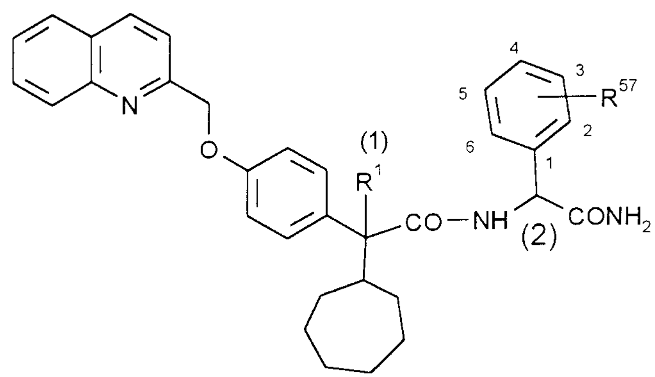

| Bsp.-Nr. | (1) | (2) | $R^1$ | $R^{57}$ | $R_f$-Wert (Solvenz) | Ausgangsmaterial Herstellung aus Bsp.-Nr. |
|---|---|---|---|---|---|---|
| 363 | rac | dia A | H | H | $R_f$ = 0,19 (O) | analog Bsp.-Nr. XXXII und XXXIII * |
| 364 | rac | dia B | H | H | $R_f$ = 0,19 (O) | analog Bsp.-Nr. XXXII und XXXIII * |

| Bsp.-Nr. | (1) | (2) | $R^1$ | $R^{57}$ | $R_f$-Wert (Solvenz) | Ausgangsmaterial Herstellung aus Bsp.-Nr. |
|---|---|---|---|---|---|---|
| 365 | R | rac | H | 4- 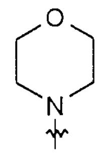 | $R_f$ = 0,30 (Z) | analog Bsp.-Nr. 76 |
| 366 | rac dia A | | OH | H | $R_f$ = 0,16 (O) | analog Bsp.-Nr. XXXII und XXXIII * |
| 367 | rac dia B | | OH | H | $R_f$ = 0,16 (O) | analog Bsp.-Nr. XXXII und XXXIII * |
| 368 | rac | rac | H | 2-OH | $R_f$ = 0,14 (O) | analog Bsp.-Nr. 76 |
| 369 | rac | rac | H | 3-OH | $R_f$ = 0,30 (AG) | analog Bsp.-Nr. 76 |
| 370 | rac | rac | H | 4- 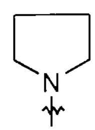 | $R_f$ = 0,28 (O) | analog Bsp.-Nr. 76 |
| 371 | rac dia A | | H | 4- 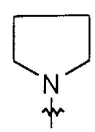 | $R_f$ = 0,28 (O) | analog Bsp.-Nr. 76 |

| Bsp.-Nr. | (1) | (2) | $R^1$ | $R^{57}$ | $R_f$-Wert (Solvenz) | Ausgangsmaterial Herstellung aus Bsp.-Nr. |
|---|---|---|---|---|---|---|
| 372 | R | dia A | H | H | $R_f$ = 0,17 (O) | analog Bsp.-Nr. XXXII und XXXIII * |
| 373 | R | dia B | H | H | $R_f$ = 0,17 (O) | analog Bsp.-Nr. XXXII und XXXIII * |
| 374 | S | dia A | H | H | $R_f$ = 0,26 (O) | analog Bsp.-Nr. XXXII und XXXIII * |
| 375 | S | dia B | H | H | $R_f$ = 0,26 (O) | analog Bsp.-Nr. XXXII und XXXIII * |

* D,L-Phenylglycinamid ist bei Bader käuflich.

Die Verbindungen folgender Tabelle 33 werden analog der Vorschrift von Beispiel-Nr. XXXII und XXXIII hergestellt:

**Tabelle 33**

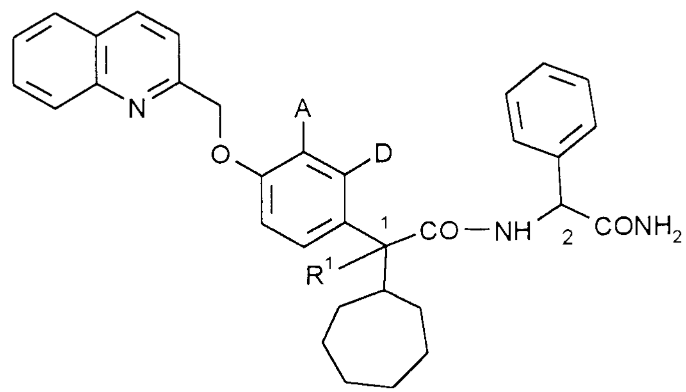

| Bsp.-Nr. | 1 | 2 | A | D | $R^1$ | $R_f$-Wert (Solvenz) |
|---|---|---|---|---|---|---|
| 376 | rac | rac | H | $-CH_2=CH_2$ | H | $R_f$ = 0,56 (AE) |
| 377 | rac | rac | H | iBu | H | $R_f$ = 0,67/0,61 (AE) |
| 378 | rac | rac | iBu | H | H | $R_f$ = 0,35 (F) |
| 379 | rac | rac | 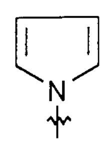 | H | H | $R_f$ = 0,33 (F) |

| Bsp.-Nr. | 1 | 2 | A | D | $R^1$ | $R_f$-Wert (Solvenz) |
|---|---|---|---|---|---|---|
| 380 | rac | rac | OMe | H | H | $R_f$ = 0,28 (F) |
| 381 | rac | rac | iBu | H | OH | $R_f$ = 0,30 (F) |
| 382 | rac | rac | F | H | H | $R_f$ = 0,30 (F) |

D,L-Phenylglycinamid ist bei Bader käuflich.

Die Verbindungen der Tabelle 34 werden analog der Vorschrift von Beispiel-Nr. XXXII und XXXIII hergestellt:

**Tabelle 34**

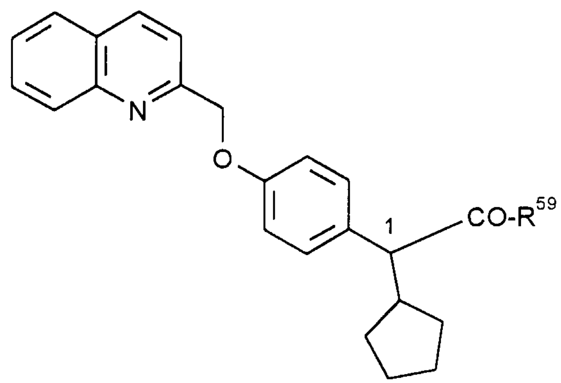

| Bsp.-Nr. | 1 | $-R^{59}$ | $R_f$-Wert (Solvenz)<br>Fp. [°C] | Ausgangsmaterial<br>a) Literatur<br>b) Vertreiberfirma<br>c) Syn. analog/aus Bsp.-Nr. |
|---|---|---|---|---|
| 383 | S | 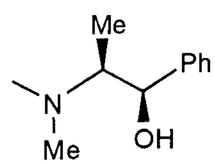 | $R_f$ = 0,49 (AK) | b) Aldrich |

| Bsp.-Nr. | 1 | $-R^{59}$ | $R_f$-Wert (Solvenz)<br>Fp. [°C] | Ausgangsmaterial<br>a) Literatur<br>b) Vertreiberfirma<br>c) Syn. analog/aus Bsp.-Nr. |
|---|---|---|---|---|
| 384 | R | 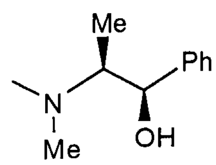 | Fp. = 109°C | b) Aldrich |
| 385 | S | 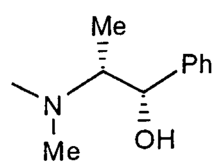 | $R_f$ = 0,49 (AK) | b) Aldrich |
| 386 | S | 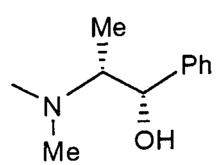 | $R_f$ = 0,48 (AK) | b) Aldrich |
| 387 | S | 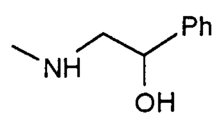 | Fp. = 173°C | b) Aldrich |

| Bsp.-Nr. | 1 | -$R^{59}$ | $R_f$-Wert (Solvenz)<br>Fp. [°C] | Ausgangsmaterial<br>a) Literatur<br>b) Vertreiberfirma<br>c) Syn. analog/aus Bsp.-Nr. |
|---|---|---|---|---|
| 388 | R | 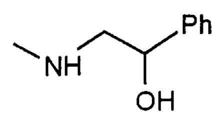 | Fp. = 161°C | b) Aldrich |
| 389 | R | 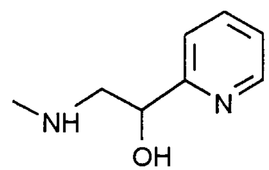 | $R_f$ = 0,43 (AL) | a) F. Zymalkowski Arch. Pharm. 291, 12 (1958). |
| 390 | S | 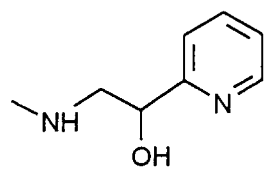 | $R_f$ = 0,42 (AL) | a) F. Zymalkowski Arch. Pharm. 291, 12 (1958). |
| 391 | R | 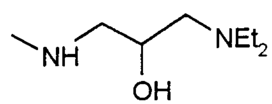 | $R_f$ = 0,39 (B) | b) K & K |

| Bsp.-Nr. | 1 | $-R^{59}$ | $R_f$-Wert (Solvenz)<br>Fp. [°C] | Ausgangsmaterial<br>a) Literatur<br>b) Vertreiberfirma<br>c) Syn. analog/aus Bsp.-Nr. |
|---|---|---|---|---|
| 392 | S | 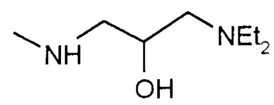 | $R_f$ = 0,39 (B) | b) K & K |
| 393 | S | 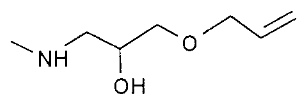 | Fp. = 118°C | b) K & K |
| 394 | R | 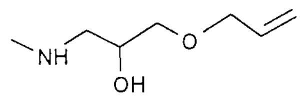 | Fp. = 120°C | b) K & K |
| 395 | S | 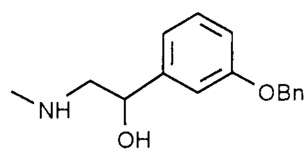 | $R_f$ = 0,53 (AK) | a) J. Millen et al., J. Med. Chem. 28, 12 (1985). |
| 396 | R | 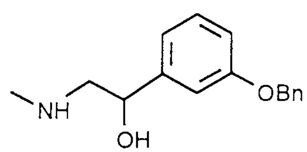 | $R_f$ = 0,53 (AK) | a) J. Millen et al., J. Med. Chem. 28, 12 (1985). |

| Bsp.-Nr. | 1 | $-R^{59}$ | $R_f$-Wert (Solvenz) Fp. [°C] | Ausgangsmaterial a) Literatur b) Vertreiberfirma c) Syn. analog/aus Bsp.-Nr. |
|---|---|---|---|---|
| 397 | S | 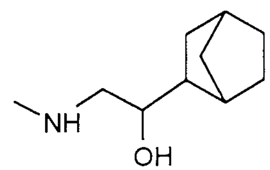 | $R_f$ = 0,58 (AK) | a) D.P. Davis et al., J. Med. Chem. 24, 12 (1981). |
| 398 | R | 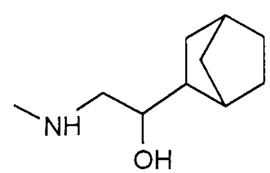 | $R_f$ = 0,57 (AK) | a) D.P. Davis et al., J. Med. Chem. 24, 12 (1981). |
| 399 | S | 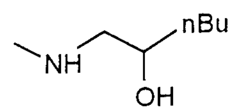 | $R_f$ = 0,60 (AK) | a) EP 518 672 |
| 400 | R | 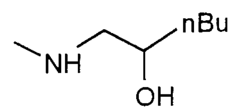 | $R_f$ = 0,60 (AK) | a) EP 518 672 |

**Patentansprüche**

1. 4-(Chinolin-2-yl-methoxy)-phenyl-essigsäurederivate der allgemeinen Formel (I)

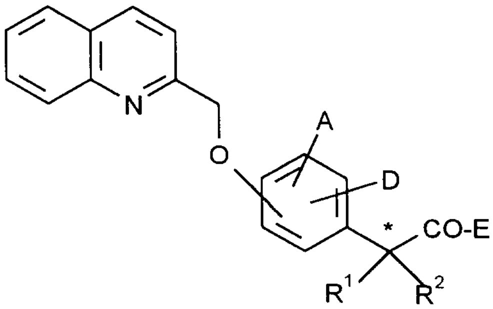

(I)

in welcher

A und D gleich oder verschieden sind und für Wasserstoff, Cycloalkyl mit 3 bis 8 Kohlenstoffatomen, Azido, Hydroxy, Halogen, geradkettiges oder verzweigtes Alkyl, Alkoxy oder Alkenyl mit jeweils bis zu 6 Kohlenstoffatomen, einen 5- bis 6-gliedrigen ungesättigten oder gesättigten Heterocyclus mit bis zu 3 Heteroatomen aus der Reihe S, N und/oder O stehen,

$R^1$ für Wasserstoff, Hydroxy oder für geradkettiges oder verzweigtes Alkyl mit bis zu 4 Kohlenstoffatomen steht,

$R^2$ für Wasserstoff, Hydroxy, Halogen, geradkettiges oder verzweigtes Alkenyl oder Alkoxy mit jeweils bis zu 8 Kohlenstoffatomen oder Cycloalkyl mit 3 bis 14 Kohlenstoffatomen steht, oder für geradkettiges oder verzweigtes Alkyl mit bis zu 8 Kohlenstoffatomen steht, das gegebenenfalls durch Cycloalkyl mit 3 bis 14 Kohlenstoffatomen, Phenyl oder Tetrahydropyranyl substituiert ist, welche ihrerseits durch Halogen substituiert sein können, oder für den Indanylrest steht,

oder

$R^1$ und $R^2$ gemeinsam mit dem Kohlenstoffatom einen gesättigten carbocyclischen Ring mit 5 bis 7 Kohlenstoffatomen bilden,

oder

$R^1$ und $R^2$ gemeinsam für einen Doppelbindungs-Rest der Formel

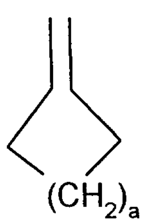

stehen,
worin

a eine Zahl 2, 3, 4, 5 oder 6 bedeutet,

E für einen Rest der Formel

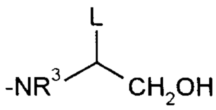 , 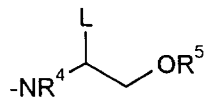 , 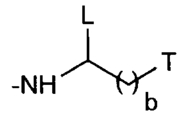 ,

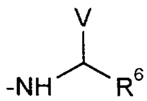 , 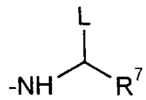 , 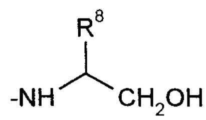 ,

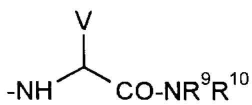 ,

-NR⁴'-W-OH,

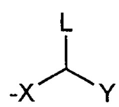 ,

$-NHR^{11}$, oder

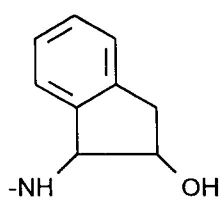

steht,

in welchen

$R^3$ Phenyl, Methyl oder eine typische Aminoschutzgruppe bedeutet,

$R^4$ und $R^{4'}$ gleich oder verschieden sind und die oben angegebene Bedeutung von $R^3$ haben oder Wasserstoff bedeuten,

$R^5$ geradkettiges oder verzweigtes Acyl oder Alkyl mit jeweils bis zu 4 Kohlenstoffatomen bedeutet,

L Phenyl, Benzyl oder Naphtyl bedeutet, das gegebenenfalls bis zu 2-fach gleich oder verschieden durch Halogen, Hydroxy, Pyrrolidinyl, Morpholino, Amino, Trifluormethyl, Trifluormethoxy, Cycloalkyl mit 3 bis 6 Kohlenstoffatomen, geradkettiges oder verzweigtes Alkyl oder Alkoxy mit jeweils bis zu 6 Kohlenstoffatomen substituiert ist, die ihrerseits durch Hydroxy substituiert sein können, oder gegebenenfalls durch Phenyl oder Phenoxy substituiert sind, die ihrerseits bis zu 2-fach gleich oder verschieden durch Halogen oder durch geradkettiges oder verzweigtes Alkyl mit bis zu 4 Kohlenstoffatomen substituiert sein können, und/oder

gegebenenfalls durch eine Gruppe der Formel - $(O)_c$-$SO_2R^{12}$ substituiert sind,
worin

c eine Zahl 0 oder 1 bedeutet und

$R^{12}$ geradkettiges oder verzweigtes Alkyl mit bis zu 4 Kohlenstoffatomen oder Phenyl bedeutet,

b eine Zahl 0, 1 oder 2 bedeutet,

T einen 5- bis 7-gliedrigen, gegebenenfalls benzokondensierten, gesättigten, partiell ungesättigten oder ungesättigten Heterocyclus mit bis zu 3 Heteroatomen aus der Reihe S, N und/oder O bedeutet, wobei beide Cyclen gegebenenfalls bis zu 3-fach gleich oder verschieden durch Halogen, Hydroxy, Morpholino, Amino, Cycloalkyl mit 3 bis 6 Kohlenstoffatomen, geradkettiges oder verzweigtes Alkyl oder Alkoxy mit jeweils bis zu 6 Kohlenstoffatomen oder Phenyl substituiert sind,

V die oben angegebene Bedeutung von L oder T hat, oder
einen Rest der Formel

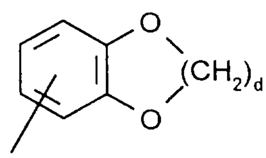

bedeutet,
worin

d eine Zahl 1, 2 oder 3 bedeutet,

$R^6$ einen Rest der Formel $-(CH_2)_e-R^{13}$ bedeutet,
worin

e eine Zahl 0 oder 1 bedeutet,

$R^{13}$ Hydroxy, Carboxy oder geradkettiges oder verzweigtes Alkoxycarbonyl mit bis zu 4 Kohlenstoffatomen bedeutet,

$R^7$ Wasserstoff, Cyano, Trifluormethyl, geradkettiges oder verzweigtes Alkenyl oder Alkyl mit bis 7 Kohlenstoffatomen bedeutet, das gegebenenfalls bis zu 2-fach gleich oder verschieden durch geradkettiges oder verzweigtes Alkoxy mit bis zu 4 Kohlenstoffatomen substituiert ist, oder Alkoxy mit bis zu 6 Kohlenstoffatomen bedeutet, oder
eine Gruppe der Formel $-CO-NH-(CH_2)_f-NR^{14}R^{15}$ bedeutet,
worin

f eine Zahl 1, 2 oder 3 bedeutet,

$R^{14}$ und $R^{15}$ gleich oder verschieden sind und
Wasserstoff, Phenyl oder geradkettiges oder verzweigtes Alkyl mit bis zu 4 Kohlenstoffatomen bedeutet,

$R^8$ Wasserstoff, Carboxy, geradkettiges oder verzweigtes Alkoxycarbonyl mit bis zu 4 Kohlenstoffatomen oder geradkettiges oder verzweigtes Alkyl mit bis zu 5 Kohlenstoffatomen bedeutet, das gegebenenfalls durch Hydroxy, Carboxy oder durch geradkettiges oder verzweigtes Alkoxycarbonyl mit jeweils bis zu 4 Kohlenstoffatomen oder Phenyl substituiert ist,

$R^9$ und $R^{10}$ gleich oder verschieden sind und Wasserstoff, Cycloalkyl mit 3 bis 6 Kohlenstoffatomen oder Phenyl bedeuten, oder geradkettiges oder verzweigtes Alkyl mit bis zu 6 Kohlenstoffatomen bedeuten, das gegebenenfalls durch Phenyl, Cycloalkyl mit 3 bis 6 Kohlenstoffatomen, Hydroxy, Carboxy, durch geradkettiges oder verzweigtes Alkoxy oder Alkoxycarbonyl mit jeweils bis zu 4 Kohlenstoffatomen oder durch eine Gruppe der Formel $-NR^{16}R^{17}$ substituiert ist
worin

$R^{16}$ und $R^{17}$ gleich oder verschieden sind und Wasserstoff, Phenyl oder geradkettiges oder verzweigtes Alkyl mit bis zu 4 Kohlenstoffatomen bedeuten,

oder

$R^9$ und $R^{10}$ gemeinsam mit dem Stickstoffatom einen Heterocyclus der Formel

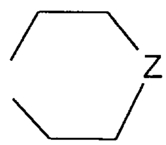

bilden,
worin

Z ein Sauerstoffatom oder die Gruppe $-NR^{18}$ oder -CH bedeutet,
worin

$R^{18}$ Wasserstoff, Acetyl, eine typische Aminoschutzgruppe oder einen Rest der Formel - $SO_2R^{19}$ bedeutet,
worin

$R^{19}$ geradkettiges oder verzweigtes Alkyl mit bis zu 4 Kohlenstoffatomen, Benzyl oder Phenyl bedeutet, das gegebenenfalls durch Phenyl oder Tolyl substiuiert ist,

W geradkettiges oder verzweigtes Alkyl von 2 bis 7 Kohlenstoffatomen bedeutet, das 1- bis 3-fach gleich oder verschieden durch Hydroxy, Pyridyl, Norbornyl oder Phenyl substituiert ist, das seinerseits durch Hydroxy oder Benzyloxy substituiert sein kann,
oder durch eine Gruppe der Formel $-OR^{20}$ oder $-NR^{21}R^{22}$ substituiert ist,
worin

$R^{20}$ geradkettiges oder verzweigtes Alkyl ist bis zu 6 Kohlenstoffatomen bedeutet, und

$R^{21}$ und $R^{22}$ gleich oder verschieden sind und die oben angegebene Bedeutung von $R^{16}$ und $R^{17}$ haben

X ein Sauerstoff- oder Schwefelatom bedeutet,

Y Formyl oder die Gruppe $-CHR^{23}R^{24}$ bedeutet,
worin

$R^{23}$ Wasserstoff bedeutet und

$R^{24}$ Hydroxy oder geradkettiges oder verzweigtes Alkoxy oder Acyl mit jeweils bis zu 4 Kohlenstoffatomen bedeutet,
oder

$R^{23}$ und $R^{24}$ gleich oder verschieden sind und geradkettiges oder verzweigtes Alkoxy mit bis zu 4 Kohlenstoffatomen bedeuten

$R^{11}$ einen Rest der Formel

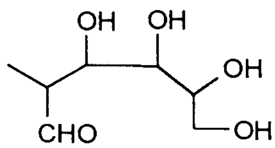 , 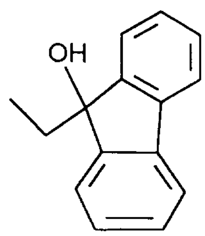 , 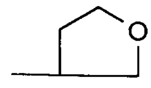

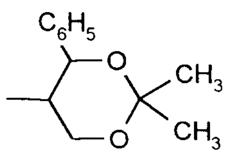 , 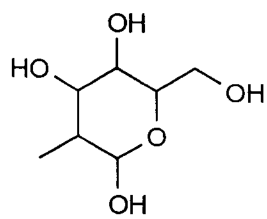

oder
Phenyl bedeutet, das gegebenenfalls bis zu 2-fach gleich oder verschieden durch Halogen, Carboxy oder geradkettiges oder verzweigtes Alkoxycarbonyl mit bis zu 5 Kohlenstoffatomen substituiert ist,
oder
geradkettiges oder verzweigtes Alkyl mit bis zu 6 Kohlenstoffatomen bedeutet, das gegebenenfalls bis zu 2-fach gleich oder verschieden durch Hydroxy, Carboxy, Halogen oder durch geradkettiges oder verzweigtes Alkoxycarbonyl mit bis zu 5 Kohlenstoffatomen substituiert ist,
und deren Salze,
wobei
2-[4-(Chinolin-2-yl-methoxy)phenyl]-2-cyclopentyl-essigsäuremethyloxycarbonylmethylamid,
2-[4-(Chinolin-2-yl-methoxy)phenyl]-2-cyclopentyl-essigsäurecarboxymethylamid,
N-Methyl-2-[3-isobutyl-4-(chinolin-2-yl-methoxy)phenyl]2-cycloheptylacetamid,
N-Methyl-2-[4-(chinolin-2-yl-methoxy)phenyl]-2-cycloheptyl-essigsäureamid,
N-Ethyl-2-[4-(chinolin-2-yl-methoxy)phenyl]-2-cyclopentyl-essigsäureamid
und
N-Ethyl-2-[4-(chinolin-2-yl-methoxy)phenyl]-2-cyclohexyl-essigsaureamid ausgenommen sind.

**2.** 4-(Chinolin-2-yl-methoxy)-phenyl-essigsäurederivate nach Anspruch 1,
worin

A und D gleich oder verschieden sind und
für Wasserstoff, Cyclopropyl, Cyclopentyl, Cyclohexyl, Azido, Hydroxy, Fluor, Chlor, Brom, geradkettiges oder verzweigtes Alkyl, Alkoxy oder Alkenyl mit jeweils bis zu 5 Kohlenstoffatomen, Pyrryl oder Imidazolyl stehen,

$R^1$ für Wasserstoff, Hydroxy oder für geradkettiges oder verzweigtes Alkyl mit bis zu 3 Kohlenstoffatomen steht,

$R^2$ für Wasserstoff, Hydroxy, Fluor, Chlor, Brom, geradkettiges oder verzweigtes Alkenyl oder Alkoxy mit jeweils bis zu 7 Kohlenstoffatomen, Cyclopentyl, Cyclohexyl, Cycloheptyl, Cyclooctyl, Cyclononyl, Cyclodecyl, Cycloundecyl oder Cyclododecyl steht, oder
für geradkettiges oder verzweigtes Alkyl mit bis zu 7 Kohlenstoffatomen steht, das gegebenenfalls durch Cyclopentyl, Cyclohexyl, Cycloheptyl, Cyclooctyl, Cyclononyl, Cyclodecyl, Cycloundecyl oder Cyclododecyl, Phenyl oder Tetrahydropyranyl substituiert ist, welche ihrerseits durch Fluor, Chlor oder Brom

substituiert sein können, oder
für den Indanylrest steht,

oder

$R^1$ und $R^2$ gemeinsam mit dem Kohlenstoffatom einen Cyclopentyl-, Cyclohexyl- oder Cycloheptylring bilden,

oder

$R^1$ und $R^2$ gemeinsam für einen Doppelbindungs-Rest der Formel

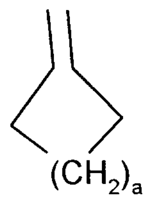

stehen,
worin

a eine Zahl 2, 3, 4 oder 5 bedeutet,

E für einen Rest der Formel

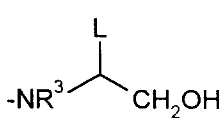 , 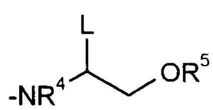 , 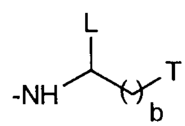 ,

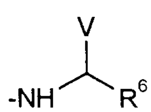 , 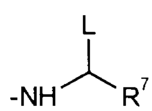 , 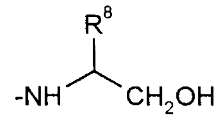 ,

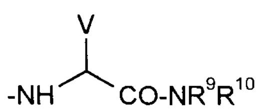 ,

$-NR^{4'}-W-OH$,

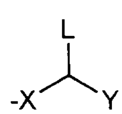 ,

$-NH-R^{11}$ oder

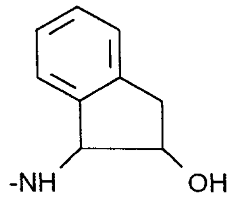

steht,
in welcher

| | |
|---|---|
| $R^3$ | Phenyl, Methyl, Acetyl oder tert. Butoxycarbonyl (Boc) bedeutet, |
| $R^4$ und $R^{4'}$ | gleich oder verschieden sind und die oben angegebene Bedeutung von $R^3$ haben oder Wasserstoff bedeuten, |
| $R^5$ | geradkettiges oder verzweigtes Acyl oder Alkyl mit jeweils bis zu 3 Kohlenstoffatomen bedeutet, |
| L | Phenyl, Benzyl oder Naphtyl bedeutet, die gegebenenfalls bis zu 2-fach gleich oder verschieden durch Fluor, Chlor, Brom, Iod, Hydroxy, Pyrrolidinyl, Morpholino, Amino, Trifluormethyl, Trifluormethoxy, Cyclopropyl, Cyclopentyl, Cyclohexyl, geradkettiges oder verzweigtes Alkyl oder Alkoxy mit jeweils bis zu 6 Kohlenstoffatomen substituiert sind, die ihrerseits durch Hydroxy substituiert sein können oder gegebenenfalls durch Phenyl oder Phenoxy substituiert sind, die ihrerseits bis zu 2-fach gleich oder verschieden durch Fluor, Chlor, Brom oder durch geradkettiges oder verzweigtes Alkyl mit bis zu 3 Kohlenstoffatomen substituiert sein können,<br>und/oder gegebenenfalls durch eine Gruppe der Formel $-(O)_cSO_2-R^{12}$ substituiert sind, worin |
| c | eine Zahl 0 oder 1 bedeutet,<br>und |
| $R^{12}$ | geradkettiges oder verzweigtes Alkyl mit bis zu 4 Kohlenstoffatomen oder Phenyl bedeutet, |
| b | eine Zahl 0 oder 1 bedeutet, |

T einen heterocyclischen Rest der Formel

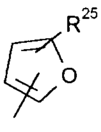 , 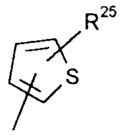 , 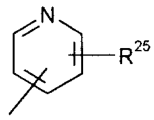 , 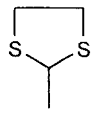 , 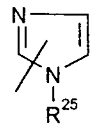 ,

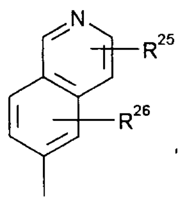 , 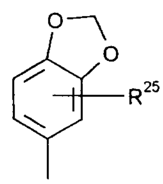 , 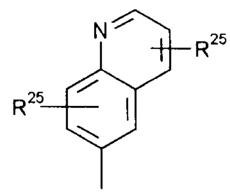 ,

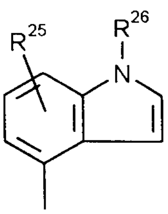 , 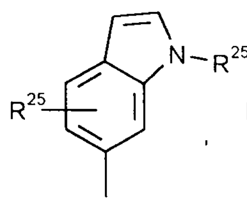 , 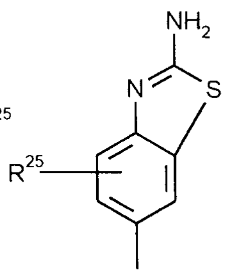 ,

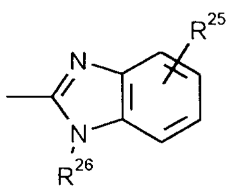 , 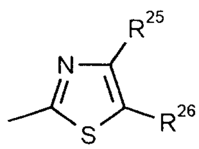 , 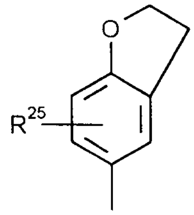 ,

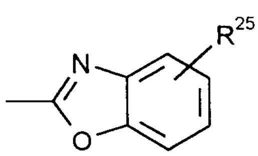

oder

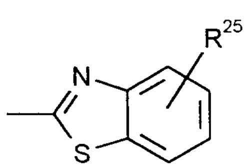

bedeutet
worin

$R^{25}$ und $R^{26}$ gleich oder verschieden sind und Wasserstoff, geradkettiges oder verzweigtes Alkyl mit bis zu 4 Kohlenstoffatomen oder Amino bedeuten,

V die oben angegebene Bedeutung von L oder T hat oder einen Rest der Formel

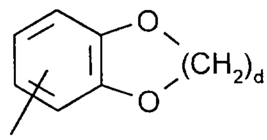

bedeutet,
worin

d eine Zahl 1 oder 2 bedeutet,

$R^6$ einen Rest der Formel $-(CH_2)_d-R^{13}$ bedeutet,
worin

e eine Zahl 0 oder 1 bedeutet,

$R^{13}$ Hydroxy, Carboxy oder geradkettiges oder verzweigtes Alkoxycarbonyl mit bis zu 3 Kohlenstoffatomen bedeutet,

$R^7$ Wasserstoff, Cyano, Trifluormethyl, Vinyl, geradkettiges oder verzweigtes Alkyl mit bis zu 6 Kohlenstoffatomen bedeutet, das gegebenenfalls bis zu 2-fach gleich oder verschieden durch geradkettiges oder verzweigtes Alkoxy mit bis zu 3 Kohlenstoffatomen substituiert ist, oder Alkoxy mit bis zu 5 Kohlenstoffatomen bedeutet, oder
eine Gruppe der Formel $-CO-NH-(CH_2)_f-NR^{14}R^{15}$ bedeutet,
worin

f eine Zahl 1, 2 oder 3 bedeutet,

$R^{14}$ und $R^{15}$ gleich oder verschieden sind und
Wasserstoff, Phenyl oder geradkettiges oder verzweigtes Alkyl mit bis zu 3 Kohlenstoffatomen bedeuten,

$R^8$ Wasserstoff, Carboxy, geradkettiges oder verzweigtes Alkoxycarbonyl mit bis zu 3 Kohlenstoffatomen oder geradkettiges oder verzweigtes Alkyl mit bis zu 5 Kohlenstoffatomen bedeutet, das gegebenenfalls durch Hydroxy, Carboxy oder durch geradkettiges oder verzweigtes Alkoxycarbonyl mit jeweils bis zu 3 Kohlenstoffatomen oder Phenyl substituiert ist,

$R^9$ und $R^{10}$ gleich oder verschieden sind und
Wasserstoff, Cyclopropyl, Cyclopentyl, Cyclohexyl oder Phenyl bedeuten, oder
geradkettiges oder verzweigtes Alkyl mit bis zu 5 Kohlenstoffatomen bedeuten, das gegebenenfalls durch Phenyl, Cyclopropyl, Cyclopentyl, Cyclohexyl, Hydroxy, Carboxy, durch geradkettiges oder verzweigtes Alkoxy oder Alkoxycarbonyl mit jeweils bis zu 3 Kohlenstoffatomen oder durch eine Gruppe der Formel $-NR^{16}R^{17}$ substituiert ist
worin

$R^{16}$ und $R^{17}$ gleich oder verschieden sind und Wasserstoff, Phenyl oder geradkettiges oder verzweigtes Alkyl mit bis zu 3 Kohlenstoffatomen bedeuten,

oder

| | |
|---|---|
| $R^9$ und $R^{10}$ | gemeinsam mit dem Stickstoffatom einen Heterocyclus der Formel |

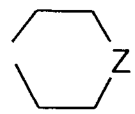

| | |
|---|---|
| | bilden,<br>worin |
| Z | ein Sauerstoffatom oder die Gruppe $-NR^{18}$ oder -CH bedeutet,<br>worin |
| $R^{18}$ | Wasserstoff, Acetyl, tert.-Butoxycarbonyl oder einen Rest der Formel $-SO_2R^{19}$ bedeutet,<br>worin |
| $R^{19}$ | geradkettiges oder verzweigtes Alkyl mit bis zu 3 Kohlenstoffatomen, Benzyl oder Phenyl bedeutet, das gegebenenfalls durch Phenyl oder Tolyl substiuiert ist, |
| W | geradkettiges oder verzweigtes Alkyl von 2 bis 7 Kohlenstoffatomen bedeutet, das 1- bis 3-fach gleich oder verschieden durch Hydroxy, Pyridyl, Norbornyl oder Phenyl substituiert ist, das seinerseits durch Hydroxy oder Benzyloxy substituiert sein kann, oder durch eine Gruppe der Formel $-OR^{20}$ oder $NR^{21}R^{22}$ substituiert ist,<br>worin |
| $R^{20}$ | geradkettiges oder verzweigtes Alkyl mit bis zu 5 Kohlenstoffatomen bedeutet, und |
| $R^{21}$ und $R^{22}$ | gleich oder verschieden sind und die oben angegebene Bedeutung von $R^{16}$ und $R^{17}$ haben, |
| X | ein Sauerstoff- oder Schwefelatom bedeutet, |
| Y | Formyl oder die Gruppe $-CHR^{23}R^{24}$ bedeutet,<br>worin |
| $R^{23}$ | Wasserstoff bedeutet, |
| $R^{24}$ | Hydroxy oder geradkettiges oder verzweigtes Alkoxy oder Acyl mit jeweils bis zu 3 Kohlenstoffatomen bedeutet,<br>oder |
| $R^{23}$ und $R^{24}$ | gleich oder verschieden sind und geradkettiges oder verzweigtes Alkoxy mit bis zu 3 Kohlenstoffatomen bedeuten, |

$R^{11}$ einen Rest der Formel

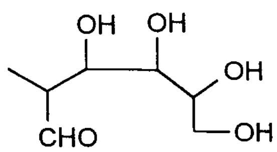
,
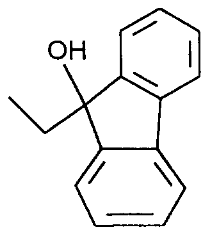
,
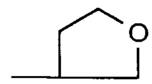

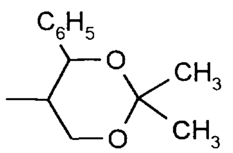
,
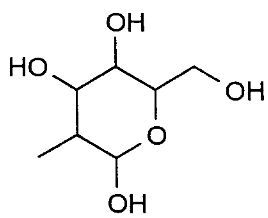

oder
Phenyl bedeutet, das gegebenenfalls bis zu 2-fach gleich oder verschieden durch Fluor, Chlor, Brom, Carboxy oder geradkettiges oder verzweigtes Alkoxycarbonyl mit bis zu 4 Kohlenstoffatomen substituiert ist,
oder
geradkettiges oder verzweigtes Alkyl mit bis zu 5 Kohlenstoffatomen bedeutet, das gegebenenfalls bis zu 2-fach gleich oder verschieden durch Hydroxy, Carboxy, Fluor, Chlor, Brom oder durch geradkettiges oder verzweigtes Alkoxycarbonyl mit bis zu 4 Kohlenstoffatomen substituiert ist,

und deren Salze,
wobei
2-[4-(Chinolin-2-yl-methoxy)phenyl]-2-cyclopentyl-essigsäuremethyloxycarbonylmethylamid,
2-[4-(Chinolin-2-yl-methoxy)phenyl]-2-cyclopentyl-essigsäurecarboxymethylamid,
N-Methyl-2-[3-isobutyl-4-(chinolin-2-yl-methoxy)phenyl]2-cycloheptylacetamid,
N-Methyl-2-[4(chinolin-2-yl-methoxy)phenyl]-2-cycloheptylessigsäureamid,
N-Ethyl-2-[4-(chinolin-2-yl-methoxy)phenyl]-2-cyclopentyl-essigsäureamid und
N-Ethyl-2-[4-(chinolin-2-yl-methoxy)phenyl]-2-cyclohexyl-essigsäureamid ausgenommen sind.

**3.** 4-(Chinolin-2-yl-methoxy)-phenyl-essigsäurederivate nach Anspruch 1,
worin

A und D gleich oder verschieden sind und
für Wasserstoff, Cyclopropyl, Cyclopentyl, Cyclohexyl, Azido, Hydroxy, Fluor, Chlor, Brom, geradkettiges oder verzweigtes Alkyl, Alkoxy oder Alkenyl mit jeweils bis zu 4 Kohlenstoffatomen, Imidazolyl oder Pyrryl stehen,

$R^1$ für Wasserstoff, Hydroxy oder geradkettiges oder verzweigtes Alkyl mit bis zu 3 Kohlenstoffatomen steht,

$R^2$ für Wasserstoff, Hydroxy, Fluor, Chlor, geradkettiges oder verzweigtes Alkenyl oder Alkoxy mit jeweils bis zu 5 Kohlenstoffatomen, Cyclopentyl, Cyclohexyl, Cycloheptyl, Cyclooctyl, Cyclononyl, Cyclodecyl oder Cycloundecyl steht, oder
für geradkettiges oder verzweigtes Alkyl mit bis zu 6 Kohlenstoffatomen steht, das gegebenenfalls durch Cyclopentyl, Cyclohexyl, Cycloheptyl, Cyclooctyl, Cyclononyl, Cycloundecyl, Phenyl oder Tetrahydropyranyl substituiert ist, welche ihrerseits durch Fluor, Chlor oder Brom substituiert sein können,
oder
für den Indanylrest steht,

$R^1$ und $R^2$ gemeinsam mit dem Kohlenstoffatom einen Cyclopentyl-, Cyclohexyl- oder Cycloheptylring bilden,

E für einen Rest der Formel

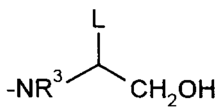 , 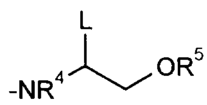 , 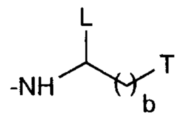 ,

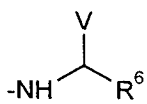 , 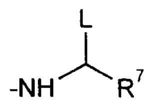 , 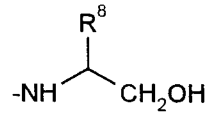 ,

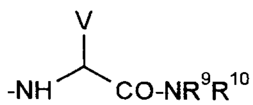 ,

$-NR^{4'}-W-OH$,

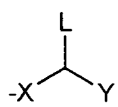 ,

$-NH-R^{11}$ oder

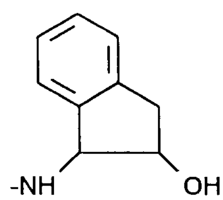

steht,

in welcher

$R^3$ Phenyl, Methyl, Acetyl oder tert. Butoxycarbonyl (Boc) bedeutet,

$R^4$ und $R^{4'}$ gleich oder verschieden sind und die oben angegebene Bedeutung von $R^3$ haben oder Wasserstoff bedeuten,

$R^5$ geradkettiges oder verzweigtes Acyl oder Alkyl mit jeweils bis zu 3 Kohlenstoffatomen bedeutet,

L Phenyl, Benzyl oder Naphthyl bedeutet, die gegebenenfalls bis zu 2-fach gleich oder verschieden durch Fluor, Chlor, Brom, Iod, Hydroxy, Pyrrolidinyl, Morpholino, Trifluormethoxy, Trifluormethyl, Amino, Cyclopropyl, Cyclopentyl, Cyclohexyl, geradkettiges oder verzweigtes Alkyl oder Alkoxy mit jeweils bis zu 6 Kohlenstoffatomen substituiert sind, die ihrerseits durch Hydroxy substituiert sein können, oder gegebenenfalls durch Phenyl oder Phenoxy substituiert sind, die ihrerseits bis zu 2-fach gleich oder verschieden durch Fluor, Chlor oder durch geradkettiges oder verzweigtes Alkyl mit bis zu 3 Kohlenstoffatomen substituiert sein können,

und/oder gegebenenfalls durch eine Gruppe der Formel $-(O)_cSO_2-R^{12}$ substituiert sind, worin

c eine Zahl 0 oder 1 bedeutet, und

$R^{12}$ geradkettiges oder verzweigtes Alkyl mit bis zu 3 Kohlenstoffatomen oder Phenyl bedeutet,

b eine Zahl 0 oder 1 bedeutet,

T einen heterocyclischen Rest der Formel

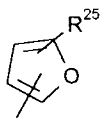 , 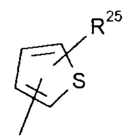 , 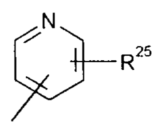 , 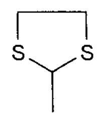 , 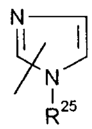 ,

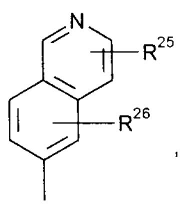 , 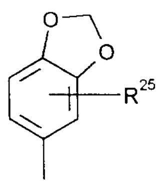 , 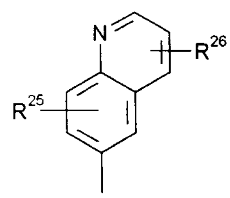 ,

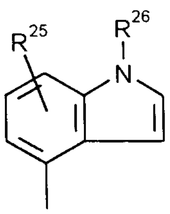 , 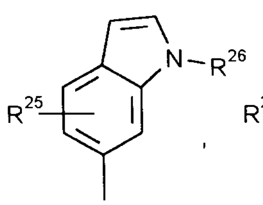 , 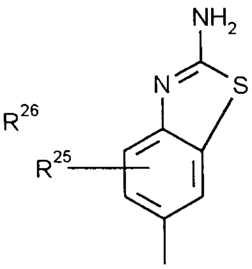 ,

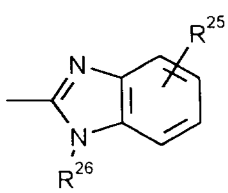 , 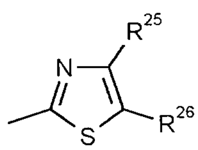 , 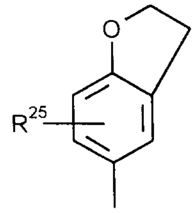

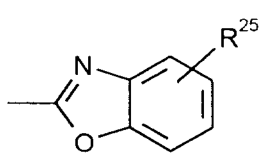

oder

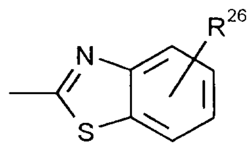

bedeutet
worin

$R^{25}$ und $R^{26}$ gleich oder verschieden sind und Wasserstoff, geradkettiges oder verzweigtes Alkyl mit bis zu 4 Kohlenstoffatomen oder Amino bedeuten,

V die oben angegebene Bedeutung von L oder T hat, oder einen Rest der Formel

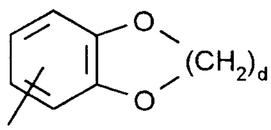

bedeutet,
worin

d eine Zahl 1 oder 2 bedeutet,

$R^6$ einen Rest der Formel $-(CH_2)_d-R^{13}$ bedeutet,
worin

e eine Zahl 0 oder 1 bedeutet,

$R^{13}$ Hydroxy, Carboxy oder geradkettiges oder verzweigtes Alkoxycarbonyl mit bis zu 3 Kohlenstoffatomen bedeutet,
wobei

$R^7$ Wasserstoff, Cyano, Trifluormethyl, Vinyl, geradkettiges oder verzweigtes Alkyl mit bis zu 5 Kohlenstoffatomen bedeutet, das gegebenenfalls bis zu 2-fach gleich oder verschieden durch geradkettiges oder verzweigtes Alkoxy mit bis zu 3 Kohlenstoffatomen substituiert ist, oder Alkoxy mit bis zu 3 Kohlenstoffatomen bedeutet, oder
eine Gruppe der Formel $-CO-NH-(CH_2)_f-NR^{14}R^{15}$ bedeutet,
worin

f eine Zahl 1, 2 oder 3 bedeutet,

$R^{14}$ und $R^{15}$ gleich oder verschieden sind und
Wasserstoff, Phenyl oder geradkettiges oder verzweigtes Alkyl mit bis zu 3 Kohlenstoffatomen bedeuten,

$R^8$ Wasserstoff, Carboxy, geradkettiges oder verzweigtes Alkoxycarbonyl mit bis zu 3 Kohlenstoffatomen oder geradkettiges oder verzweigtes Alkyl mit bis zu 4 Kohlenstoffatomen bedeutet, das gegebenenfalls durch Hydroxy, Carboxy oder durch geradkettiges oder verzweigtes Alkoxycarbonyl mit jeweils bis zu 3 Kohlenstoffatomen oder Phenyl substituiert ist,

$R^9$ und $R^{10}$ gleich oder verschieden sind und
Wasserstoff, Cyclopropyl, Cyclopentyl, Cyclohexyl oder Phenyl bedeuten, oder

geradkettiges oder verzweigtes Alkyl mit bis zu 5 Kohlenstoffatomen bedeuten, das gegebenenfalls durch Phenyl, Cyclopropyl, Cyclopentyl, Cyclohexyl, Hydroxy, Carboxy, durch geradkettiges oder verzweigtes Alkoxy oder Alkoxycarbonyl mit jeweils bis zu 3 Kohlenstoffatomen oder durch eine Gruppe der Formel $-NR^{16}R^{17}$ substituiert ist
worin

$R^{16}$ und $R^{17}$ gleich oder verschieden sind und Wasserstoff, Phenyl oder geradkettiges oder verzweigtes Alkyl mit bis zu 3 Kohlenstoffatomen bedeuten,

oder

$R^9$ und $R^{10}$ gemeinsam mit dem Stickstoffatom einen Heterocyclus der Formel

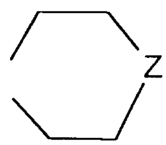

bilden,
worin

Z ein Sauerstoffatom oder die Gruppe $-NR^{18}$ oder -CH bedeutet,
worin

$R^{18}$ Wasserstoff, Acetyl, tert.-Butoxycarbonyl oder einen Rest der Formel $-SO_2R^{19}$ bedeutet,
worin

$R^{19}$ geradkettiges oder verzweigtes Alkyl mit bis zu 3 Kohlenstoffatomen, Benzyl oder Phenyl bedeutet, das gegebenenfalls durch Phenyl oder Tolyl substiuiert ist,

W geradkettiges oder verzweigtes Alkyl von 2 bis 7 Kohlenstoffatomen bedeutet, das 1- bis 3-fach gleich oder verschieden durch Hydroxy, Pyridyl, Norbornyl oder Phenyl substituiert ist, das seinerseits durch Hydroxy oder Benzyloxy substituiert sein kann, oder durch eine Gruppe der Formel $-OR^{20}$ oder $-NR^{21}R^{22}$ substituiert ist,
worin

$R^{20}$ geradkettiges oder verzweigtes Alkyl mit bis zu 4 Kohlenstoffatomen bedeutet und

$R^{21}$ und $R^{22}$ gleich oder verschieden sind und die oben angegebene Bedeutung von $R^{16}$ und $R^{17}$ haben,

X ein Sauerstoff- oder Schwefelatom bedeutet,

Y Formyl oder die Gruppe $-CHR^{23}R^{24}$ bedeutet,
worin

$R^{23}$ Wasserstoff bedeutet,

$R^{24}$ Hydroxy oder geradkettiges oder verzweigtes Alkoxy oder Acyl mit jeweils bis zu 3 Kohlenstoffatomen bedeutet,
oder

$R^{23}$ und $R^{24}$ gleich oder verschieden sind und geradkettiges oder verzweigtes Alkoxy mit bis zu 3 Kohlenstoffatomen bedeuten,

$R^{11}$ einen Rest der Formel

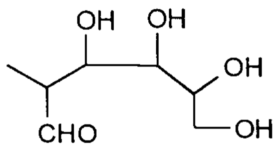 , 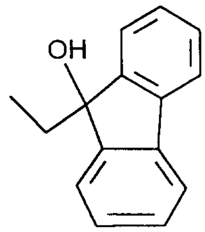 , 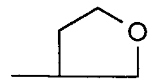

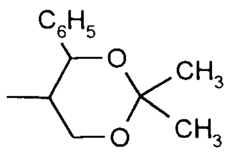 , 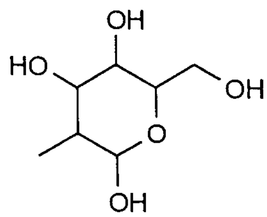

oder

Phenyl bedeutet, das gegebenenfalls bis zu 2-fach gleich oder verschieden durch Fluor, Chlor, Brom, Carboxy oder geradkettiges oder verzweigtes Alkoxycarbonyl mit bis zu 3 Kohlenstoffatomen substituiert ist,

oder

geradkettiges oder verzweigtes Alkyl mit bis zu 5 Kohlenstoffatomen bedeutet, das gegebenenfalls bis zu 2-fach gleich oder verschieden durch Hydroxy, Carboxy, Fluor, Chlor, Brom oder durch geradkettiges oder verzweigtes Alkoxycarbonyl mit bis zu 3 Kohlenstoffatomen substituiert ist,

und deren Salze,
wobei
2-[4-(Chinolin-2-yl-methoxy)phenyl]-2-cyclopentyl-essigsäuremethyloxycarbonylmethylamid,
2-[4-(Chinolin-2-yl-methoxy)phenyl]-2-cyclopentyl-essigsäurecarboxymethylamid,
N-Methyl-2-[3-isobutyl-4-(chinolin-2-yl-methoxy)phenyl]2-cycloheptylacetamid,
N-Methyl-2-[4-(chinolin-2-yl-methoxy)phenyl]-2-cycloheptyl-essigsäureamid,
N-Ethyl-2-[4-(chinolin-2-yl-methoxy)phenyl]-2-cyclopentyl-essigsäureamid
und
N-Ethyl-2-[4-(chinolin-2-yl-methoxy)phenyl]-2-cyclohexyl-essigsäureamid ausgenommen sind.

4. 4-(Chinolin-2-yl-methoxy)-phenyl-essigsäurederivate nach Anspruch 1 bis 3, zur therapeutischen Anwendung.

5. Verfahren zur Herstellung von 4-(Chinolin-2-yl-methoxy)-phenyl-essigsäurederivate nach Anspruch 1 bis 3, dadurch gekennzeichnet, daß man
[A] im Fall, daß E nicht für den Rest der Formel

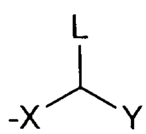

steht,
Carbonsäuren der allgemeinen Formel (II)

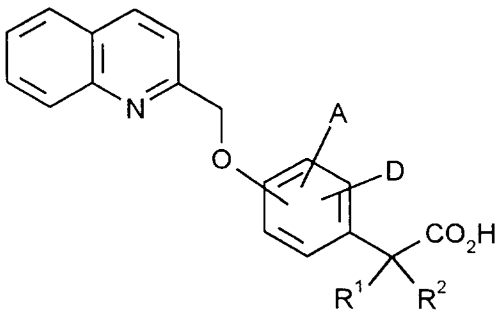

(II)

in welcher

A, D, $R^1$ und $R^2$ die angegebene Bedeutung haben,

gegebenenfalls unter vorgeschalteter Aktivierung der Carbonsäurefunktion, mit Glycinolen oder deren Ester der allgemeinen Formel (III)

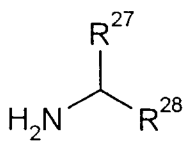

(III)

in welcher

$R^{27}$ den oben angegebenen Bedeutungsumfang von L, V, W und $R^7$ umfaßt

und

$R^{28}$ für die $-CH_2-OH$ oder für $(C_1-C_8)$-Alkoxycarbonyl steht,

in inerten Lösemitteln, in Anwesenheit einer Base und gegebenenfalls eines Hilfsstoffes umsetzt, und [B] im Fall, daß E für den Rest der Gruppe

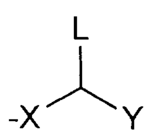

steht,
die Carbonsäuren der allgemeinen Formel (II), nach vorgeschalteter Aktivierung der Carbonsäurefunktion, mit Verbindungen der allgemeinen Formel (IV)

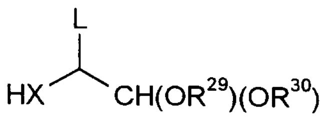

(IV)

in welcher
X und L die oben angegebene Bedeutung haben,
$R^{29}$ und $R^{30}$ gleich oder verschieden sind und $C_1$-$C_6$-Alkyl bedeuten,
in inerten Lösemitteln, gegebenenfalls in Anwesenheit einer Base und/oder Hilfsmittels, umsetzt,
und im Fall Y = CHO eine Oxidation anschließt,
und in Abhängigkeit der jeweiligen Definition des oben aufgeführten Substituenten E gegebenenfalls eine Acylierung, Reduktion, Verseifung, Amidierung, Alkylierung, Sulfoamidierung und/oder Eliminierung nach üblichen Methoden durchführt.

**6.** Verfahren nach Anspruch 5, dadurch gekennzeichnet, daß man die entsprechenden Ausgangsverbindungen (III) bzw. (IV) in optisch reiner Form einsetzt.

**7.** Arzneimittel enthaltend mindestens ein 4-(Chinolin-2-yl-methoxy)-phenyl-essigsäurederivat nach Anspruch 1 bis 3.

**8.** Arzneimittel nach Anspruch 7, zur Präventation und Behandlung von Atherosklerose, der Fettsucht und als Abführmittel.

**9.** Verwendung von 4-(Chinolin-2-yl-methoxy)-phenyl-essigsäurederivaten nach Anspruch 1 bis 4, zur Herstellung von Arzneimitteln.

**10.** Verwendung von 4-(Chinolin-2-yl-methoxy)-phenyl-essigsäurederivaten nach Anspruch 1 bis 3, zur Herstellung von antiatherosklerotischen Arzneimitteln.

Europäisches Patentamt

# EUROPÄISCHER RECHERCHENBERICHT

Nummer der Anmeldung
EP 95 11 8606

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | EP-A-0 545 170 (BAYER AG) 9.Juni 1993<br>* das ganze Dokument *<br>--- | 1-10 | C07D215/14<br>A61K31/47<br>C07D405/12<br>C07D403/12<br>C07D401/12<br>C07D417/12<br>C07D409/12 |
| X | EP-A-0 582 908 (BAYER AG) 16.Februar 1994<br>* das ganze Dokument *<br>--- | 1-10 | |
| X,D | EP-A-0 344 519 (BAYER AG) 6.Dezember 1989<br>* das ganze Dokument *<br>--- | 1-10 | |
| Y | EP-A-0 530 639 (BAYER AG) 10.März 1993<br>* das ganze Dokument *<br>--- | 1-10 | |
| Y | WO-A-94 10148 (ABBOTT LAB) 11.Mai 1994<br>* das ganze Dokument *<br>--- | 1-10 | |
| Y | EP-A-0 574 774 (BAYER AG) 22.Dezember 1993<br>* das ganze Dokument *<br>--- | 1-10 | |
| D,Y | EP-A-0 529 450 (BAYER AG) 3.März 1993<br>* das ganze Dokument *<br>--- | 1-10 | RECHERCHIERTE SACHGEBIETE (Int.Cl.6) |
| Y | EP-A-0 499 926 (BAYER AG) 26.August 1992<br>* das ganze Dokument *<br>--- | 1-10 | C07D<br>A61K |
| Y | EP-A-0 414 076 (BAYER AG) 27.Februar 1991<br>* das ganze Dokument *<br>--- | 1-10 | |
| Y | EP-A-0 399 291 (BAYER AG) 28.November 1990<br>* das ganze Dokument *<br>--- | 1-10 | |
| Y | EP-A-0 582 916 (BAYER AG) 16.Februar 1994<br>* das ganze Dokument *<br>--- | 1-10 | |
| Y | EP-A-0 549 879 (BAYER AG) 7.Juli 1993<br>* siehe auch Beispiele II und III, Seite 11/12 *<br>* das ganze Dokument *<br>---<br>-/-- | 1-10 | |

Der vorliegende Recherchenbericht wurde für alle Patentansprüche erstellt

| Recherchenort | Abschlußdatum der Recherche | Prüfer |
|---|---|---|
| MÜNCHEN | 24.April 1996 | Stellmach, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 95 11 8606

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y | EP-A-0 545 171 (BAYER AG) 9.Juni 1993<br>* siehe auch Beispiel III, Seite 8 *<br>* das ganze Dokument *<br>--- | 1-10 | |
| D,X | EP-A-0 518 672 (MERCK & CO INC)<br>16.Dezember 1992<br>* das ganze Dokument *<br>--- | 1-10 | |
| D,X | EP-A-0 514 267 (ADIR) 19.November 1992<br>* das ganze Dokument *<br>--- | 1-10 | |
| D,X | WO-A-91 18897 (WELLCOME FOUND) 12.Dezember 1991<br>* das ganze Dokument *<br>----- | 1-10 | |
| | | | RECHERCHIERTE SACHGEBIETE (Int.Cl.6) |

Der vorliegende Recherchenbericht wurde für alle Patentansprüche erstellt

| Recherchenort | Abschlußdatum der Recherche | Prüfer |
|---|---|---|
| MÜNCHEN | 24.April 1996 | Stellmach, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)